# EUROPEAN PATENT APPLICATION

(11) **EP 4 733 322 A1**
(43) Date of publication of application: **29.04.2026**
(21) Application number: 24825341.1
(22) Date of filing: 21.06.2024
(51) Int. Cl.: C07K 16/28, C07K 19/00, C07K 16/46, C12N 15/13, C12N 5/10, G01N 33/574, A61K 45/06, A61K 38/17

(54) **MODIFIED TCR CONSTANT REGION AND USE THEREOF IN TCR FUSION PROTEIN**

(30) Priority: 21.06.2023 WO PCT/CN2023/101881; 25.06.2023 WO PCT/CN2023/102211; 27.12.2023 CN 202311826282
(71) Applicant: Corregene Biotechnology Co., Ltd., Beijing 102206 (CN)
(72) Inventor: XIE, Xingwang, Beijing 102206 (CN); JIANG, Dong, Beijing 102206 (CN); BI, Jinglei, Beijing 102206 (CN); YAN, Baoqi, Beijing 102206 (CN); FU, Shuangli, Beijing 102206 (CN); QIAO, Xuewei, Beijing 102206 (CN); LI, Xian, Beijing 102206 (CN); LI, Yonghong, Beijing 102206 (CN); WANG, Jianghua, Beijing 102206 (CN); WANG, Xueyan, Beijing 102206 (CN)
(74) Representative: Maiwald GmbH
(86) International application number: PCT/CN2024/100701
(87) International publication number: WO 2024/260454

(57) **Abstract**

Provided are a modified TCR constant region and a use thereof in a TCR fusion protein, specifically relating to a modified TCR a chain constant region, TCR β chain constant region, and TCR constant region, as well as a polypeptide molecule, a TCR, and a multi-specific antigen binding molecule comprising same. Further provided is a method for treating diseases by using the polypeptide molecule, the TCR, or the multi-specific antigen binding molecule.

## Description

This application claims the priority of International Patent Application PCT/CN2023/101881 filed on June 21, 2023, International Patent Application PCT/CN2023/102211 filed on June 25, 2023, and Chinese Patent Application 202311826282.6 filed on December 27, 2023, the entire contents of which are incorporated herein by reference.

### Technical Field

The present disclosure relates to a modified T cell receptor (TCR) constant region and a use thereof in TCR-based multi-specific antigen binding molecules, in particular to the use of the polypeptide molecules in the treatment of cancer, infectious diseases, autoimmune diseases, and/or inflammatory diseases.

### Background Art

It is well known that the human immune system comprises "three lines of defense," namely: the first line formed by the skin and mucosa and their secretions; the second line formed by bactericidal substances (such as lysozyme) in body fluids and phagocytes; and the third line formed by immune organs and immune cells (T-cells and B cells). The former two are collectively referred to as the innate immune system, and the third is referred to as the adaptive immune system. The primary function of innate immune system is anti-infection; it is the body's first line of defense against invasion by pathogenic microorganisms and is characterized by being congenitally acquired, acting immediately, non-specific, and lacking memory. The adaptive immune system is a targeted and evolutionarily more advanced immune function that is produced after humans adapt to the living environment and encounter antigenic substances, and is characterized by being acquired postnatally, having a delayed response, and possessing specificity and memory. By using the characteristics of the specificity and memory of the adaptive immune response, a large number of biopharmaceutical products have been developed for the treatment of viral infections, tumors, and autoimmune diseases, achieving substantial clinical benefits. These products mainly achieve therapeutic goals by mimicking the mechanism by which the products of B-cell immune response (antibodies) exert immune functions in vivo, e.g. monoclonal antibodies, bispecific antibodies, multi-specific antibodies, antibody-drug conjugates, and CAT-T/CAR-NK products.

However, antibodies can only recognize proteins displayed on the surface of the target cell membrane (accounting for about 10% of total proteins). This means that more than 90% of diseases cannot be treated with antibodies and antibody-derived products and cannot benefit therefrom, whereas TCR-related products derived from T-cell immunity can perfectly fill this gap. TCR recognition of the major histocompatibility complex (MHC)-antigenic peptide complexes (pMHC) is an important step in T-cell-mediated immune responses. TCRs can recognize antigenic peptides which are derived by processing the tumor-specific antigens from intracellular and cell-surface and are presented on the cell-surface MHC molecules, thereby covering a much broader recognition scope. In TCR-based T-cell-redirecting bispecific antibodies, the target cell recognition moiety is replaced from a traditional antibody to a TCR, which allows using the broad recognition characteristics of TCRs to increase target selectivity. In recent years, numerous studies have focused on developing TCR-T products for late-stage/end-stage cancer treatment. Abundant in vitro and in vivo data have demonstrated the effectiveness of such products in cancer therapy, and multiple products have advanced to clinical trials both domestically and internationally. Due to process limitations of autologous TCR-T, the high cost of TCR-T therapy, long manufacturing cycles, low success rates of manufacturing, the difficulty of reuse, and other factors have greatly limited the widespread application of this class of products.

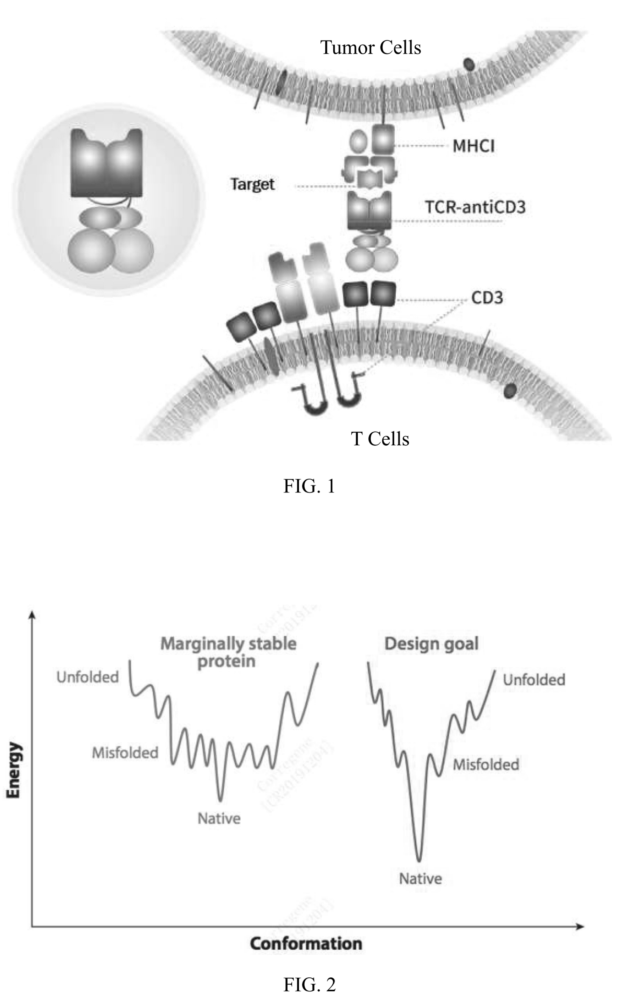

A T-cell redirection molecule (ImmTAC) developed by expressing a TCR in soluble form fused to an anti-CD3 antibody can fully exploit the advantage of TCRs in broadly recognizing numerous targets while also retain the low marginal-cost advantage of antibody drugs; its mechanism of action is shown in FIG. 1. Based on this structure, the first clinical drug has already been approved for marketing, demonstrating safety and efficacy in clinical trials, and the treatment cost is far lower than adoptive therapies (TCR-T). Because a natural TCR is a transmembrane protein molecule, it does not possess excellent soluble expression properties like antibody; therefore, improving the stability of TCR soluble expression has become an urgent priority to be solved.

Scientists initially attempted to express TCR Va-Vb in a single-chain fusion in the form of ScFv, but found that most TCRs were difficult to express successfully, which was presumed to be due to exposure of hydrophobic amino acid residues at the Va-Ca and Vb-Cb junctions. Mutations were therefore introduced into these amino acids, and mutant ScFv molecules capable of soluble expression were screened out; however, these mutations are TCR-specific and are not universal across different TCRs. Subsequently, introducing a new disulfide bond into a truncated TCR constant region improved the success rate and yield of soluble TCR expression, but for TCRs with a relatively poor baseline expression level, such a strategy still could not achieve successful soluble expression.

### Summary of the Invention

By combining computer prediction with wet-lab validation, the inventors successfully screened a series of novel TCR constant-region mutations that improve soluble expression of TCRs, in particular the mutation combination CMM1. The CMM1 mutation combination can enhance the stability and solubility of TCR fusion proteins, increase the success rate and yield of soluble expression of TCR fusion proteins, enhance the activity of TCR fusion proteins, and reduce non-specific activation of PBMC cells under conditions of high-concentration TCR fusion-protein, thereby improving the therapeutic safety window. This discovery breaks through an important barrier to the development of soluble TCR molecular drugs and is expected to greatly reduce the cost and timeline of TCR screening and TCR soluble expression during drug development.

Accordingly, in a first aspect, the present disclosure provides a T cell receptor (TCR) α chain constant region, wherein the α chain constant region (TRAC) comprises amino acid substitution(s) at one or more of the positions: Q6, S21, K83, S84.6, and I114; wherein the amino acid positions are numbered according to the International Immunogenetics Information System (IMGT) numbering rules based on the TCR α chain constant region as shown in SEQ ID NO. 1.

In some embodiments, (1) the amino acid substitution at position Q6 of the α chain constant region is selected from Q6R, Q6K, Q6D, and Q6E, preferably selected from Q6R and Q6K; and/or
(2) the amino acid substitution at position S21 of the α chain constant region is selected from S21I, S21L, S21V, S21G, S21A, and S21P, preferably selected from S21I, S21L, and S21V; and/or
(3) the amino acid substitution at position K83 of the α chain constant region is selected from K83P, K83G, K83A, K83I, K83L, and K83V, preferably selected from K83P, K83G, and K83A; and/or
(4) the amino acid substitution at position S84.6 of the α chain constant region is selected from S84.6D, S84.6E, S84.6N, S84.6Q, S84.6K, and S84.6R, preferably selected from S84.6D, S84.6E, S84.6N, and S84.6Q; and/or
(5) the amino acid substitution at position I114 of the α chain constant region is selected from 1114E, I114D, I114K, and I114R, preferably selected from I114E and I114D.

In some embodiments, (1) the amino acid substitution at position Q6 of the α chain constant region is Q6R; and/or
(2) the amino acid substitution at position S21 of the α chain constant region is S21I; and/or
(3) the amino acid substitution at position K83 of the α chain constant region is K83P; and/or
(4) the amino acid substitution at position S84.6 of the α chain constant region is selected from S84.6D, S84.6E, and S84.6N; and/or
(5) the amino acid substitution at position I114 of the α chain constant region is I114E.

In some embodiments, the α chain constant region comprises amino acid substitutions at positions S21, K83, and S84.6.

In some embodiments, the α chain constant region comprises one or more of the amino acid substitutions S21I, K83P, and S84.6D; preferably comprises at least two of the amino acid substitutions S21I, K83P and S84.6D; more preferably comprises amino acid substitutions S21I, K83P and S84.6D.

In some embodiments, the α chain constant region comprises amino acid substitutions selected from any one of the following groups (1)-(4):
(1) S211, K83P, and S84.6D;
(2) K83P and S84.6D;
(3) S21I and S84.6D;
(4) S21I and K83P.

In some embodiments, the α chain constant region further comprises one or more of the amino acid mutations N1.2K, T84C and an FFPSP deletion at positions 120-124; preferably comprises T84C, and optionally N1.2K and/or an FFPSP deletion at positions 120-124; more preferably comprises N1.2K, T84C and an FFPSP deletion at positions 120-124.

In some embodiments, the α chain constant region comprises an amino acid sequence as shown in any one of SEQ ID NOs.3-13, or an amino acid sequence having at least 80%, at least 85%, at least 90%, at least 95%, or at least 99% sequence identity with any one of SEQ ID NOs.3-13.

In a second aspect, the present disclosure provides a TCR α chain constant region comprising amino acid substitutions S211, K83P and S84.6D, wherein the amino acid positions are numbered according to the International Immunogenetics Information System (IMGT) numbering rules based on the TCR α chain constant region as shown in SEQ ID NO. 1; preferably, the TCR α chain constant region comprises an amino acid sequence as shown in SEQ ID NO. 13 or an amino acid sequence having at least 80%, at least 85%, at least 90%, at least 95% or at least 99% sequence identity to SEQ ID NO.13.

In a preferred embodiment, the TCR α chain constant region comprises or consists of the amino acid sequence as shown in SEQ ID NO.13.

In a third aspect, the present disclosure provides a TCR β chain constant region, wherein the β chain constant region comprises amino acid substitution(s) at one or more of the positions S92, T94 and Q107; wherein the amino acid positions are numbered according to the International Immunogenetics Information System (IMGT) numbering rules based on the TCR β constant region as shown in SEQ ID NO. 14.

In some embodiments, (1) the amino acid substitution at position S92 of the β chain constant region is selected fromS92P, S92E, S92D, S92L, S92N, S92G, S92A, S92I, S92V, S92Q, S92K and S92R, preferably selected from S92P, S92E, S92D, S92L, S92N, S92G, S92A, S92I, S92V and S92Q; and/or
(2) the amino acid substitution at position T94 of the β chain constant region is selected from T94D, T94E, T94K and T94R, preferably selected from T94D and T94E; and/or
(3) the amino acid substitution at position Q107 of the β chain constant region is selected from Q107L, Q107I, Q107V, Q107G, Q107A and Q107P, preferably selected from Q107L, Q107I and Q107V.

In some embodiments, (1) the amino acid substitution at position S92 of the β chain constant region is selected from S92P, S92E, S92D, S92L and S92N; and/or
(2) the amino acid substitution at position T94 of the β chain constant region is T94D; and/or
(3) the amino acid substitution at position Q107 of the β chain constant region is Q107L.

In some embodiments, the β chain constant region comprises amino acid substitutions at T94 and Q107.

In some embodiments, the β chain constant region comprises one or more of the amino acid substitutions T94D and Q107L; preferably comprises amino acid substitutions T94D and Q107L.

In some embodiments, the β chain constant region comprises amino acid substitution(s) selected from any one of the following groups (1)-(3):
(1) T94D and Q107L;
(2) Q107L;
(3) T94D.

In some embodiments, the TCR β chain constant region further comprises one or more of the amino acid mutations S79C, C85.1A and N97D; preferably comprises S79C and optionally C85.1A and/or N97D; more preferably comprises S79C, C85.1A and N97D.

In some embodiments, the TCR β chain constant region comprises an amino acid sequence as shown in any one of SEQ ID NOs.16-23, or an amino acid sequence having at least 80%, at least 85%, at least 90%, at least 95%, or at least 99% sequence identity with any one of SEQ ID NOs.16-23.

In a fourth aspect, the present disclosure provides a TCR β chain constant region comprising amino acid substitutions T94D and Q107L, wherein the amino acid positions are numbered according to the International Immunogenetics Information System (IMGT) numbering rules based on the TCR β chain constant region as shown in SEQ ID NO. 14;
preferably, the TCR β chain constant region comprises an amino acid sequence as shown in SEQ ID NO.23 or an amino acid sequence having at least 80%, at least 85%, at least 90%, at least 95% or at least 99% sequence identity to SEQ ID NO.23

In a preferred embodiment, the TCR β chain constant region comprises an amino acid sequence as shown in SEQ ID NO.23.

In a fifth aspect, the present disclosure provides a TCR constant region comprising the TCR α chain constant region as described herein and the TCR β chain constant region as described herein.

In some embodiments, the TCR constant region comprises amino acid substitutions selected from any one of the following groups (1)-(6):
(1) S21I, K83P and S84.6D of the α chain constant region, and T94D and Q107L of the β chain constant region;
(2) K83P and S84.6D of the α chain constant region, and T94D and Q107L of the β chain constant region;
(3) S21I and S84.6D of the α chain constant region, and T94D and Q107L of the β chain constant region;
(4) S21I and K83P of the α chain constant region, and T94D and Q107L of the β chain constant region;
(5) S21I, K83P and S84.6D of the α chain constant region, and Q107L of the β chain constant region;
(6) S21I, K83P and S84.6D of the α chain constant region, and T94D of the β chain constant region.

In some embodiments, the α chain constant region comprises amino acid substitutions S21I, K83P, and S84.6D, and the β chain constant region comprises amino acid substitutions T94D and Q107L.

In some embodiments, the TCR α chain constant region comprises an amino acid sequence as shown in SEQ ID NO.13 or an amino acid sequence having at least 80%, at least 85%, at least 90%, at least 95% or at least 99% sequence identity to SEQ ID NO.13; and the TCR β chain constant region comprises an amino acid sequence as shown in SEQ ID NO.23 or an amino acid sequence having at least 80%, at least 85%, at least 90%, at least 95% or at least 99% sequence identity to SEQ ID NO.23.

In a sixth aspect, the present disclosure provides a polypeptide molecule comprising the TCR α chain constant region, TCR β chain constant region, or TCR constant region as described herein.

In some embodiments, the polypeptide molecule further comprises an antigen binding region that binds at least one antigen.

In some embodiments, the antigen binding region is selected from an antigen binding region derived from an antibody and an antigen binding region derived from a TCR.

In a seventh aspect, the present disclosure provides a T cell receptor (TCR) comprising an α chain and a β chain, wherein the α chain comprises the TCR α chain constant region as described herein, and/or the β chain comprises the TCR β chain constant region as described herein; or the TCR comprises the TCR constant region as described herein.

In some embodiments, the TCR binds an antigen selected from melanoma-associated antigens (e.g., gp 100, MAGEA1), HPV antigens (e.g., HPV E6 or E7) and KRAS antigens (e.g., KRAS G12V, KRAS G12D).

In some embodiments, the TCR comprises a variable region derived from a murine TCR or a human TCR.

In some embodiments, the TCR is isolated or purified.

In an eighth aspect, the present disclosure provides a T cell receptor (TCR) comprising an α chain and a β chain,
wherein the α chain constant region comprises an amino acid substitution at one or more of the positions Q6, S21, K83, S84.6, and I114; and/or
wherein the β chain constant region comprises an amino acid substitution at one or more of the positions S92, T94, and Q107;
wherein the amino acid positions are numbered according to the International Immunogenetics Information System (IMGT) numbering rules based on the TCR α chain constant region as shown in SEQ ID NO.1 and the TCR β chain constant region as shown in SEQ ID NO.14.

In some embodiments, (1) the amino acid substitution at position Q6 of the α chain constant region is selected from Q6R, Q6K, Q6D, and Q6E, preferably selected from Q6R and Q6K; and/or
(2) the amino acid substitution at position S21 of the α chain constant region is selected from S21I, S21L, S21V, S21G, S21A, and S21P, preferably selected from S21I, S21L, and S21V; and/or
(3) the amino acid substitution at position K83 of the α chain constant region is selected from K83P, K83G, K83A, K83I, K83L, and K83V, preferably selected from K83P, K83G, and K83A; and/or
(4) the amino acid substitution at position S84.6 of the α chain constant region is selected from S84.6D, S84.6E, S84.6N, S84.6Q, S84.6K, and S84.6R, preferably selected from S84.6D, S84.6E, S84.6N, and S84.6Q; and/or
(5) the amino acid substitution at position I114 of the α chain constant region is selected from 1114E, 1114D, I114K, or I114R, preferably selected from I114E and I114D; and/or
(6) the amino acid substitution at position S92 of the β chain constant region is selected from S92P, S92E, S92D, S92L, S92N, S92G, S92A, S92I, S92V, S92Q, S92K and S92R, preferably selected from S92P, S92E, S92D, S92L, S92N, S92G, S92A, S92I, S92V and S92Q; and/or
(7) the amino acid substitution at position T94 of the β chain constant region is selected from T94D, T94E, T94K, or T94R, preferably selected from T94D and T94E; and/or
(8) the amino acid substitution at position Q107 of the β chain constant region is selected from Q107L, Q107I, Q107V, Q107G, Q107A, or Q107P, preferably selected from Q107L, Q107I, and Q107V.

In some embodiments, (1) the amino acid substitution at position Q6 of the α chain constant region is Q6R; and/or
(2) the amino acid substitution at position S21 of the α chain constant region is S21I; and/or
(3) the amino acid substitution at position K83 of the α chain constant region is K83P; and/or
(4) the amino acid substitution at position S84.6 of the α chain constant region is selected from S84.6D, S84.6E, and S84.6N; and/or
(5) the amino acid substitution at position I114 of the α chain constant region is I114E; and/or
(6) the amino acid substitution at position S92 of the β chain constant region is selected from S92P, S92E, S92D, S92L, and S92N; and/or
(7) the amino acid substitution at position T94 of the β chain constant region is T94D; and/or
(8) the amino acid substitution at position Q107 of the β chain constant region is Q107L.

In some embodiments, the α chain constant region comprises amino acid substitutions at S21, K83, and S84.6, and/or the β chain constant region comprises amino acid substitutions at T94 and Q107.

In some embodiments, the α chain constant region comprises one or more of the amino acid substitutions S21I, K83P, and S84.6D; preferably comprises at least two of S21I, K83P, and S84.6D; more preferably comprises S21I, K83P, and S84.6D.

In some embodiments, the β chain constant region comprises one or more of the amino acid substitutions T94D and Q107L; preferably comprises T94D and Q107L.

In some embodiments, the TCR comprises amino acid substitutions selected from any one of the following groups (1)-(6):
(1) S21I, K83P and S84.6D of the α chain constant region, and T94D and Q107L of the β chain constant region;
(2) K83P and S84.6D of the α chain constant region, and T94D and Q107L of the β chain constant region;
(3) S21I and S84.6D of the α chain constant region, and T94D and Q107L of the β chain constant region;
(4) S21I and K83P of the α chain constant region, and T94D and Q107L of the β chain constant region;
(5) S21I, K83P and S84.6D of the α chain constant region, and Q107L of the β chain constant region;
(6) S21I, K83P and S84.6D of the α chain constant region, and T94D of the β chain constant region;
preferably, the α chain constant region comprises amino acid substitutions S21I, K83P, and S84.6D, and the β chain constant region comprises amino acid substitutions T94D and Q107L.

In some embodiments, the α chain constant region further comprises one or more of the mutations: N1.2K, T84C, and an FFPSP deletion at positions 120-124; and/or the β chain constant region further comprises one or more of the mutations: S79C, C85.1A, and N97D; preferably, the α chain constant region further comprises N1.2K, T84C, and the FFPSP deletion at positions 120-124, and the β chain constant region further comprises S79C, C85.1A, and N97D.

In some embodiments, the TCR comprises an α chain constant region having an amino acid sequence as shown in any one of SEQ ID NOs.3-13 or an amino acid sequence having at least 80%, at least 85%, at least 90%, at least 95%, or at least 99% sequence identity with any one of SEQ ID NOs.3-13; and/or comprises a β chain constant region having an amino acid sequence as shown in any one of SEQ ID NOs. 16-23 or an amino acid sequence having at least 80%, at least 85%, at least 90%, at least 95%, or at least 99% sequence identity with any one of SEQ ID NOs.16-23.

In some embodiments, the TCR binds an antigen is selected from melanoma-associated antigens (e.g., gp100, MAGEA1), HPV antigens (e.g., HPV E6 or E7), and KRAS antigens (e.g., KRAS G12V, KRAS G12D).

In some embodiments, the TCR does not comprise a TRAV having an amino acid sequence as shown in SEQ ID NO.24 and does not comprise a TRBV having an amino acid sequence as shown in SEQ ID NO.25. In some embodiments, the TCR does not comprise a TRAV having CDR1, CDR2, and CDR3 with amino acid sequences as shown in SEQ ID NOs.38-40, respectively, and does not comprise a TRBV having CDR1, CDR2, and CDR3 with amino acid sequences as shown in SEQ ID NOs.41-43, respectively. In some embodiments, the TCR does not bind the KRAS antigen epitope VVGAVGVGK or a complex thereof with HLA-A*11. In some embodiments, the TCR does not bind KRAS antigens.

In some embodiments, the TCR comprises a variable region derived from a murine TCR or a human TCR.

In some embodiments, the TCR is isolated or purified.

In a ninth aspect, the present disclosure provides a multi-specific antigen binding molecule comprising the TCR α chain constant region, the TCR β chain constant region, the TCR constant region, the polypeptide molecule, or the TCR as described herein.

In a tenth aspect, the present disclosure provides a multi-specific antigen binding molecule comprising at least one antigen binding region derived from a TCR and any one selected from the group consisting of the following (1)-(2):
(1) the TCR α chain constant region (TRAC) as described herein, and/or the TCR β chain constant region (TRBC) as described herein; or
(2) the TCR constant region as described herein.

In an eleventh aspect, the present disclosure provides a multi-specific antigen binding molecule comprising at least one antigen binding region derived from a TCR, and a TCR α chain constant region (TRAC) and/or a TCR β chain constant region (TRBC);
wherein the α chain constant region comprises amino acid substitution(s) at one or more of the positions Q6, S21, K83, S84.6, and I114; and/or
wherein the β chain constant region comprises amino acid substitution(s) at one or more of the positions S92, T94, and Q107;
wherein the amino acid positions are numbered according to the International Immunogenetics Information System (IMGT) numbering rules based the TCR α chain constant region as shown in SEQ ID NO.1 and the TCR β chain constant region as shown in SEQ ID NO.14.

In some embodiments, (1) the amino acid substitution at position Q6 of the α chain constant region is selected from Q6R, Q6K, Q6D, and Q6E, preferably selected from Q6R and Q6K; and/or
(2) the amino acid substitution at position S21 of the α chain constant region is selected from S21I, S21I, S21V, S21G, S21A, and S21P, preferably selected from S21I, S21L, and S21V; and/or
(3) the amino acid substitution at position K83 of the α chain constant region is selected from K83P, K83G, K83A, K83I, K83L, and K83V, preferably selected from K83P, K83G, and K83A; and/or
(4) the amino acid substitution at position S84.6 of the α chain constant region is selected from S84.6D, S84.6E, S84.6N, S84.6Q, S84.6K, and S84.6R, preferably selected from S84.6D, S84.6E, S84.6N, and S84.6Q; and/or
(5) the amino acid substitution at position I114 of the α chain constant region is selected from I114E, I114D, I114K, or I114R, preferably selected from I114E and I114D; and/or
(6) the amino acid substitution at position S92 of the β chain constant region is selected from S92P, S92E, S92D, S92L, S92N, S92G, S92A, S92I, S92V, S92Q, S92K and S92R, preferably selected from S92P, S92E, S92D, S92L, S92N, S92G, S92A, S92I, S92V and S92Q; and/or
(7) the amino acid substitution at position T94 of the β chain constant region is selected from T94D, T94E, T94K, or T94R, preferably selected from T94D and T94E; and/or
(8) the amino acid substitution at position Q107 of the β chain constant region is selected from Q107L, Q107I, Q107V, Q107G, Q107A, or Q107P, preferably selected from Q107L, Q107I, and Q107V.

In some embodiments, (1) the amino acid substitution at position Q6 of the α chain constant region is Q6R; and/or
(2) the amino acid substitution at position S21 of the α chain constant region is S21I; and/or
(3) the amino acid substitution at position K83 of the α chain constant region is K83P; and/or
(4) the amino acid substitution at position S84.6 of the α chain constant region is selected from S84.6D, S84.6E, and S84.6N; and/or
(5) the amino acid substitution at position I114 of the α chain constant region is I114E; and/or
(6) the amino acid substitution at position S92 of the β chain constant region is selected from S92P, S92E, S92D, S92L, and S92N; and/or
(7) the amino acid substitution at position T94 of the β chain constant region is T94D; and/or
(8) the amino acid substitution at position Q107 of the β chain constant region is Q107L.

In some embodiments, the α chain constant region comprises amino acid substitutions at S21, K83, and S84.6, and/or the β chain constant region comprises amino acid substitutions at T94 and Q107.

In some embodiments, the α chain constant region comprises one or more of the amino acid substitutions S21I, K83P, and S84.6D; preferably comprises at least two of S21I, K83P, and S84.6D; more preferably comprises S21I, K83P, and S84.6D.

In some embodiments, the β chain constant region comprises one or more of the amino acid substitutions T94D and Q107L; preferably comprises T94D and Q107L.

In some embodiments, the multi-specific antigen binding molecule comprises amino acid substitutions selected from any one of the following groups (1)-(6):
(1) S21I, K83P, and S84.6D of the α chain constant region, and T94D and Q107L of the β chain constant region;
(2) K83P and S84.6D of the α chain constant region, and T94D and Q107L of the β chain constant region;
(3) S21I and S84.6D of the α chain constant region, and T94D and Q107L of the β chain constant region;
(4) S21I and K83P of the α chain constant region, and T94D and Q107L of the β chain constant region;
(5) S21I, K83P, and S84.6D of the α chain constant region, and Q107L of the β chain constant region;
(6) S21I, K83P, and S84.6D of the α chain constant region, and T94D of the β chain constant region;
preferably, the α chain constant region comprises amino acid substitutions S21I, K83P, and S84.6D, and the β chain constant region comprises amino acid substitutions T94D and Q107L.

In some embodiments, the α chain constant region further comprises one or more of the mutations: N1.2K, T84C, and an FFPSP deletion at positions 120-124; and/or the β chain constant region further comprises one or more of the mutations: S79C, C85.1A, and N97D; preferably, the α chain constant region further comprises N1.2K, T84C, and the FFPSP deletion at positions 120-124, and the β chain constant region further comprises S79C, C85.1A, and N97D.

In some embodiments, the α chain constant region comprises an amino acid sequence as shown in any one of SEQ ID NOs.3-13 or an amino acid sequence having at least 80%, at least 85%, at least 90%, at least 95%, or at least 99% sequence identity with any one of SEQ ID NOs.3-13; and/or the β chain constant region comprises an amino acid sequence as shown in any one of SEQ ID NOs.16-23 or an amino acid sequence having at least 80%, at least 85%, at least 90%, at least 95%, or at least 99% sequence identity with any one of SEQ ID NOs.16-23.

In some embodiments, the multi-specific antigen binding molecule comprises two or more antigen binding regions that bind two or more antigens, and the two or more antigens are each independently selected from tumor-associated antigens (TAAs), tumor-specific antigens (TSAs), viral antigens, autoantigens, and immune-cell surface molecules;
preferably, one of the two or more antigens is selected from tumor-associated antigens (TAAs), tumor-specific antigens (TSAs), viral antigens and autoantigens, and another is immune-cell surface molecules;
more preferably, the TAA is a melanoma-associated antigen (e.g., gp100, MAGEA1); and/or the TSA is a KRAS antigen (e.g., KRAS G12V, KRAS G12D); and/or the viral antigen is an HPV antigen (e.g., HPV E6 or E7); and/or the immune-cell surface molecule is selected from CD3, CD28, 4-1BB (CD137), PD-1, and PD-L1.

In some embodiments, the multi-specific antigen binding molecule does not comprise a TRAV having an amino acid sequence as shown in SEQ ID NO.24 and does not comprise a TRBV having an amino acid sequence as shown in SEQ ID NO.25. In some embodiments, the multi-specific antigen binding molecule does not comprise a TRAV having CDR1, CDR2, and CDR3 with amino acid sequences as shown in SEQ ID NOs.38-40, respectively, and does not comprise a TRBV having CDR1, CDR2, and CDR3 with amino acid sequences as shown in SEQ ID NOs.41-43, respectively. In some embodiments, the multi-specific antigen binding molecule does not bind the KRAS antigen epitope VVGAVGVGK or a complex thereof with HLA-A*11. In some embodiments, the multi-specific antigen binding molecule does not bind KRAS antigens.

In some embodiments, the multi-specific antigen binding molecule does not bind the gp100 antigen.

In some embodiments, the multi-specific antigen binding molecule comprises, on at least two polypeptide chains, a first binding region that binds a first antigen and a second binding region that binds a second antigen,
wherein the first binding region comprises an α chain variable region (TRAV) and a β chain variable region (TRBV) derived from a TCR that binds the first antigen-MHC complex;
wherein the second binding region comprises a heavy-chain variable region (VH) and a light-chain variable region (VL) derived from an antibody that binds the second antigen;
wherein the first antigen is selected from tumor-associated antigens (TAAs), tumor-specific antigens (TSAs), viral antigens and self-antigens, and the second antigen is immune-cell surface molecules;
and wherein the multi-specific antigen binding molecule further comprises at least one functional domain selected from the group consisting of:
   1) a hinge region derived from an antibody;
   2) an Fc domain of an antibody or a dimerization portion thereof;
   3) an CH3 domain derived from an antibody;
   4) an albumin (Alb) or a binding portion thereof;
   5) a TCR constant region or a fragment thereof.

In some embodiments, TRAC and TRBC are respectively connected to the C terminus of TRAV and TRBV via optional linkers; or TRAC and TRBC are respectively connected to the C terminus of TRBV and TRAV via optional linkers.

In some embodiments, the TRAC or TRBC of the present disclosure is not connected to the C terminus of VH or VL, e.g., whether via a linker or without a linker.

In some embodiments, the N terminus of the antibody is connected to the C terminus of the TCR.

In some embodiments, the functional domain is derived from an antibody hinge region, and/or an antibody Fc domain or a dimerization portion thereof, and/or an antibody CH3 domain, and/or a TCR constant region or a fragment thereof; and any two polypeptide chains of the multi-specific antigen binding molecule are connected via covalent or non-covalent interactions between the two chains of the functional domain;
preferably, the two chains of the functional domain are respectively connected to the C terminus or N terminus, preferably to the C terminus, of any two polypeptide chains of the multi-specific antigen binding molecule, via optional linkers; or
the first binding region and the second binding region are connected via covalent or non-covalent bonds between the two chains of the functional domain;
more preferably, the multi-specific antigen binding molecule comprises a first polypeptide chain and a second polypeptide chain, wherein
the first polypeptide chain, from the N-terminus to the C-terminus, comprises: TRBV-optional linker-TRBC-linker-VL-linker-VH-optional linker-hinge region-CH2-CH3, and the second polypeptide chain, from the N-terminus to the C-terminus, comprises: TRAV-optional linker-TRAC-optional linker-hinge region-CH2-CH3; or
the first polypeptide chain, from the N-terminus to the C-terminus, comprises: TRAV-optional linker-TRAC-linker-VL-linker-VH-optional linker-hinge region-CH2-CH3, and the second polypeptide chain, from the N-terminus to the C-terminus, comprises: TRBV-optional linker-TRBC-optional linker-hinge region-CH2-CH3; or
the first polypeptide chain, from the N-terminus to the C-terminus, comprises: TRBV-optional linker-TRBC-linker-VH-linker-VL-optional linker-hinge region-CH2-CH3, and the second polypeptide chain, from the N-terminus to the C-terminus, comprises: TRAV-optional linker-TRAC-optional linker-hinge region-CH2-CH3; or
the first polypeptide chain, from the N-terminus to the C-terminus, comprises: TRAV-optional linker-TRBC-linker-VH-linker-VL-optional linker-hinge region-CH2-CH3, and the second polypeptide chain, from the N-terminus to the C-terminus, comprises: TRBV-optional linker-TRBC-optional linker-hinge region-CH2-CH3; or
the multi-specific antigen binding molecule comprises a first polypeptide chain, a second polypeptide chain and a third polypeptide chain, wherein the first polypeptide chain, from the N-terminus to the C-terminus, comprises: TRBV-optional linker-TRBC-linker-VL, the second polypeptide chain, from the N-terminus to the C-terminus, comprises: VH-optional linker-hinge region-CH2-CH3, and the third polypeptide chain, from the N-terminus to the C-terminus, comprises: TRAV-optional linker-TRAC-optional linker-hinge region-CH2-CH3.

In some embodiments, the multi-specific antigen binding molecule does not comprise amino acid sequences as shown in SEQ ID NOs.92 and 93.

In some embodiments, the multi-specific antigen binding molecule does not comprise the following first polypeptide chain and second polypeptide chain: the first polypeptide chain, from the N terminus to the C terminus, comprises: TRAV-linker-VH-linker-TRAC, and the second polypeptide chain, from the N terminus to the C terminus, comprises: VL-linker-TRBV-TRBC.

In a twelfth aspect, the present disclosure provides a nucleic acid encoding the TCR α chain constant region, the TCR β chain constant region, the TCR constant region, the polypeptide molecule, the TCR, or the multi-specific antigen binding molecule as described herein.

In a thirteenth aspect, the present disclosure provides a vector comprising the nucleic acid as described herein.

In some embodiments, the vector is selected from lentiviral vectors, retroviral vectors, plasmids, DNA vectors, mRNA vectors, transposon-based vectors, and artificial chromosomes.

In a fourteenth aspect, the present disclosure provides a host-cell comprising the TCR α chain constant region, the TCR β chain constant region, the TCR constant region, the polypeptide molecule, the TCR, the multi-specific antigen binding molecule, the nucleic acid, or the vector as described herein.

In a fifteenth aspect, the present disclosure provides a conjugate comprising the polypeptide molecule, the TCR, or the multi-specific antigen binding molecule as described herein, and a chemical moiety conjugated to the polypeptide molecule, the TCR, or the multi-specific antigen binding molecule.

In some embodiments, the chemical moiety is selected from detectable labels, immunostimulatory molecules and therapeutic agents;
preferably, the detectable label is selected from biotins, streptavidin, enzymes or a catalytically active fragment thereof, radionuclides, nanoparticles, paramagnetic metal ions, nucleic-acid probes, contrast agents, fluorescents, phosphorescent and chemiluminescent molecules;
preferably, the immunostimulatory molecule is selected from the group consisting of cytokines, chemokines, platelet factors and complement activators;
preferably, the therapeutic agent is selected from the group consisting of immunomodulators, radioactive compounds, enzymes, chemotherapeutic agents and toxins.

In a sixteenth aspect, the present disclosure provides a composition comprising the polypeptide molecule, the TCR, the multi-specific antigen binding molecule, the nucleic acids, vector, the cell, or the conjugate as described herein, and a pharmaceutically acceptable carrier or excipient.

In some embodiments, the composition further comprises a second therapeutic agent; preferably, the second therapeutic agent is selected from the group consisting of antibodies, chemotherapeutic agents, and small-molecule drugs.

In a seventeenth aspect, the present disclosure provides a method of treating a disease in a subject, comprising administering to the subject an effective amount of the polypeptide molecule, the TCR, the multi-specific antigen binding molecule, the nucleic acid, the vector, the host-cell, the conjugate, or the compositions a described herein.

In some embodiments, the disease is selected from the group consisting of cancers, infectious diseases, autoimmune diseases, and inflammatory diseases. In some embodiments, the method further comprises administering a second therapeutic agent; preferably, the second therapeutic agent is selected from the group consisting of antibodies, chemotherapeutic agents, and small-molecule drugs.

In an eighteenth aspect, the present disclosure provides a TCR comprising a TCR α chain variable region (TRAV) and a TCR β chain variable region (TRBV), wherein the TRAV comprises α chain CDR1, CDR2, and CDR3 having amino acid sequences as shown in SEQ ID NO.56 (DSSMTY), SEQ ID NO.57 (IFAFESM) and SEQ ID NO.58 (AGSGGGTDK), respectively, or functional variants formed by insertion, deletion, or substitution of one or several amino acids; and/or the TRBV comprises β chain CDR1, CDR2, and CDR3 having amino acid sequences as shown in SEQ ID NO.59 (DFEATT), SEQ ID NO.60 (TDYGSKA) and SEQ ID NO.61 (SAREPGQGPWE), respectively, or functional variants formed by insertion, deletion, or substitution of one or several amino acids.

In some embodiments, the TRAV comprises α chain CDR1, CDR2, and CDR3 having amino acid sequences as shown in SEQ ID NO.56 (DSSMTY), SEQ ID NO.57 (IFAFESM) and SEQ ID NO.58 (AGSGGGTDK), respectively; and/or the TRBV comprises β chain CDR1, CDR2, and CDR3 having amino acid sequences as shown in SEQ ID NO.59 (DFEATT), SEQ ID NO.60 (TDYGSKA) and SEQ ID NO.61 (SAREPGQGPWE), respectively.

In some embodiments, the TRAV comprises an amino acid sequence having at least 80%, at least 85%, at least 90%, at least 95%, or 100% sequence identity with SEQ ID NO.30, and/or the TRBV comprises an amino acid sequence having at least 80%, at least 85%, at least 90%, at least 95%, or 100% sequence identity with SEQ ID NO.31.

In a nineteenth aspect, the present disclosure provides a TCR comprising a TCR α chain variable region (TRAV) and a TCR β chain variable region (TRBV), wherein the TRAV comprises α chain CDR1, CDR2, and CDR3 having amino acid sequences as shown in SEQ ID NO.62 (DSSMTY), SEQ ID NO.63 (IFFYQDM) and SEQ ID NO.64 (AGSGGGTDK), respectively, or functional variants formed by insertion, deletion, or substitution of one or several amino acids; and/or the TRBV comprises β chain CDR1, CDR2, and CDR3 having amino acid sequences as shown in SEQ ID NO.65 (DFEATT), SEQ ID NO.66 (TDYGSKA) and SEQ ID NO.67 (SAREPGQAWSD), respectively, or functional variants formed by insertion, deletion, or substitution of one or several amino acids.

In some embodiments, the TRAV comprises α chain CDR1, CDR2, and CDR3 having amino acid sequences as shown in SEQ ID NO.62 (DSSMTY), SEQ ID NO.63 (IFFYQDM) and SEQ ID NO.64 (AGSGGGTDK), respectively; and/or the TRBV comprises β chain CDR1, CDR2, and CDR3 having amino acid sequences as shown in SEQ ID NO.65 (DFEATT), SEQ ID NO.66 (TDYGSKA) and SEQ ID NO.67 (SAREPGQAWSD), respectively.

In some embodiments, the TRAV comprises an amino acid sequence having at least 80%, at least 85%, at least 90%, at least 95%, or 100% sequence identity with SEQ ID NO.32, and the TRBV comprises an amino acid sequence having at least 80%, at least 85%, at least 90%, at least 95%, or 100% sequence identity with SEQ ID NO.33.

### Brief Description of the Drawings

FIG. 1 shows the mechanism of action of a TCR-anti-CD3 antibody fusion protein.
FIG. 2 shows the principles for protein-stability design.
FIG. 3 shows the workflow for optimization of soluble expression of TCR fusion proteins.
FIG. 4 shows a TCR three-dimensional structure predicted by AFold2.

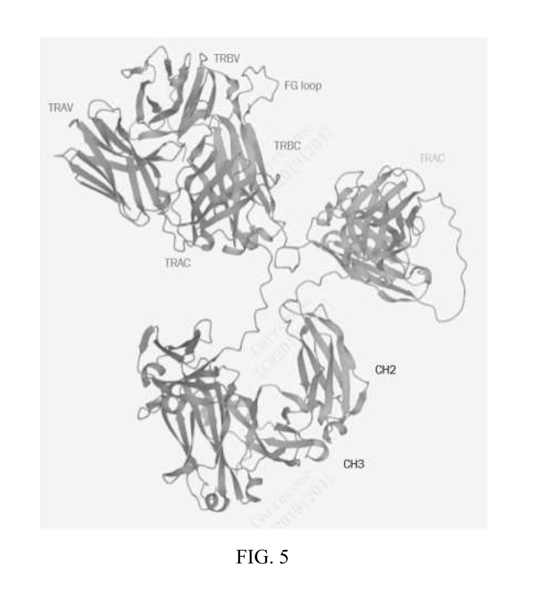

FIG. 5 shows a three-dimensional structure of a gp100-B60 TCR fusion protein predicted by AFold2.
FIG. 6 shows protein-protein interaction (PPI) sites of the α chain of the gp100-B60 TCR fusion protein predicted by ISPRED4.
FIG. 7 shows ELISPOT results for application of single-point mutants of the TCR constant region to format 62.
FIG. 8 shows a comparison between the activity gains predicted by single-parameter Stable scoring, single-parameter Soluble scoring, and dual-parameter Stable/Soluble scoring, and the activity gains verified by the ELISPOT assay.
FIG. 9 shows the expression of CR-KVA11-N03 TCR on the T-cell surface detected by flow cytometry.
FIG. 10 shows a three-dimensional structural model and mutation sites in the constant region of the CR-KVA11-N03 TCR predicted computer.
FIG. 11 shows SDS-PAGE results of CR-KVA11-N03-B62 molecules with different mutations.
FIG. 12 shows SDS-PAGE results and calculated protein yields for application of the CMM1 mutations to different TCR sequences and different TCR fusion-protein formats.
FIG. 13 shows ELISPOT results for application of the CMM1 mutations to different TCR sequences and different TCR fusion-protein formats.
FIG. 14 shows ELISPOT results for application of single-point mutants of the TCR constant region to format 22-2.
FIG. 15 shows SDS-PAGE results and calculated protein yields for application of CMM1 mutations and reverse mutations thereof (CMM2, CMM3, CMM4, and CMM5) to format 62.

### Detailed Description

Unless otherwise limited, all technical and scientific terms used herein have the meanings commonly understood by persons of ordinary skill in the art. e.g., the definitions of terms used herein are as set forth in: Janeway CA Jr., Travers P., Walport M., et al., "Immunobiology", 5th ed., New York: Garland Science (2001); and "A multilingual glossary of biotechnological terms: (IUPAC Recommendations)," edited by Leuenberger, H.G.W., Nagel, B., and Kölbl, H. (1995), Helvetica Chimica Acta, CH-4010 Basel, Switzerland.

It should be noted that, as used herein and in the appended claims, the singular forms "a," "an," and "said/the" include plural referents unless the context clearly dictates otherwise. Thus, the terms "a," "an,"one or more," and "at least one" may be used interchangeably. Likewise, the terms "comprise," "include," and "have" may be used interchangeably.

When the term "comprise" is used herein and in the appended claims, it does not exclude other elements. For the purposes of the present invention, the term "consist of" is considered to be a preferred embodiment of the term "comprise". If a group is defined below as including or comprising at least a certain number of embodiments, it is also to be understood as disclosing a group that preferably consists of only those embodiments.

By computer prediction and wet-lab experimental validation, the inventors have found that, relative to a parent TCR constant region, the modified TCR constant regions of the present invention, comprising amino acid substitutions at one or more of the positions, can improve soluble expression and in-vitro activity of TCR fusion proteins: Q6, S21, K83, S84.6, and I114 in TRAC; and S92, T94, and Q107 in TRBC, e.g., Q6R, S21I, K83P, S84.6D, S84.6E, S84.6N, I114E in TRAC; and S92P, S92E, S92D, S92L, S92N, T94D, Q107L in TRBC; wherein the amino acid positions are numbered according to the International Immunogenetics Information System (IMGT) numbering rules based on the TCR α chain constant region as shown in SEQ ID NO. 1 and the TCR β chain constant region as shown in SEQ ID NO. 14. Any of these single-point mutations and any combinations thereof can improve soluble expression and in-vitro activity of TCR fusion proteins.

The terms "parent TCR constant region," "parent sequence," "wild-type (WT) TCR constant region," and "wild-type (WT) sequence" are used interchangeably herein. In this context, TRAC comprises N1.2K, T84C, and an FFPSP deletion at positions 120-124 relative to human TRAC*01 as shown in SEQ ID NO.1, with amino acid sequence thereof as shown in SEQ ID NO.2; and TRBC comprises S79C, C85.1A, and N97D relative to human TRBC2*01 as shown in SEQ ID NO.14, with amino acid sequence thereof as shown in SEQ ID NO.15.

In addition, conservative substitutions of these mutated amino acids are also expected to improve soluble expression of TCR fusion proteins, because the amino acids chosen as conservative substitutions possess similarities in properties such as polarity, charge, solubility, hydrophobicity, hydrophilicity, and/or amphiphilicity. For example, conservative substitutions of amino acids may be selected with reference to the following table, preferably, conservative substitutions are chosen within the same block in the first column and, more preferably, conservative substitutions are chosen within the same row in the second column.

| | |
|---|---|
| Non-polar | P G A |
| | I L V |
| Polar uncharged | C S T M |
| | N Q |
| Polar charged | D E |
| | R K |

Accordingly, the modified TCR constant regions of the present invention may comprise one or more of the following amino acid substitutions Q6R, Q6K, Q6D, Q6E; S21I, S21L, S21V, S21G, S21A, S21P; K83P, K83G, K83A, K83I, K83L, K83V; S84.6D, S84.6E, S84.6N, S84.6Q, S84.6K, S84.6R; 1114E, 1114D, 1114N, 1114Q, I114K, 1114R; S92P, S92E, S92D, S92L, S92N, S92G, S92A, S92I, S92V, S92Q, S92K, S92R; T94D, T94E, T94N, T94Q, T94K, T94R; Q107L, Q107I, Q107V, Q107G, Q107A, Q107P.

Accordingly, in a first aspect, the present disclosure provides a T cell receptor (TCR) α chain constant region, wherein the α chain constant region (TRAC) comprises amino acid substitution(s) at one or more of the positions Q6, S21, K83, S84.6, and I114; wherein the amino acid positions are numbered according to the International Immunogenetics Information System (IMGT) numbering rules based on the TCR α chain constant region as shown in SEQ ID NO. 1.

In a natural form, the TCR exists as a complex of several proteins on the surface of T cells. The T cell receptor consists of two (separate) protein chains that are produced by separate T cell receptor alpha and beta (TCRα and TCRβ) genes and are referred to as alpha chain and beta chain. Each chain of the TCR has a\n N-terminal immunoglobulin-like (Ig)-variable (V) region/domain, an Ig-constant (C) region/domain, a transmembrane/cytomembrane spanning region that anchors the chain into the plasma membrane, and a C-terminal short cytoplasmic tail.

Typically, the TCR is a heterodimer of one α chain and one β chain, which can bind MHC molecules presenting antigenic peptides.

Antigen specificity is conferred by the variable regions ofα chain and β chain of TCR. Both variable regions of TCR α and β chains contain three highly variable or complementary determining regions (CDR1 α/β, CDR2α/β, and CDR3α/β) surrounded by framework (FR) regions. CDR3 is the major determinant for antigen recognition and specificity (i.e., the ability to recognize and interact with the specific antigen), whereas CDR1 and CDR2 interact primarily with MHC molecules that present antigenic peptides.

The TCR recognizes an antigenic peptide that binds to a major histocompatibility complex (MHC) molecule at the surface of the antigen-presenting cell ("presenting/exhibiting on the MHC molecule"). The antigenic peptide presented on the MHC molecule is also referred to herein as a "complex of epitope with MHC molecule", "epitope-MHC complex" or "target antigenic peptide-MHC complex". There are two different classes of MHC molecules: MHC I and MHC II, which present peptides from different cellular compartments. MHC class I molecules are expressed on the surface of all nucleated cells in the human and present peptides or protein fragments from intracellular compartments to cytotoxic T cells. In human, MHC is also known as human leukocyte antigen (HLA). There are three main types of MHC class I molecules: HLA-A, HLA-B, and HLA-C. Once the TCR binds to its specific epitope-MHC complex, the T cells are activated and exert a biological effector function.

Sequences of TCR constant regions can be found in the public IMGT database, e.g., the constant region sequence of the TCR α chain is "TRAC*01," and the constant region sequence of the TCR β chain is "TRBC1*01" or "TRBC2*01."

Amino acid positions of TCRs in the present invention are numbered according to the IMGT nomenclature. For example, positions in the amino acid sequences of the TCR α chain constant region (TRAC) and the TCR β chain constant region (TRBC) are numbered as listed in IMGT. For example, if an amino acid in the TRAC is designated with a position number of 84.6 in the IMGT, it is described herein as the amino acid at position 84.6 of the TRAC; if an amino acid in the TRBC is designated with a position number of 92 in the IMGT, it is described herein as the amino acid at position 92 of the TRBC; and so on. When the sequence position numbering of other amino acids is specifically described in the present invention, they are numbered as specifically described.

In some embodiments, the amino acid substitution at position Q6 of the α chain constant region is selected from Q6R, Q6K, Q6D, and Q6E, preferably selected from Q6R and Q6K.

In some embodiments, the amino acid substitution at position S21 of the α chain constant region is selected from S21I, S21L, S21V, S21G, S21A, and S21P, preferably selected from S21I, S21L, and S21V.

In some embodiments, the amino acid substitution at position K83 of the α chain constant region is selected from K83P, K83G, K83A, K83I, K83L, and K83V, preferably selected from K83P, K83G, and K83A.

In some embodiments, the amino acid substitution at position S84.6 of the α chain constant region is selected from S84.6D, S84.6E, S84.6N, S84.6Q, S84.6K, and S84.6R, preferably selected from S84.6D, S84.6E, S84.6N, and S84.6Q.

In some embodiments, the amino acid substitution at position I114 of the α chain constant region is selected from I114E, 1114D, 1114K, and 1114R, preferably selected from I114E and 1114D.

In some embodiments, the amino acid substitution at position Q6 of the α chain constant region is Q6R.

In some embodiments, the amino acid substitution at position S21 of the α chain constant region is S21I.

In some embodiments, the amino acid substitution at position K83 of the α chain constant region is K83P.

In some embodiments, the amino acid substitution at position S84.6 of the α chain constant region is selected from S84.6D, S84.6E, and S84.6N.

In some embodiments, the amino acid substitution at position I114 of the α chain constant region is 1114E.

In some embodiments, the α chain constant region comprises an amino acid substitution at any one position of Q6, S21, K83, S84.6, and I114.

In some embodiments, the α chain constant region comprises amino acid substitutions at any two positions of Q6, S21, K83, S84.6, and I114. In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114, or S84.6 and I114.

In some embodiments, the α chain constant region comprises amino acid substitutions at any three positions of Q6, S21, K83, S84.6, and I114. In some embodiments, the α chain constant region comprises amino acid substitutions at Q6, S21 and K83, Q6, S21 and S84.6, Q6, S21 and I114, Q6, K83 and S84.6, Q6, K83 and I114, Q6, S84.6 and I114, S21, K83 and S84.6, S21, K83 and I114, S21, S84.6 and I114, or K83, S84.6 and I114.

In some embodiments, the α chain constant region comprises amino acid substitutions at any four positions of Q6, S21, K83, S84.6, and I114. In some embodiments, the α chain constant region comprises amino acid substitutions at Q6, S21, K83 and S84.6, Q6, S21, K83 and I114, Q6, S21, S84.6 and I114, Q6, K83, S84.6 and I114, or S21, K83, S84.6 and I114.

In some embodiments, the α chain constant region comprises amino acid substitutions at positions Q6, S21, K83, S84.6, and I114.

In some embodiments, the α chain constant region comprises one or more of the amino acid substitutions S21I, K83P, and S84.6D; preferably comprises at least two amino acid substitutions of S21I, K83P, and S84.6D; more preferably comprises amino acid substitutions of S21I, K83P, and S84.6D.

In some embodiments, the α chain constant region comprises amino acid substitutions selected from any one of the following groups (1)-(4):
(1) S211, K83P and S84.6D;
(2) K83P and S84.6D;
(3) S21I and S84.6D;
(4) S21I and K83P.

In some embodiments, the α chain constant region comprises S21I, K83P, and S84.6D. In some embodiments, the α chain constant region comprises S21I and S84.6D.

In some embodiments, the α chain constant region further comprises one or more of the amino acid mutations N1.2K, T84C, and an FFPSP deletion at positions 120-124. In some preferred embodiments, the α chain constant region further comprises T84C and optionally N1.2K and/or a deletion of FFPSP at positions 120-124. In some more preferred embodiments, the α chain constant region further comprises N1.2K, T84C, and an FFPSP deletion at positions 120-124.

In some embodiments, the α chain constant region comprises an amino acid sequence having at least 80%, at least 85%, at least 90%, at least 91%, at least 92%, at least 93%, at least 94%, at least 95%, at least 96%, at least 97%, at least 98%, or at least 99% sequence identity to SEQ ID NO.1.

In some embodiments, the α chain constant region comprises an amino acid sequence having at least 80%, at least 85%, at least 90%, at least 91%, at least 92%, at least 93%, at least 94%, at least 95%, at least 96%, at least 97%, at least 98%, or at least 99% sequence identity to SEQ ID NO.2.

In some embodiments, the α chain constant region comprises an amino acid sequence as shown in any one of SEQ ID NOs.3-13 or an amino acid sequence having at least 80%, at least 85%, at least 90%, at least 95% or at least 99% sequence identity to any one of SEQ ID NOs.3-13.

As used herein, the term "sequence identity" indicates the extent to which two (nucleotide or amino acid) sequences have the same residues at the same positions in alignment, and is typically expressed as a percentage. Preferably, identity is determined over the overall length of the sequences being compared. Thus, two copies with the identical sequence have 100% identity, while sequences that are less highly conserved and have deletions, additions or substitutions may have a lower degree of identity. Those skilled in the art will recognize that a number of algorithms can be used to determine sequence identity using standard parameters, such as Blast (Altschul et al. (1997) Nucleic Acids Res. 25: 3389-3402), Blast2 (Altschul et al. (1990) J. Mol. Biol. 215: 403-410), Smith-Waterman (Smith et al. (1981) J. Mol. Biol. 147: 195-197), and ClustalW.

Thus, the amino acid sequence of SEQ ID NO. 3 may, for example, be a "subject sequence" or a "reference sequence", while a different amino acid sequence of TCR alpha or beta chain variable region may be a "query sequence".

In some embodiments, the α chain constant region comprises Q6R. In preferred embodiments, the α chain constant region comprises an amino acid sequence as shown in SEQ ID NO.3.

In some embodiments, the α chain constant region comprises S21I. In preferred embodiments, the α chain constant region comprises an amino acid sequence as shown in SEQ ID NO.4.

In some embodiments, the α chain constant region comprises K83P. In preferred embodiments, the α chain constant region comprises an amino acid sequence as shown in SEQ ID NO.5.

In some embodiments, the α chain constant region comprises S84.6D. In preferred embodiments, the α chain constant region comprises an amino acid sequence as shown in SEQ ID NO.6.

In some embodiments, the α chain constant region comprises S84.6E. In preferred embodiments, the α chain constant region comprises an amino acid sequence as shown in SEQ ID NO.7.

In some embodiments, the α chain constant region comprises S84.6N. In preferred embodiments, the α chain constant region comprises an amino acid sequence as shown in SEQ ID NO.8.

In some embodiments, the α chain constant region comprises I114E. In preferred embodiments, the α chain constant region comprises an amino acid sequence as shown in SEQ ID NO.9.

In some embodiments, the α chain constant region comprises K83P and S84.6D. In preferred embodiments, the α chain constant region comprises an amino acid sequence as shown in SEQ ID NO.10.

In some embodiments, the α chain constant region comprises S21I and S84.6D. In preferred embodiments, the α chain constant region comprises an amino acid sequence as shown in SEQ ID NO.11.

In some embodiments, the α chain constant region comprises S21I and K83P. In preferred embodiments, the α chain constant region comprises an amino acid sequence as shown in SEQ ID NO.12.

In some embodiments, the α chain constant region comprises S211, K83P, and S84.6D. In preferred embodiments, the α chain constant region comprises an amino acid sequence as shown in SEQ ID NO.13.

In a second aspect, the present disclosure provides a TCR α chain constant region comprising amino acid substitutions S21I, K83P, and S84.6D, wherein the amino acid positions are numbered according to the International Immunogenetics Information System (IMGT) numbering rules based on the TCR α chain constant region as shown in SEQ ID NO. 1.

In some preferred embodiments, the TCR α chain constant region comprises an amino acid sequence as shown in SEQ ID NO.13 or an amino acid sequence having at least 80%, at least 85%, at least 90%, at least 95%, or at least 99% sequence identity with SEQ ID NO.13.

In further preferred embodiments, the TCR α chain constant region comprises or consists of the amino acid sequence as shown in SEQ ID NO.13. Accordingly, the present disclosure provides a TCR α chain constant region comprising or consisting of the amino acid sequence as shown in SEQ ID NO.13.

In a third aspect, the present disclosure provides a TCR β chain constant region, wherein the β chain constant region comprises amino acid substitution(s) at one or more of the positions S92, T94 and Q107; wherein the amino acid positions are numbered according to the International Immunogenetics Information System (IMGT) numbering rules based on the TCR β constant region as shown in SEQ ID NO. 14.

In some embodiments, the amino acid substitution at position S92 of the β chain constant region is selected from S92P, S92E, S92D, S92L, S92N, S92G, S92A, S92I, S92V, S92Q, S92K and S92R, preferably selected from S92P, S92E, S92D, S92L, S92N, S92G, S92A, S92I, S92V and S92Q.

In some embodiments, the amino acid substitution at position T94 of the β chain constant region is T94D, T94E, T94K, or T94R, preferably selected from T94D and T94E.

In some embodiments, the amino acid substitution at position Q107 of the β chain constant region is Q107L, Q107I, Q107V, Q107G, Q107A and Q107P, preferably selected from Q107L, Q107I, and Q107V.

In some embodiments, the amino acid substitution at position S92 of the β chain constant region is selected from S92P, S92E, S92D, S92L, and S92N.

In some embodiments, the amino acid substitution at position T94 of the β chain constant region is T94D.

In some embodiments, the amino acid substitution at position Q107 of the β chain constant region is Q107L.

In some embodiments, the β chain constant region comprises amino acid substitutions at S92, T94, and Q107. In some embodiments, the β chain constant region comprises amino acid substitutions at T94 and Q107. In some embodiments, the β chain constant region comprises amino acid substitutions at S92 and T94. In some embodiments, the β chain constant region comprises amino acid substitutions at S92 and Q107. In some embodiments, the β chain constant region comprises an amino acid substitution at S92. In some embodiments, the β chain constant region comprises an amino acid substitution at T94. In some embodiments, the β chain constant region comprises an amino acid substitution at Q107.

In some embodiments, the β chain constant region comprises one or more of the amino acid substitutions T94D and Q107L; preferably comprises amino acid substitutions T94D and Q107L.

In some embodiments, the β chain constant region comprises amino acid substitution(s) selected from any one of the following groups (1)-(3):
(1) T94D and Q107L;
(2) Q107L;
(3) T94D.

In some embodiments, the β chain constant region comprises T94D and Q107L. In some embodiments, the β chain constant region comprises T94D. In some embodiments, the β chain constant region comprises Q107L.

In some embodiments, the TCR β chain constant region further comprises one or more of the amino acid mutations S79C, C85.1A, and N97D. In some preferred embodiments, the β chain constant region further comprises S79C and optionally C85.1A and/or N97D. In some more preferred embodiments, the TCR β chain constant region further comprises S79C, C85.1A, and N97D.

In some embodiments, the β chain constant region comprises an amino acid sequence having at least 80%, at least 85%, at least 90%, at least 91%, at least 92%, at least 93%, at least 94%, at least 95%, at least 96%, at least 97%, at least 98%, or at least 99% sequence identity with SEQ ID NO.14.

In some embodiments, the β chain constant region comprises an amino acid sequence having at least 80%, at least 85%, at least 90%, at least 91%, at least 92%, at least 93%, at least 94%, at least 95%, at least 96%, at least 97%, at least 98%, or at least 99% sequence identity with SEQ ID NO.15.

In some embodiments, the TCR β chain constant region comprises an amino acid sequence as shown in any one of SEQ ID NOs.16-23 or an amino acid sequence having at least 80%, at least 85%, at least 90%, at least 95%, or at least 99% sequence identity with any one of SEQ ID NOs.16-23.

In some embodiments, the β chain constant region comprises S92P. In preferred embodiments, the β chain constant region comprises an amino acid sequence as shown in SEQ ID NO.16.

In some embodiments, the β chain constant region comprises S92E. In preferred embodiments, the β chain constant region comprises an amino acid sequence as shown in SEQ ID NO.17.

In some embodiments, the β chain constant region comprises S92D. In preferred embodiments, the β chain constant region comprises an amino acid sequence as shown in SEQ ID NO.18.

In some embodiments, the β chain constant region comprises S92L. In preferred embodiments, the β chain constant region comprises an amino acid sequence as shown in SEQ ID NO.19.

In some embodiments, the β chain constant region comprises S92N. In preferred embodiments, the β chain constant region comprises an amino acid sequence as shown in SEQ ID NO.20.

In some embodiments, the β chain constant region comprises T94D. In preferred embodiments, the β chain constant region comprises an amino acid sequence as shown in SEQ ID NO.21.

In some embodiments, the β chain constant region comprises Q107L. In preferred embodiments, the β chain constant region comprises an amino acid sequence as shown in SEQ ID NO.22.

In some embodiments, the β chain constant region comprises T94D and Q107L. In preferred embodiments, the β chain constant region comprises an amino acid sequence as shown in SEQ ID NO.23.

In a fourth aspect, the present disclosure provides a TCR β chain constant region comprising amino acid substitutions T94D and Q107L, wherein the amino acid positions are numbered according to the International Immunogenetics Information System (IMGT) numbering rules based on the TCR β chain constant region as shown in SEQ ID NO. 14.

In preferred embodiments, the TCR β chain constant region comprises an amino acid sequence as shown in SEQ ID NO.23 or an amino acid sequence having at least 80%, at least 85%, at least 90%, at least 95%, or at least 99% sequence identity with SEQ ID NO.23.

In more preferred embodiments, the TCR β chain constant region comprises or consists of the amino acid sequence as shown in SEQ ID NO.23. Accordingly, the present disclosure provides a TCR β chain constant region comprising or consisting of the amino acid sequence as shown in SEQ ID NO.23.

In a fifth aspect, the present disclosure provides a TCR constant region comprising the TCR α chain constant region and the TCR β chain constant region as described herein.

It is to be understood that, having been apprised of the TCR α chain constant regions of the first aspect and the TCR β chain constant regions of the second aspect of the present disclosure, those skilled in the art will be able to combine any one of the TCR α chain constant regions disclosed in the first aspect with any one of the TCR β chain constant regions disclosed in the second aspect.

In some embodiments, the α chain constant region comprises an amino acid substitution at any one position of Q6, S21, K83, S84.6, and I114; and the β chain constant region comprises an amino acid substitution at S92.

In some embodiments, the α chain constant region comprises an amino acid substitution at any one position of Q6, S21, K83, S84.6, and I114; and the β chain constant region comprises an amino acid substitution at T94.

In some embodiments, the α chain constant region comprises an amino acid substitution at any one position of Q6, S21, K83, S84.6, and I114; and the β chain constant region comprises an amino acid substitution at Q107.

In some embodiments, the α chain constant region comprises an amino acid substitution at any one position of Q6, S21, K83, S84.6, and I114; and the β chain constant region comprises amino acid substitutions at S92 and T94.

In some embodiments, the α chain constant region comprises an amino acid substitution at any one position of Q6, S21, K83, S84.6, and I114; and the β chain constant region comprises amino acid substitutions at S92 and Q107.

In some embodiments, the α chain constant region comprises an amino acid substitution at any one position of Q6, S21, K83, S84.6, and I114; and the β chain constant region comprises amino acid substitutions at T94 and Q107.

In some embodiments, the α chain constant region comprises an amino acid substitution at any one position of Q6, S21, K83, S84.6, and I114; and the β chain constant region comprises amino acid substitutions at S92, T94, and Q107.

In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114, or S84.6 and I114; and the β chain constant region comprises an amino acid substitution at S92.

In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I11, 4 or S84.6 and I114; and the β chain constant region comprises an amino acid substitution at T94.

In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114, or S84.6 and I114; and the β chain constant region comprises an amino acid substitution at Q107.

In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114, or S84.6 and I114; and the β chain constant region comprises amino acid substitutions at S92 and T94.

In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114, or S84.6 and I114; and the β chain constant region comprises amino acid substitutions at S92 and Q107.

In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114, or S84.6 and I114; and the β chain constant region comprises amino acid substitutions at T94 and Q107.

In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114, or S84.6 and I114; and the β chain constant region comprises amino acid substitutions at S92, T94, and Q107.

In some embodiments, the α chain constant region comprises amino acid substitutions at Q6, S21 and K83, Q6, S21 and S84.6, Q6, S21 and I114, Q6, K83 and S84.6, Q6, K83 and I114, Q6, S84.6 and I114, S21, K83 and S84.6, S21, K83 and I114, S21, S84.6 and I114, or K83, S84.6 and I114; and the β chain constant region comprises an amino acid substitution at S92.

In some embodiments, the α chain constant region comprises amino acid substitutions at Q6, S21 and K83, Q6, S21 and S84.6, Q6, S21 and I114, Q6, K83 and S84.6, Q6, K83 and I114, Q6, S84.6 and I114, S21, K83 and S84.6, S21, K83 and I114, S21, S84.6 and I114, or K83, S84.6 and I114; and the β chain constant region comprises an amino acid substitution at T94.

In some embodiments, the α chain constant region comprises amino acid substitutions at Q6, S21 and K83, Q6, S21 and S84.6, Q6, S21 and I114, Q6, K83 and S84.6, Q6, K83 and I114, Q6, S84.6 and I114, S21, K83 and S84.6, S21, K83 and I114, S21, S84.6 and I114, or K83, S84.6 and I114; and the β chain constant region comprises an amino acid substitution at Q107.

In some embodiments, the α chain constant region comprises amino acid substitutions at Q6, S21 and K83, Q6, S21 and S84.6, Q6, S21 and I114, Q6, K83 and S84.6, Q6, K83 and I114, Q6, S84.6 and I114, S21, K83 and S84.6, S21, K83 and I114, S21, S84.6 and I114, or K83, S84.6 and I114; and the β chain constant region comprises amino acid substitutions at S92 and T94.

In some embodiments, the α chain constant region comprises amino acid substitutions at Q6, S21 and K83, Q6, S21 and S84.6, Q6, S21 and I114, Q6, K83 and S84.6, Q6, K83 and I114, Q6, S84.6 and I114, S21, K83 and S84.6, S21, K83 and I114, S21, S84.6 and I114, or K83, S84.6 and I114; and the β chain constant region comprises amino acid substitutions at S92 and Q107.

In some embodiments, the α chain constant region comprises amino acid substitutions at Q6, S21 and K83, Q6, S21 and S84.6, Q6, S21 and I114, Q6, K83 and S84.6, Q6, K83 and I114, Q6, S84.6 and I114, S21, K83 and S84.6, S21, K83 and I114, S21, S84.6 and I114, or K83, S84.6 and I114; and the β chain constant region comprises amino acid substitutions at T94 and Q107.

In some embodiments, the α chain constant region comprises amino acid substitutions at Q6, S21 and K83, Q6, S21 and S84.6, Q6, S21 and I114, Q6, K83 and S84.6, Q6, K83 and I114, Q6, S84.6 and I114, S21, K83 and S84.6, S21, K83 and I114, S21, S84.6 and I114, or K83, S84.6 and I114; and the β chain constant region comprises amino acid substitutions at S92, T94, and Q107.

In some embodiments, the α chain constant region comprises amino acid substitutions at Q6, S21, K83 and S84.6, Q6, S21, K83 and I114, Q6, S21, S84.6 and I114, Q6, K83, S84.6 and I114, or S21, K83, S84.6 and I114; and the β chain constant region comprises an amino acid substitution at S92.

In some embodiments, the α chain constant region comprises amino acid substitutions at Q6, S21, K83 and S84.6, Q6, S21, K83 and I114, Q6, S21, S84.6 and I114, Q6, K83, S84.6 and I114, or S21, K83, S84.6 and I114; and the β chain constant region comprises an amino acid substitution at T94.

In some embodiments, the α chain constant region comprises amino acid substitutions at Q6, S21, K83 and S84.6, Q6, S21, K83 and I114, Q6, S21, S84.6 and I114, Q6, K83, S84.6 and I114, or S21, K83, S84.6 and I114; and the β chain constant region comprises an amino acid substitution at Q107.

In some embodiments, the α chain constant region comprises amino acid substitutions at Q6, S21, K83 and S84.6, Q6, S21, K83 and I114, Q6, S21, S84.6 and I114, Q6, K83, S84.6 and I114, or S21, K83, S84.6 and I114; and the β chain constant region comprises amino acid substitutions at S92 and T94.

In some embodiments, the α chain constant region comprises amino acid substitutions at Q6, S21, K83 and S84.6, Q6, S21, K83 and I114, Q6, S21, S84.6 and I114, Q6, K83, S84.6 and I114, or S21, K83, S84.6 and I114; and the β chain constant region comprises amino acid substitutions at S92 and Q107.

In some embodiments, the α chain constant region comprises amino acid substitutions at Q6, S21, K83 and S84.6, Q6, S21, K83 and I114, Q6, S21, S84.6 and I114, Q6, K83, S84.6 and I114, or S21, K83, S84.6 and I114; and the β chain constant region comprises amino acid substitutions at T94 and Q107.

In some embodiments, the α chain constant region comprises amino acid substitutions at Q6, S21, K83 and S84.6, Q6, S21, K83 and I114, Q6, S21, S84.6 and I114, Q6, K83, S84.6 and I114, or S21, K83, S84.6 and I114; and the β chain constant region comprises amino acid substitutions at S92, T94, and Q107.

In some embodiments, the α chain constant region comprises amino acid substitutions at Q6, S21, K83, S84.6, and I114; and the β chain constant region comprises an amino acid substitution at S92.

In some embodiments, the α chain constant region comprises amino acid substitutions at Q6, S21, K83, S84.6, and I114; and the β chain constant region comprises an amino acid substitution at T94.

In some embodiments, the α chain constant region comprises amino acid substitutions at Q6, S21, K83, S84.6, and I114; and the β chain constant region comprises an amino acid substitution at Q107.

In some embodiments, the α chain constant region comprises amino acid substitutions at Q6, S21, K83, S84.6, and I114; and the β chain constant region comprises amino acid substitutions at S92 and T94.

In some embodiments, the α chain constant region comprises amino acid substitutions at Q6, S21, K83, S84.6, and I114; and the β chain constant region comprises amino acid substitutions at S92 and Q107.

In some embodiments, the α chain constant region comprises amino acid substitutions at Q6, S21, K83, S84.6, and I114; and the β chain constant region comprises amino acid substitutions at T94 and Q107.

In some embodiments, the α chain constant region comprises amino acid substitutions at Q6, S21, K83, S84.6, and I114; and the β chain constant region comprises amino acid substitutions at S92, T94, and Q107.

In some embodiments, the α chain constant region comprises one or more of the amino acid substitutions selected from S21I, K83P, and S84.6D; preferably comprises at least two of the amino acid substitutions selected from S21I, K83P, and S84.6D; more preferably comprises the amino acid substitutions S21I, K83P, and S84.6D. In some embodiments, the α chain constant region comprises S21I, K83P, and S84.6D. In some embodiments, the α chain constant region comprises S21I and S84.6D.

In some embodiments, the β chain constant region comprises one or more of the amino acid substitutions selected from T94D and Q107L; preferably comprises the amino acid substitutions T94D and Q107L. In some embodiments, the β chain constant region comprises T94D and Q107L. In some embodiments, the β chain constant region comprises T94D. In some embodiments, the β chain constant region comprises Q107L.

In some embodiments, the TCR comprises amino acid substitutions selected from any one of the following groups (1 )-(21):
(1) S21I, K83P, and S84.6D of the α chain constant region, and T94D and Q107L of the β chain constant region;
(2) K83P and S84.6D of the α chain constant region, and T94D and Q107L of the β chain constant region;
(3) S21I and S84.6D of the α chain constant region, and T94D and Q107L of the β chain constant region;
(4) S21I and K83P of the α chain constant region, and T94D and Q107L of the β chain constant region;
(5) S21I of the α chain constant region, and T94D and Q107L of the β chain constant region;
(6) K83P of the α chain constant region, and T94D and Q107L of the β chain constant region;
(7) S84.6D of the α chain constant region, and T94D and Q107L of the β chain constant region;
(8) S21I, K83P, and S84.6D of the α chain constant region, and Q107L of the β chain constant region;
(9) S21I, K83P, and S84.6D of the α chain constant region, and T94D of the β chain constant region;
(10) K83P and S84.6D of the α chain constant region, and T94D of the β chain constant region;
(11) K83P and S84.6D of the α chain constant region, and Q107L of the β chain constant region;
(12) S21I and S84.6D of the α chain constant region, and T94D of the β chain constant region;
(13) S21I and S84.6D of the α chain constant region, and Q107L of the β chain constant region;
(14) S21I and K83P of the α chain constant region, and T94D of the β chain constant region;
(15) S21I and K83P of the α chain constant region, and Q107L of the β chain constant region;
(16) S21I of the α chain constant region, and Q107L of the β chain constant region;
(17) S21I of the α chain constant region, and T94D of the β chain constant region;
(18) K83P of the α chain constant region, and T94D of the β chain constant region;
(19) K83P of the α chain constant region, and Q107L of the β chain constant region;
(20) S84.6D of the α chain constant region, and T94D of the β chain constant region;
(21) S84.6D of the α chain constant region, and Q107L of the β chain constant region.

In some embodiments, the α chain constant region further comprises one or more of the amino acid mutations N1.2K, T84C, and an FFPSP deletion at positions 120-124; the β chain constant region further comprises one or more of the amino acid mutations S79C, C85.1A, and N97D. In some preferred embodiments, the α chain constant region further comprises T84C, and optionally N1.2K and/or an FFPSP deletion at positions 120-124; the β chain constant region further comprises S79C, and optionally C85.1A and/or N97D. In some more preferred embodiments, the α chain constant region further comprises N1.2K, T84C, and an FFPSP deletion at positions 120-124, and the β chain constant region further comprises S79C, C85.1A, and N97D.

In some embodiments, the α chain constant region has at least 80%, at least 85%, at least 90%, at least 91%, at least 92%, at least 93%, at least 94%, at least 95%, at least 96%, at least 97%, at least 98%, or at least 99% sequence identity to SEQ ID NO.1, and the β chain constant region has at least 80%, at least 85%, at least 90%, at least 91%, at least 92%, at least 93%, at least 94%, at least 95%, at least 96%, at least 97%, at least 98%, or at least 99% sequence identity to SEQ ID NO.14.

In some embodiments, the α chain constant region has at least 80%, at least 85%, at least 90%, at least 91%, at least 92%, at least 93%, at least 94%, at least 95%, at least 96%, at least 97%, at least 98%, or at least 99% sequence identity to SEQ ID NO.2, and the β chain constant region has at least 80%, at least 85%, at least 90%, at least 91%, at least 92%, at least 93%, at least 94%, at least 95%, at least 96%, at least 97%, at least 98%, or at least 99% sequence identity to SEQ ID NO.15.

In some embodiments, the α chain constant region comprises an amino acid sequence as shown in any one of SEQ ID NOs.3-13 or an amino acid sequence having at least 80%, at least 85%, at least 90%, at least 95%, or at least 99% sequence identity to any one of SEQ ID NOs.3-13, and the β chain constant region comprises an amino acid sequence as shown in any one of SEQ ID NOs.16-23 or an amino acid sequence having at least 80%, at least 85%, at least 90%, at least 95%, or at least 99% sequence identity to any one of SEQ ID NOs.16-23. In some preferred embodiments, the TCR α chain constant region comprises an amino acid sequence as shown in SEQ ID NO.13 or an amino acid sequence having at least 80%, at least 85%, at least 90%, at least 95%, or at least 99% sequence identity to SEQ ID NO.13; and the TCR β chain constant region comprises an amino acid sequence as shown in SEQ ID NO.23 or an amino acid sequence having at least 80%, at least 85%, at least 90%, at least 95%, or at least 99% sequence identity to SEQ ID NO.23.

In some embodiments, the α chain constant region comprises S21I, K83P, and S84.6D, and the β chain constant region comprises T94D and Q107L. In preferred embodiments, the α chain constant region comprises an amino acid sequence as shown in SEQ ID NO.13, and the β chain constant region comprises an amino acid sequence as shown in SEQ ID NO.23.

In some embodiments, the α chain constant region comprises K83P and S84.6D, and the β chain constant region comprises T94D and Q107L. In preferred embodiments, the α chain constant region comprises an amino acid sequence as shown in SEQ ID NO.10, and the β chain constant region comprises an amino acid sequence as shown in SEQ ID NO.23.

In some embodiments, the α chain constant region comprises S21I and S84.6D, and the β chain constant region comprises T94D and Q107L. In preferred embodiments, the α chain constant region comprises an amino acid sequence as shown in SEQ ID NO.11, and the β chain constant region comprises an amino acid sequence as shown in SEQ ID NO.23.

In some embodiments, the α chain constant region comprises S211 and K83P, and the β chain constant region comprises T94D and Q107L. In preferred embodiments, the α chain constant region comprises an amino acid sequence as shown in SEQ ID NO.12, and the β chain constant region comprises an amino acid sequence as shown in SEQ ID NO.23.

In some embodiments, the α chain constant region comprises S21I, K83P, and S84.6D, and the β chain constant region comprises Q 107L. In preferred embodiments, the α chain constant region comprises an amino acid sequence as shown in SEQ ID NO.13, and the β chain constant region comprises an amino acid sequence as shown in SEQ ID NO.22.

In some embodiments, the α chain constant region comprises S21I, K83P, and S84.6D, and the β chain constant region comprises T94D. In preferred embodiments, the α chain constant region comprises an amino acid sequence as shown in SEQ ID NO.13, and the β chain constant region comprises an amino acid sequence as shown in SEQ ID NO.21.

In a sixth aspect, the present disclosure provides a polypeptide molecule comprising the TCR α chain constant region, the TCR β chain constant region, or the TCR constant region as described herein.

In some embodiments, the polypeptide molecule further comprises an antigen binding region that binds at least one antigen.

In some embodiments, the antigen binding region is selected from an antigen binding region derived from an antibody and an antigen binding region derived from a TCR. In some embodiments, the polypeptide molecule is a TCR. In some embodiments, the polypeptide molecule is a chimeric molecule in which a TCR constant region is fused to an antibody variable region. In some embodiments, the polypeptide molecule is a CAR.

In a seventh aspect, the present disclosure provides a T-cell receptor (TCR) comprising an α chain and a β chain, wherein the α chain comprises a TCR α chain constant region as described herein, and/or the β chain comprises a TCR β chain constant region as described herein.

In some embodiments, the TCR comprises an α chain and a β chain, and comprises a TCR constant region as described herein.

In an eighth aspect, the present disclosure provides a T-cell receptor (TCR) comprising an α chain and a β chain,
wherein the α chain constant region comprises amino acid substitution(s) at one or more of positions Q6, S21, K83, S84.6, and I114; and/or
wherein the β chain constant region comprises amino acid substitution(s) at one or more of positions S92, T94, and Q107;
wherein the amino acid positions are numbered according to the International Immunogenetics Information System (IMGT) numbering rules based on the TCR α chain constant region as shown in SEQ ID NO. 1 and the TCR β chain constant region as shown in SEQ ID NO. 14.

In some embodiments, the TCR comprises an α chain constant region having amino acid substitution(s) at one or more of positions Q6, S21, K83, S84.6, and I114. In some embodiments, the TCR comprises a β chain constant region having amino acid substitution(s) at one or more of positions S92, T94, and Q107. In some embodiments, the TCR comprises an α chain constant region having amino acid substitution(s) at one or more of positions Q6, S21, K83, S84.6, and I114, and a β chain constant region having amino acid substitution(s) at one or more of positions S92, T94, and Q107.

In some embodiments, the amino acid substitution at position Q6 of the α chain constant region is selected from Q6R, Q6K, Q6D, and Q6E, preferably selected from Q6R and Q6K.

In some embodiments, the amino acid substitution at position S21 of the α chain constant region is selected from S21I, S21L, S21V, S21G, S21A, and S21P, preferably selected from S21I, S21L, and S21V.

In some embodiments, the amino acid substitution at position K83 of the α chain constant region is selected from K83P, K83G, K83A, K83I, K83L, and K83V, preferably selected from K83P, K83G, and K83A.

In some embodiments, the amino acid substitution at position S84.6 of the α chain constant region is selected from S84.6D, S84.6E, S84.6N, S84.6Q, S84.6K, and S84.6R, preferably selected from S84.6D, S84.6E, S84.6N, and S84.6Q.

In some embodiments, the amino acid substitution at position I114 of the α chain constant region is I114E, 1114D, I114K, or I114R, preferably selected from I114E and I114D.

In some embodiments, the amino acid substitution at position S92 of the β chain constant region is selected from S92P, S92E, S92D, S92L, S92N, S92G, S92A, S92I, S92V, S92Q, S92K and S92R, preferably selected from S92P, S92E, S92D, S92L, S92N, S92G, S92A, S92I, S92V and S92Q.

In some embodiments, the amino acid substitution at position T94 of the β chain constant region is T94D, T94E, T94K, or T94R, preferably selected from T94D and T94E.

In some embodiments, the amino acid substitution at position Q107 of the β chain constant region is Q107L, Q107I, Q107V, Q107G, Q107A, or Q107P, preferably selected from Q107L, Q107I, and Q107V.

In some embodiments, the amino acid substitution at position Q6 of the α chain constant region is Q6R.

In some embodiments, the amino acid substitution at position S21 of the α chain constant region is S21I.

In some embodiments, the amino acid substitution at position K83 of the α chain constant region is K83P.

In some embodiments, the amino acid substitution at position S84.6 of the α chain constant region is selected from S84.6D, S84.6E, and S84.6N.

In some embodiments, the amino acid substitution at position I114 of the α chain constant region is 1114E.

In some embodiments, the amino acid substitution at position S92 of the β chain constant region is selected from S92P, S92E, S92D, S92L, and S92N.

In some embodiments, the amino acid substitution at position T94 of the β chain constant region is T94D.

In some embodiments, the amino acid substitution at position Q107 of the β chain constant region is Q107L.

In some embodiments, the α chain constant region comprises an amino acid substitution at any one of positions Q6, S21, K83, S84.6, and I114.

In some embodiments, the α chain constant region comprises amino acid substitutions at any two of positions Q6, S21, K83, S84.6, and I114. In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114 or S84.6 and I114.

In some embodiments, the α chain constant region comprises amino acid substitutions at any three of positions Q6, S21, K83, S84.6, and I114. In some embodiments, the α chain constant region comprises amino acid substitutions at Q6, S21 and K83, Q6, S21 and S84.6, Q6, S21 and I114, Q6, K83 and S84.6, Q6, K83 and I114, Q6, S84.6 and I114, S21, K83 and S84.6, S21, K83 and I114, S21, S84.6 and I114, or K83, S84.6 and I114.

In some embodiments, the α chain constant region comprises amino acid substitutions at any four of positions Q6, S21, K83, S84.6, and I114. In some embodiments, the α chain constant region comprises amino acid substitutions at Q6, S21, K83 and S84.6, Q6, S21, K83 and I114, Q6, S21, S84.6 and I114, Q6, K83, S84.6 and I114, or S21, K83, S84.6 and I114.

In some embodiments, the α chain constant region comprises amino acid substitutions at Q6, S21, K83, S84.6, and I114.

In some embodiments, the β chain constant region comprises an amino acid substitution at any one of positions S92, T94, and Q107.

In some embodiments, the β chain constant region comprises amino acid substitutions at any two of positions S92, T94, and Q107. In some embodiments, the β chain constant region comprises amino acid substitutions at S92 and T94. In some embodiments, the β chain constant region comprises amino acid substitutions at T94 and Q107. In some embodiments, the β chain constant region comprises amino acid substitutions at S92 and Q107.

In some embodiments, the β chain constant region comprises amino acid substitutions at S92, T94, and Q107.

In some embodiments, the α chain constant region comprises an amino acid substitution at any one of positions Q6, S21, K83, S84.6, and I114; and the β chain constant region comprises an amino acid substitution at S92.

In some embodiments, the α chain constant region comprises an amino acid substitution at any one of positions Q6, S21, K83, S84.6, and I114; and the β chain constant region comprises an amino acid substitution at T94.

In some embodiments, the α chain constant region comprises an amino acid substitution at any one of positions Q6, S21, K83, S84.6, and I114; and the β chain constant region comprises an amino acid substitution at Q107.

In some embodiments, the α chain constant region comprises an amino acid substitution at any one of positions Q6, S21, K83, S84.6, and I114; and the β chain constant region comprises amino acid substitutions at S92 and T94.

In some embodiments, the α chain constant region comprises an amino acid substitution at any one of positions Q6, S21, K83, S84.6, and I114; and the β chain constant region comprises amino acid substitutions at S92 and Q107.

In some embodiments, the α chain constant region comprises an amino acid substitution at any one of positions Q6, S21, K83, S84.6, and I114; and the β chain constant region comprises amino acid substitutions at T94 and Q107.

In some embodiments, the α chain constant region comprises an amino acid substitution at any one of positions Q6, S21, K83, S84.6, and I114; and the β chain constant region comprises amino acid substitutions at S92, T94, and Q107.

In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114, or S84.6 and I114; and the β chain constant region comprises an amino acid substitution at S92.

In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114, or S84.6 and I114; and the β chain constant region comprises an amino acid substitution at T94.

In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114, or S84.6 and I114; and the β chain constant region comprises an amino acid substitution at Q107.

In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114, or S84.6 and I114; and the β chain constant region comprises amino acid substitutions at S92 and T94.

In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114, or S84.6 and I114; and the β chain constant region comprises amino acid substitutions at S92 and Q107.

In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114, or S84.6 and I114; and the β chain constant region comprises amino acid substitutions at T94 and Q107.

In some embodiments, the α chain constant region comprises an amino acid substitution at Q6 and S21, Q6 and K83, Q6 and S84.6, Q6 and I114, S21 and K83, S21 and S84.6, S21 and I114, K83 and S84.6, K83 and I114, or S84.6 and I114; and the β chain constant region comprises amino acid substitutions at S92, T94, and Q107.

In some embodiments, the α chain constant region comprises amino acid substitutions at Q6, S21 and K83, Q6, S21 and S84.6, Q6, S21 and I114, Q6, K83 and S84.6, Q6, K83 and I114, Q6, S84.6 and I114, S21, K83 and S84.6, S21, K83 and I114, S21, S84.6 and I114, or K83, S84.6 and I114; and the β chain constant region comprises an amino acid substitution at S92.

In some embodiments, the α chain constant region comprises amino acid substitutions at Q6, S21 and K83, Q6, S21 and S84.6, Q6, S21 and I114, Q6, K83 and S84.6, Q6, K83 and I114, Q6, S84.6 and I114, S21, K83 and S84.6, S21, K83 and I114, S21, S84.6 and I114, or K83, S84.6 and I114; and the β chain constant region comprises an amino acid substitution at T94.

In some embodiments, the α chain constant region comprises amino acid substitutions at Q6, S21 and K83, Q6, S21 and S84.6, Q6, S21 and I114, Q6, K83 and S84.6, Q6, K83 and I114, Q6, S84.6 and I114, S21, K83 and S84.6, S21, K83 and I114, S21, S84.6 and I114, or K83, S84.6 and I114; and the β chain constant region comprises an amino acid substitution at Q107.

In some embodiments, the α chain constant region comprises amino acid substitutions at Q6, S21 and K83, Q6, S21 and S84.6, Q6, S21 and I114, Q6, K83 and S84.6, Q6, K83 and I114, Q6, S84.6 and I114, S21, K83 and S84.6, S21, K83 and I114, S21, S84.6 and I114, or K83, S84.6 and I114; and the β chain constant region comprises amino acid substitutions at S92 and T94.

In some embodiments, the α chain constant region comprises amino acid substitutions at Q6, S21 and K83, Q6, S21 and S84.6, Q6, S21 and I114, Q6, K83 and S84.6, Q6, K83 and I114, Q6, S84.6 and I114, S21, K83 and S84.6, S21, K83 and I114, S21, S84.6 and I114, or K83, S84.6 and I114; and the β chain constant region comprises amino acid substitutions at S92 and Q107.

In some embodiments, the α chain constant region comprises amino acid substitutions at Q6, S21 and K83, Q6, S21 and S84.6, Q6, S21 and I114, Q6, K83 and S84.6, Q6, K83 and I114, Q6, S84.6 and I114, S21, K83 and S84.6, S21, K83 and I114, S21, S84.6 and I114, or K83, S84.6 and I114; and the β chain constant region comprises amino acid substitutions at T94 and Q107.

In some embodiments, the α chain constant region comprises amino acid substitutions at Q6, S21 and K83, Q6, S21 and S84.6, Q6, S21 and I114, Q6, K83 and S84.6, Q6, K83 and I114, Q6, S84.6 and I114, S21, K83 and S84.6, S21, K83 and I114, S21, S84.6 and I114, or K83, S84.6 and I114; and the β chain constant region comprises amino acid substitutions at S92, T94, and Q107.

In some embodiments, the α chain constant region comprises amino acid substitutions at Q6, S21, K83 and S84.6, Q6, S21, K83 and I114, Q6, S21, S84.6 and I114, Q6, K83, S84.6 and I114, or S21, K83, S84.6 and I114; and the β chain constant region comprises an amino acid substitution at S92.

In some embodiments, the α chain constant region comprises amino acid substitutions at Q6, S21, K83 and S84.6, Q6, S21, K83 and I114, Q6, S21, S84.6 and I114, Q6, K83, S84.6 and I114, or S21, K83, S84.6 and I114; and the β chain constant region comprises an amino acid substitution at T94.

In some embodiments, the α chain constant region comprises amino acid substitutions at Q6, S21, K83 and S84.6, Q6, S21, K83 and I114, Q6, S21, S84.6 and I114, Q6, K83, S84.6 and I114, or S21, K83, S84.6 and I114; and the β chain constant region comprises an amino acid substitution at Q107.

In some embodiments, the α chain constant region comprises amino acid substitutions at Q6, S21, K83 and S84.6, Q6, S21, K83 and I114, Q6, S21, S84.6 and I114, Q6, K83, S84.6 and I114, or S21, K83, S84.6 and I114; and the β chain constant region comprises amino acid substitutions at S92 and T94.

In some embodiments, the α chain constant region comprises amino acid substitutions at Q6, S21, K83 and S84.6, Q6, S21, K83 and I114, Q6, S21, S84.6 and I114, Q6, K83, S84.6 and I114, or S21, K83, S84.6 and I114; and the β chain constant region comprises amino acid substitutions at S92 and Q107.

In some embodiments, the α chain constant region comprises amino acid substitutions at Q6, S21, K83 and S84.6, Q6, S21, K83 and I114, Q6, S21, S84.6 and I114, Q6, K83, S84.6 and I114, or S21, K83, S84.6 and I114; and the β chain constant region comprises amino acid substitutions at T94 and Q107.

In some embodiments, the α chain constant region comprises amino acid substitutions at Q6, S21, K83 and S84.6, Q6, S21, K83 and I114, Q6, S21, S84.6 and I114, Q6, K83, S84.6 and I114, or S21, K83, S84.6 and I114; and the β chain constant region comprises amino acid substitutions at S92, T94, and Q107.

In some embodiments, the α chain constant region comprises amino acid substitutions at Q6, S21, K83, S84.6, and I114; and the β chain constant region comprises an amino acid substitution at S92.

In some embodiments, the α chain constant region comprises amino acid substitutions at Q6, S21, K83, S84.6, and I114; and the β chain constant region comprises an amino acid substitution at T94.

In some embodiments, the α chain constant region comprises amino acid substitutions at Q6, S21, K83, S84.6, and I114; and the β chain constant region comprises an amino acid substitution at Q107.

In some embodiments, the α chain constant region comprises amino acid substitutions at Q6, S21, K83, S84.6, and I114; and the β chain constant region comprises amino acid substitutions at S92 and T94.

In some embodiments, the α chain constant region comprises amino acid substitutions at Q6, S21, K83, S84.6, and I114; and the β chain constant region comprises amino acid substitutions at S92 and Q107.

In some embodiments, the α chain constant region comprises amino acid substitutions at Q6, S21, K83, S84.6, and I114; and the β chain constant region comprises amino acid substitutions at T94 and Q107.

In some embodiments, the α chain constant region comprises amino acid substitutions at Q6, S21, K83, S84.6, and I114; and the β chain constant region comprises amino acid substitutions at S92, T94, and Q107.

In some embodiments, the α chain constant region comprises amino acid substitutions at Q6, S21, K83, S84.6, and I114, and/or the β chain constant region comprises amino acid substitutions at S92, T94, and Q107. In some embodiments, the α chain constant region comprises amino acid substitutions at S21, K83, and S84.6, and/or the β chain constant region comprises amino acid substitutions at T94 and Q107. In some embodiments, the α chain constant region comprises amino acid substitutions at S21, K83, S84.6, and I114, and/or the β chain constant region comprises amino acid substitutions at T94 and Q107. In some embodiments, the α chain constant region comprises amino acid substitutions at S21, K83, and S84.6, and/or the β chain constant region comprises amino acid substitutions at T94 and Q107. In some embodiments, the α chain constant region comprises amino acid substitutions at S21 and S84.6, and/or the β chain constant region comprises amino acid substitutions at T94 and Q107.

In some embodiments, the α chain constant region comprises one or more of the amino acid substitutions S21I, K83P, and S84.6D; preferably comprises at least two of the amino acid substitutions S21I, K83P, and S84.6D; and more preferably comprises the amino acid substitutions S21I, K83P, and S84.6D. In some embodiments, the α chain constant region comprises S21I, K83P, and S84.6D. In some embodiments, the α chain constant region comprises S21I and S84.6D.

In some embodiments, the β chain constant region comprises one or more of the amino acid substitutions T94D and Q107L; preferably comprises the amino acid substitutions T94D and Q107L. In some embodiments, the β chain constant region comprises T94D and Q107L. In some embodiments, the β chain constant region comprises T94D. In some embodiments, the β chain constant region comprises Q107L.

In some embodiments, the TCR comprises amino acid substitutions selected from any one of the following groups (1)-(21):
(1) S21I, K83P, and S84.6D of the α chain constant region, and T94D and Q107L of the β chain constant region;
(2) K83P and S84.6D of the α chain constant region, and T94D and Q107L of the β chain constant region;
(3) S21I and S84.6D of the α chain constant region, and T94D and Q107L of the β chain constant region;
(4) S21I and K83P of the α chain constant region, and T94D and Q107L of the β chain constant region;
(5) S21I of the α chain constant region, and T94D and Q107L of the β chain constant region;
(6) K83P of the α chain constant region, and T94D and Q107L of the β chain constant region;
(7) S84.6D of the α chain constant region, and T94D and Q107L of the β chain constant region;
(8) S21I, K83P, and S84.6D of the α chain constant region, and Q107L of the β chain constant region;
(9) S21I, K83P, and S84.6D of the α chain constant region, and T94D of the β chain constant region;
(10) K83P and S84.6D of the α chain constant region, and T94D of the β chain constant region;
(11) K83P and S84.6D of the α chain constant region, and Q107L of the β chain constant region;
(12) S21I and S84.6D of the α chain constant region, and T94D of the β chain constant region;
(13) S21I and S84.6D of the α chain constant region, and Q107L of the β chain constant region;
(14) S21I and K83P of the α chain constant region, and T94D of the β chain constant region;
(15) S21I and K83P of the α chain constant region, and Q107L of the β chain constant region;
(16) S21I of the α chain constant region, and Q107L of the β chain constant region;
(17) S21I of the α chain constant region, and T94D of the β chain constant region;
(18) K83P of the α chain constant region, and T94D of the β chain constant region;
(19) K83P of the α chain constant region, and Q107L of the β chain constant region;
(20) S84.6D of the α chain constant region, and T94D of the β chain constant region;
(21) S84.6D of the α chain constant region, and Q107L of the β chain constant region.

In some embodiments, the TCR comprises amino acid substitutions selected from any one of (1)-(21) above.

In some embodiments, the α chain constant region further comprises one or more of the amino acid mutations N1.2K, T84C, and an FFPSP deletion at positions 120-124; and/or the β chain constant region further comprises one or more of the amino acid mutations S79C, C85.1A, and N97D. In some preferred embodiments, the α chain constant region further comprises T84C and optionally N1.2K and/or an FFPSP deletion at positions 120-124; and/or the β chain constant region further comprises S79C and optionally C85.1A and/or N97D. In some more preferred embodiments, the α chain constant region further comprises N1.2K, T84C, and an FFPSP deletion at positions 120-124, and the β chain constant region further comprises S79C, C85.1A, and N97D.

In some embodiments, the α chain constant region has at least 80%, at least 85%, at least 90%, at least 91%, at least 92%, at least 93%, at least 94%, at least 95%, at least 96%, at least 97%, at least 98%, or at least 99% sequence identity to SEQ ID NO.1, and/or the β chain constant region has at least 80%, at least 85%, at least 90%, at least 91%, at least 92%, at least 93%, at least 94%, at least 95%, at least 96%, at least 97%, at least 98%, or at least 99% sequence identity to SEQ ID NO.14.

In some embodiments, the α chain constant region has at least 80%, at least 85%, at least 90%, at least 91%, at least 92%, at least 93%, at least 94%, at least 95%, at least 96%, at least 97%, at least 98%, or at least 99% sequence identity to SEQ ID NO.2, and/or the β chain constant region has at least 80%, at least 85%, at least 90%, at least 91%, at least 92%, at least 93%, at least 94%, at least 95%, at least 96%, at least 97%, at least 98%, or at least 99% sequence identity to SEQ ID NO.15.

In some embodiments, the TCR comprises an α chain constant region having an amino acid sequence as shown in any one of SEQ ID NOs.3-13 or an amino acid sequence having at least 80%, at least 85%, at least 90%, at least 95%, or at least 99% sequence identity to any one of SEQ ID NOs.3-13, and/or a β chain constant region having an amino acid sequence as shown in any one of SEQ ID NOs.16-23 or an amino acid sequence having at least 80%, at least 85%, at least 90%, at least 95%, or at least 99% sequence identity to any one of SEQ ID NOs.16-23.

In some embodiments, the α chain constant region comprises the amino acid substitutions S211, K83P, and S84.6D, and the β chain constant region comprises the amino acid substitutions T94D and Q107L. In preferred embodiments, the α chain constant region comprises an amino acid sequence as shown in SEQ ID NO.13, and the β chain constant region comprises an amino acid sequence as shown in SEQ ID NO.23.

In some embodiments, the α chain constant region comprises K83P and S84.6D, and the β chain constant region comprises T94D and Q107L. In preferred embodiments, the α chain constant region comprises an amino acid sequence as shown in SEQ ID NO.10, and the β chain constant region comprises an amino acid sequence as shown in SEQ ID NO.23.

In some embodiments, the α chain constant region comprises S21I and S84.6D, and the β chain constant region comprises T94D and Q107L. In preferred embodiments, the α chain constant region comprises an amino acid sequence as shown in SEQ ID NO.11, and the β chain constant region comprises an amino acid sequence as shown in SEQ ID NO.23.

In some embodiments, the α chain constant region comprises S211 and K83P, and the β chain constant region comprises T94D and Q107L. In preferred embodiments, the α chain constant region comprises an amino acid sequence as shown in SEQ ID NO.12, and the β chain constant region comprises an amino acid sequence as shown in SEQ ID NO.23.

In some embodiments, the α chain constant region comprises S21I, K83P, and S84.6D, and the β chain constant region comprises Q107L. In preferred embodiments, the α chain constant region comprises an amino acid sequence as shown in SEQ ID NO.13, and the β chain constant region comprises an amino acid sequence as shown in SEQ ID NO.22.

In some embodiments, the α chain constant region comprises S21I, K83P, and S84.6D, and the β chain constant region comprises T94D. In preferred embodiments, the α chain constant region comprises an amino acid sequence as shown in SEQ ID NO.13, and the β chain constant region comprises an amino acid sequence as shown in SEQ ID NO.21.

In some embodiments, the TCR binds an antigen selected from a group consisting of tumor-associated antigens (TAA), tumor-specific antigens (TSA), viral antigens, and self-antigens.

In some embodiments, the TCR binds a TAA selected from a group consisting of melanoma-associated antigens (e.g., gp100, MAGEA1, MAGEA3, MAGEA6, MAGEA4, MAGEA2, MAGEA12, MAGEA2B, MAGEA9B, MAGEA10, MAGEA11, MAGEB2, MAGEC1, MAGEC2), IGF2BP1, GNGT1, PI4K2B, CCR8, NPSR1, COX7B2, ONECUT3, SMC1B, FOXI3, GAGE2A, FBXO43, BRDT, PAGE2, GAGE13, POU5F1B, CTAG1A, and endogenous retroviral antigens.

In some embodiments, the TCR binds a TSA selected from a group consisting of from KRAS mutant antigens (e.g., KRAS G12 mutant antigens, such as G12D, G12V, G12C, G12R, G12A; G13D; Q61H; G125), TP53 (e.g., R175H, R173H, R273C, R248W, R248Q, R282W, Y220C, V157F, G245S, Y163C, R249S), PIK3CA (e.g., E542K, E545K, H1047R), CTNNB1 (e.g., S45P, T41A), EGFR (e.g., L858R, T790M), BRAF (e.g., V600E), and GNAS (e.g., R201C, R201H).

In some embodiments, the TCR binds a viral antigen selected from a group consisting of HPV antigens (e.g., HPV E6 or E7 antigens), CMV antigens, HBV antigens, EBV antigens, herpesvirus antigens, human immunodeficiency virus (HIV) antigens, influenza virus antigens, and coronavirus antigens.

In some embodiments, the TCR binds a self-antigen selected from a group consisting of AFP, CEA, CD19, CD20, BCMA, CD22, CD30, SLAM, CLDN18.2, GD2, mesothelin, CD38, Her2, GPC3, MUC1, Ro52, Ro60, La, Jo-1, SRP, IFIH1, CENPA, CENPB, SNRPA1, SNRNP70, SNR-PD3, RNAP3, TOPO1, Insulin, GAD65, IA2, Znt8, PL7, TARS, ARS, MI2, topoisomerase 1, EXOSC9, EXOSC107, POLR3A, POLR3K, PTRN, GAD2, SLC30A8, AchR, MUSK, LRP4, PLA2R, THSD7A, TSHR, IFN-γ, CHRNA1, MUSK, LRP4, AQP4, MOG, GRIN1, COL4A3, PLA2R, GM-CSF, PR3 and MPO.

In preferred embodiments, the TCR binds an antigen selected from a group consisting of melanoma-associated antigens (e.g., gp100 and MAGEA1), HPV antigens (e.g., HPV E6 or E7), and KRAS antigens (e.g., KRAS G12V and KRAS G12D).

In some embodiments, the TCR binds KRAS G12V and comprises a TCR α chain variable region (TRAV) and a TCR β chain variable region (TRBV) that bind KRAS G12V. In some embodiments, the TRAV comprises α chain CDR1, CDR2, and CDR3 having amino acid sequences as shown in SEQ ID NO.38 (NSASQS), SEQ ID NO.39 (VYSSGN) and SEQ ID NO.40 (VVPGGTGGGNKLT), respectively, and the TRBV comprises β chain CDR1, CDR2, and CDR3 having amino acid sequences as shown in SEQ ID NO.41 (LGHDT), SEQ ID NO.42 (YNNKEL) and SEQ ID NO.43 (ASSHWGAQETQY), respectively. In some embodiments, the TRAV comprises an amino acid sequence having at least 80%, at least 85%, at least 90%, at least 95%, or 100% sequence identity to SEQ ID NO.24, and the TRBV comprises an amino acid sequence having at least 80%, at least 85%, at least 90%, at least 95%, or 100% sequence identity to SEQ ID NO.25. In some embodiments, the TRAV comprises α chain CDR1, CDR2, and CDR3 having amino acid sequences as shown in SEQ ID NO.44 (NSASDY), SEQ ID NO.45 (IRSNMDK) and SEQ ID NO.46 (AENSDGTSYGKLT), respectively, and the TRBV comprises β chain CDR1, CDR2, and CDR3 having amino acid sequences as shown in SEQ ID NO.47 (SGHAT), SEQ ID NO.48 (FQNNGV) and SEQ ID NO.49 (ASSLVGSPDYEQY), respectively. In some embodiments, the TRAV comprises an amino acid sequence having at least 80%, at least 85%, at least 90%, at least 95%, or 100% sequence identity to SEQ ID NO.26, and the TRBV comprises an amino acid sequence having at least 80%, at least 85%, at least 90%, at least 95%, or 100% sequence identity to SEQ ID NO.27. In some embodiments, the TRAV comprises α chain CDR1, CDR2, and CDR3 having amino acid sequences as shown in SEQ ID NO.50 (VSGNPY), SEQ ID NO.51 (YITGDNLV) and SEQ ID NO.52 (AVRDIEGAGNNRKLI), respectively, and the TRBV comprises β chain CDR1, CDR2, and CDR3 having amino acid sequences as shown in SEQ ID NO.53 (SGHNS), SEQ ID NO.54 (FNNNVP) and SEQ ID NO.55 (ASSEGFYTEAF), respectively. In some embodiments, the TRAV comprises an amino acid sequence having at least 80%, at least 85%, at least 90%, at least 95%, or 100% sequence identity to SEQ ID NO.28, and the TRBV comprises an amino acid sequence having at least 80%, at least 85%, at least 90%, at least 95%, or 100% sequence identity to SEQ ID NO.29.

In some embodiments, the TCR binds KRAS G12D and comprises a TCR α chain variable region (TRAV) and a TCR β chain variable region (TRBV) that bind KRAS G12D. In some embodiments, the TRAV comprises α chain CDR1, CDR2, and CDR3 having amino acid sequences as shown in SEQ ID NO.78 (NSASQS), SEQ ID NO.79 (VYSSGN) and SEQ ID NO.80 (VVTNARLM), respectively, and the TRBV comprises β chain CDR1, CDR2, and CDR3 having amino acid sequences as shown in SEQ ID NO.81 (MNHEY), SEQ ID NO.82 (SMNVEV) and SEQ ID NO.83 (ASTSIVSSGRGEQF), respectively. In some embodiments, the TRAV comprises an amino acid sequence having at least 80%, at least 85%, at least 90%, at least 95%, or 100% sequence identity to SEQ ID NO.34, and the TRBV comprises an amino acid sequence having at least 80%, at least 85%, at least 90%, at least 95%, or 100% sequence identity to SEQ ID NO.35. In some embodiments, the TRAV comprises α chain CDR1, CDR2, and CDR3 having amino acid sequences as shown in SEQ ID NO.84 (DRGSQS), SEQ ID NO.85 (IYSNGD) and SEQ ID NO.86 (AVNLGAAGNKLT), respectively, and the TRBV comprises β chain CDR1, CDR2, and CDR3 having amino acid sequences as shown in SEQ ID NO.87 (SGHAT), SEQ ID NO.88 (FQNNGV) and SEQ ID NO.89 (ASSFSDSYNSPLH), respectively. In some embodiments, the TRAV comprises an amino acid sequence having at least 80%, at least 85%, at least 90%, at least 95%, or 100% sequence identity to SEQ ID NO.90 (QKEVEQNSGPLSVPEGAIASLNCTYSDRGSQSFFWYRQYSGKSPELIMSIYSNGDKEDGRFTAQLNKA SQYVSLLIRDSQPSDSATYLCAVNLGAAGNKLTFGGGTRVLVKP), and the TRBV comprises an amino acid sequence having at least 80%, at least 85%, at least 90%, at least 95%, or 100% sequence identity to SEQ ID NO.91 (EAGVAQSPRYKIIEKRQSVAFWCNPISGHATLYWYQQILGQGPKLLIQFQNNGVVDDSQLPKDRFSAE RLKGVDSTLKIQPAKLEDSAVYLCASSFSDSYNSPLHFGNGTRLTVT).

In some embodiments, the TCR binds MAGEA1 and comprises a TCR α chain variable region (TRAV) and a TCR β chain variable region (TRBV) that bind MAGEA1. In some embodiments, the TRAV comprises α chain CDR1, CDR2 and CDR3 having amino acid sequences as shown in SEQ ID NO.56 (DSSMTY), SEQ ID NO.57 (IFAFESM) and SEQ ID NO.58 (AGSGGGTDK), respectively, and the TRBV comprises β chain CDR1, CDR2 and CDR3 having amino acid sequences as shown in SEQ ID NO.59 (DFEATT), SEQ ID NO.60 (TDYGSKA) and SEQ ID NO.61 (SAREPGQGPWE), respectively. In some embodiments, the TRAV comprises an amino acid sequence having at least 80%, at least 85%, at least 90%, at least 95% or 100% sequence identity to SEQ ID NO.30, and the TRBV comprises an amino acid sequence having at least 80%, at least 85%, at least 90%, at least 95% or 100% sequence identity to SEQ ID NO.31. In some embodiments, the TRAV comprises α chain CDR1, CDR2 and CDR3 having amino acid sequences as shown in SEQ ID NO.62 (DSSMTY), SEQ ID NO.63 (IFFYQDM) and SEQ ID NO.64 (AGSGGGTDK), respectively, and the TRBV comprises β chain CDR1, CDR2 and CDR3 having amino acid sequences as shown in SEQ ID NO.65 (DFEATT), SEQ ID NO.66 (TDYGSKA) and SEQ ID NO.67 (SAREPGQAWSD), respectively. In some embodiments, the TRAV comprises an amino acid sequence having at least 80%, at least 85%, at least 90%, at least 95% or 100% sequence identity to SEQ ID NO.32, and the TRBV comprises an amino acid sequence having at least 80%, at least 85%, at least 90%, at least 95% or 100% sequence identity to SEQ ID NO.33. In some embodiments, the TRAV comprises an amino acid sequence having at least 80%, at least 85%, at least 90%, at least 95% or 100% sequence identity to SEQ ID NO.68 (GEDVEQSLFLSVREGDSSVINCTYTDSSSTYLYWYKQEPGAGLQLLTYIFSNMDMKQDQRLTVLLNK KDKHLSLRIADTQTGDSAIYFCAGSGGGTDKLIFGTGTRLQVFPN), and the TRBV comprises an amino acid sequence having at least 80%, at least 85%, at least 90%, at least 95% or 100% sequence identity to SEQ ID NO.69 (GAVVSQHPSWVICKSGTSVKIECRSLDFQATTMFWYRQFPKQSLMLMATSDYQTCVTYEQGVEKDK FLINHASLTLSTLTVTSAHPEDSSFYICSAREPGQGPFEQYFGPGTRLTVTE).

In some embodiments, the TCR binds HPV E7 and comprises a TCR α chain variable region (TRAV) and a TCR β chain variable region (TRBV) that bind HPV E7. In some embodiments, the TRAV comprises α chain CDR1, CDR2 and CDR3 having amino acid sequences as shown in SEQ ID NO.70 (NSASQS), SEQ ID NO.71 (VYSSGN) and SEQ ID NO.72 (AVISAGTALI), respectively, and the TRBV comprises β chain CDR1, CDR2 and CDR3 having amino acid sequences as shown in SEQ ID NO.73 (SGHDT), SEQ ID NO.74 (YYEEEE) and SEQ ID NO.75 (ASSLGWRGGLYTEAF), respectively. In some embodiments, the TRAV comprises an amino acid sequence having at least 80%, at least 85%, at least 90%, at least 95% or 100% sequence identity to SEQ ID NO.76 (RKEVEQDPGPFNVPEGATVAFNCTYSNSASQSFFWYRQDCRKEPKLLMSVYSSGNEDGRFTAQLNR ASQYISLLIRDSKLSDSATYLCAVISAGTALIFGKGTTLSVSS), and the TRBV comprises an amino acid sequence having at least 80%, at least 85%, at least 90%, at least 95% or 100% sequence identity to SEQ ID NO.77 (DAGVTQSPTHLIKTRGQQVTLRCSPKSGHDTVSWYQQALGQGPQFIFQYYEEEERQRGNFPDRFSGH QFPNYSSELNVNALLLGDSALYLCASSLGWRGGLYTEAFFGQGTRLTVV).

In some embodiments, the TCR binds gp100 and comprises a TCR α chain variable region (TRAV) and a TCR β chain variable region (TRBV) that bind gp100. In some embodiments, the TRAV comprises an amino acid sequence having at least 80%, at least 85%, at least 90%, at least 95% or 100% sequence identity to SEQ ID NO.94, and the TRBV comprises an amino acid sequence having at least 80%, at least 85%, at least 90%, at least 95% or 100% sequence identity to SEQ ID NO.95.

In some embodiments, the TCR does not comprise a TRAV having an amino acid sequence as shown in SEQ ID NO.24 and does not comprise a TRBV having an amino acid sequence as shown in SEQ ID NO.25. In some embodiments, the TCR does not comprise a TRAV having CDR1, CDR2 and CDR3 with amino acid sequences as shown in SEQ ID NOs.38-40, respectively, and does not comprise a TRBV having CDR1, CDR2 and CDR3 with amino acid sequences as shown in SEQ ID NOs.41-43, respectively. In some embodiments, the TCR does not bind a KRAS antigen epitope comprising VVGAVGVGK or a complex thereof with HLA-A*11. In some embodiments, the TCR does not bind a KRAS antigen.

In some embodiments, the TCR does not comprise a TRAV having an amino acid sequence as shown in SEQ ID NO.94 and does not comprise a TRBV having an amino acid sequence as shown in SEQ ID NO.95. In some embodiments, the TCR does not bind gp 100.

In some embodiments, the TCR comprises variable regions derived from a mouse TCR or a human TCR.

In some embodiments, the TCR is isolated or purified.

As used herein, the term "isolated or purified" means that the "isolated or purified" antigen binding protein has been identified, isolated and/or recovered from components of the environment in which it is produced, such that the "isolated or purified" antigen binding protein is free or substantially free of other contaminant components from the environment in which it is produced that may interfere with its therapeutic or diagnostic use. Contaminant components may include enzymes, hormones, and other proteins or non-protein solutes. Thus, the "isolated or purified" antigen binding protein may be prepared by at least one purification step that removes or substantially removes these contaminant components.

In a ninth aspect, the present disclosure provides a multi-specific antigen binding molecule comprising the TCR α chain constant region (TRAC), the TCR β chain constant region (TRBC), the TCR constant region, the polypeptide molecule, or the TCR as described herein.

In a tenth aspect, the present disclosure provides a multi-specific antigen binding molecule comprising at least one antigen binding region derived from a TCR and any one selected from the following groups (1)-(2):
(1) the TCR α chain constant region (TRAC) as described herein and/or the TCR β chain constant region (TRBC) as described herein;
(2) the TCR constant region as described herein.

In some embodiments, the multi-specific antigen binding molecule comprises the TCR as described herein.

In an eleventh aspect, the present disclosure provides a multi-specific antigen binding molecule comprising at least one antigen binding region derived from a TCR and a TCR-derived α chain constant region (TRAC) and/or a TCR-derived β chain constant region (TRBC);
wherein the α chain constant region comprises amino acid substitution(s) at one or more of the positions Q6, S21, K83, S84.6 and I114; and/or
wherein the β chain constant region comprises amino acid substitution(s) at one or more of the positions S92, T94 and Q107;
wherein the amino acid positions are numbered according to the International Immunogenetics Information System (IMGT) numbering rules based on the TCR α chain constant region as shown in SEQ ID NO. 1 and the TCR β chain constant region as shown in SEQ ID NO. 14.

In some embodiments, the multi-specific antigen binding molecule comprises an α chain constant region having amino acid substitution(s) at one or more of the positions Q6, S21, K83, S84.6 and I114. In some embodiments, the multi-specific antigen binding molecule comprises a β chain constant region having amino acid substitution(s) at one or more of the positions S92, T94 and Q107. In some embodiments, the multi-specific antigen binding molecule comprises an α chain constant region having amino acid substitution(s) at one or more of the positions Q6, S21, K83, S84.6 and I114, and a β chain constant region having amino acid substitution(s) at one or more of the positions S92, T94 and Q107.

In some embodiments, the amino acid substitution at position Q6 of the α chain constant region is selected from Q6R, Q6K, Q6D and Q6E, and is preferably selected from Q6R and Q6K.

In some embodiments, the amino acid substitution at position S21 of the α chain constant region is selected from S21I, S21L, S21V, S21G, S21A and S21P, and is preferably selected from S21I, S21L and S21V.

In some embodiments, the amino acid substitution at position K83 of the α chain constant region is selected from K83P, K83G, K83A, K83I, K83L and K83V, and is preferably selected from K83P, K83G and K83A.

In some embodiments, the amino acid substitution at position S84.6 of the α chain constant region is selected from S84.6D, S84.6E, S84.6N, S84.6Q, S84.6K and S84.6R, and is preferably selected from S84.6D, S84.6E, S84.6N and S84.6Q.

In some embodiments, the amino acid substitution at position I114 of the α chain constant region is selected from I114E, 1114D, 1114K or I114R, and is preferably selected from 1114E and 1114D.

In some embodiments, the amino acid substitution at position S92 of the β chain constant region is selected from S92P, S92E, S92D, S92L, S92N, S92G, S92A, S92I, S92V, S92Q, S92K and S92R, and is preferably selected from S92P, S92E, S92D, S92L, S92N, S92G, S92A, S92I, S92V and S92Q.

In some embodiments, the amino acid substitution at position T94 of the β chain constant region is selected from T94D, T94E, T94K or T94R, and is preferably selected from T94D and T94E.

In some embodiments, the amino acid substitution at position Q107 of the β chain constant region is selected from Q107L, Q107I, Q107V, Q107G, Q107A and Q107P, and is preferably selected from Q107L, Q107I and Q107V.

In some embodiments, the amino acid substitution at position Q6 of the α chain constant region is Q6R.

In some embodiments, the amino acid substitution at position S21 of the α chain constant region is S21I.

In some embodiments, the amino acid substitution at position K83 of the α chain constant region is K83P.

In some embodiments, the amino acid substitution at position S84.6 of the α chain constant region is selected from S84.6D, S84.6E and S84.6N.

In some embodiments, the amino acid substitution at position I114 of the α chain constant region is 1114E.

In some embodiments, the amino acid substitution at position S92 of the β chain constant region is selected from S92P, S92E, S92D, S92L and S92N.

In some embodiments, the amino acid substitution at position T94 of the β chain constant region is T94D.

In some embodiments, the amino acid substitution at position Q107 of the β chain constant region is Q107L.

In some embodiments, the α chain constant region comprises an amino acid substitution at any one of positions Q6, S21, K83, S84.6 and I114.

In some embodiments, the α chain constant region comprises amino acid substitutions at any two of positions Q6, S21, K83, S84.6 and I114. In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114 or S84.6 and I114.

In some embodiments, the α chain constant region comprises amino acid substitutions at any three of positions Q6, S21, K83, S84.6 and I114. In some embodiments, the α chain constant region comprises amino acid substitutions at Q6, S21 and K83, Q6, S21 and S84.6, Q6, S21 and I114, Q6, K83 and S84.6, Q6, K83 and I114, Q6, S84.6 and I114, S21, K83 and S84.6, S21, K83 and I114, S21, S84.6 and I114, or K83, S84.6 and I114.

In some embodiments, the α chain constant region comprises amino acid substitutions at any four of positions Q6, S21, K83, S84.6 and I114. In some embodiments, the α chain constant region comprises amino acid substitutions at Q6, S21, K83 and S84.6, Q6, S21, K83 and I114, Q6, S21, S84.6 and I114, Q6, K83, S84.6 and I114, or S21, K83, S84.6 and I114.

In some embodiments, the α chain constant region comprises amino acid substitutions at Q6, S21, K83, S84.6 and I114.

In some embodiments, the β chain constant region comprises an amino acid substitution at any one of positions S92, T94 and Q107.

In some embodiments, the β chain constant region comprises amino acid substitutions at any two of positions S92, T94 and Q107. In some embodiments, the β chain constant region comprises amino acid substitutions at S92 and T94. In some embodiments, the β chain constant region comprises amino acid substitutions at T94 and Q107. In some embodiments, the β chain constant region comprises amino acid substitutions at S92 and Q107.

In some embodiments, the β chain constant region comprises amino acid substitutions at S92, T94 and Q107.

In some embodiments, the α chain constant region comprises an amino acid substitution at any one of positions Q6, S21, K83, S84.6 and I114; and the β chain constant region comprises an amino acid substitution at S92.

In some embodiments, the α chain constant region comprises an amino acid substitution at any one of positions Q6, S21, K83, S84.6 and I114; and the β chain constant region comprises an amino acid substitution at T94.

In some embodiments, the α chain constant region comprises an amino acid substitution at any one of positions Q6, S21, K83, S84.6 and I114; and the β chain constant region comprises an amino acid substitution at Q107.

In some embodiments, the α chain constant region comprises an amino acid substitution at any one of positions Q6, S21, K83, S84.6 and I114; and the β chain constant region comprises amino acid substitutions at S92 and T94.

In some embodiments, the α chain constant region comprises an amino acid substitution at any one of positions Q6, S21, K83, S84.6 and I114; and the β chain constant region comprises amino acid substitutions at S92 and Q107.

In some embodiments, the α chain constant region comprises an amino acid substitution at any one of positions Q6, S21, K83, S84.6 and I114; and the β chain constant region comprises amino acid substitutions at T94 and Q107.

In some embodiments, the α chain constant region comprises an amino acid substitution at any one of positions Q6, S21, K83, S84.6 and I114; and the β chain constant region comprises amino acid substitutions at S92, T94 and Q107.

In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114, or S84.6 and I114; and the β chain constant region comprises an amino acid substitution at S92.

In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114, or S84.6 and I114; and the β chain constant region comprises an amino acid substitution at T94.

In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114, or S84.6 and I114; and the β chain constant region comprises an amino acid substitution at Q107.

In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114, or S84.6 and I114; and the β chain constant region comprises amino acid substitutions at S92 and T94.

In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114, or S84.6 and I114; and the β chain constant region comprises amino acid substitutions at S92 and Q107.

In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114, or S84.6 and I114; and the β chain constant region comprises amino acid substitutions at T94 and Q107.

In some embodiments, the α chain constant region comprises amino acid substitutions at Q6 and S21, Q6 and K83, Q6 and S84.6, Q6 and I114, S21 and K83, S21 and S84.6, S21 and I114, K83 and S84.6, K83 and I114, or S84.6 and I114; and the β chain constant region comprises amino acid substitutions at S92, T94 and Q107.

In some embodiments, the α chain constant region comprises amino acid substitutions at Q6, S21 and K83; Q6, S21 and S84.6; Q6, S21 and I114; Q6, K83 and S84.6; Q6, K83 and I114; Q6, S84.6 and I114; S21, K83 and S84.6; S21, K83 and I114; S21, S84.6 and I114; or K83, S84.6 and I114; and the β chain constant region comprises an amino acid substitution at S92.

In some embodiments, the α chain constant region comprises amino acid substitutions at Q6, S21 and K83; Q6, S21 and S84.6; Q6, S21 and I114; Q6, K83 and S84.6; Q6, K83 and I114; Q6, S84.6 and I114; S21, K83 and S84.6; S21, K83 and I114; S21, S84.6 and I114; or K83, S84.6 and I114; and the β chain constant region comprises an amino acid substitution at T94.

In some embodiments, the α chain constant region comprises amino acid substitutions at Q6, S21 and K83; Q6, S21 and S84.6; Q6, S21 and I114; Q6, K83 and S84.6; Q6, K83 and I114; Q6, S84.6 and I114; S21, K83 and S84.6; S21, K83 and I114; S21, S84.6 and I114; or K83, S84.6 and I114; and the β chain constant region comprises an amino acid substitution at Q107.

In some embodiments, the α chain constant region comprises amino acid substitutions at Q6, S21 and K83; Q6, S21 and S84.6; Q6, S21 and I114; Q6, K83 and S84.6; Q6, K83 and I114; Q6, S84.6 and I114; S21, K83 and S84.6; S21, K83 and I114; S21, S84.6 and I114; or K83, S84.6 and I114; and the β chain constant region comprises amino acid substitutions at S92 and T94.

In some embodiments, the α chain constant region comprises amino acid substitutions at Q6, S21 and K83; Q6, S21 and S84.6; Q6, S21 and I114; Q6, K83 and S84.6; Q6, K83 and I114; Q6, S84.6 and I114; S21, K83 and S84.6; S21, K83 and I114; S21, S84.6 and I114; or K83, S84.6 and I114; and the β chain constant region comprises amino acid substitutions at S92 and Q107.

In some embodiments, the α chain constant region comprises amino acid substitutions at Q6, S21 and K83; Q6, S21 and S84.6; Q6, S21 and I114; Q6, K83 and S84.6; Q6, K83 and I114; Q6, S84.6 and I114; S21, K83 and S84.6; S21, K83 and I114; S21, S84.6 and I114; or K83, S84.6 and I114; and the β chain constant region comprises amino acid substitutions at T94 and Q107.

In some embodiments, the α chain constant region comprises amino acid substitutions at Q6, S21 and K83; Q6, S21 and S84.6; Q6, S21 and I114; Q6, K83 and S84.6; Q6, K83 and I114; Q6, S84.6 and I114; S21, K83 and S84.6; S21, K83 and I114; S21, S84.6 and I114; or K83, S84.6 and I114; and the β chain constant region comprises amino acid substitutions at S92, T94 and Q107.

In some embodiments, the α chain constant region comprises amino acid substitutions at Q6, S21, K83 and S84.6; Q6, S21, K83 and I114; Q6, S21, S84.6 and I114; Q6, K83, S84.6 and I114; or S21, K83, S84.6 and I114; and the β chain constant region comprises an amino acid substitution at S92.

In some embodiments, the α chain constant region comprises amino acid substitutions at Q6, S21, K83 and S84.6; Q6, S21, K83 and I114; Q6, S21, S84.6 and I114; Q6, K83, S84.6 and I114; or S21, K83, S84.6 and I114; and the β chain constant region comprises an amino acid substitution at T94.

In some embodiments, the α chain constant region comprises amino acid substitutions at Q6, S21, K83 and S84.6; Q6, S21, K83 and I114; Q6, S21, S84.6 and I114; Q6, K83, S84.6 and I114; or S21, K83, S84.6 and I114; and the β chain constant region comprises an amino acid substitution at Q107.

In some embodiments, the α chain constant region comprises amino acid substitutions at Q6, S21, K83 and S84.6; Q6, S21, K83 and I114; Q6, S21, S84.6 and I114; Q6, K83, S84.6 and I114; or S21, K83, S84.6 and I114; and the β chain constant region comprises amino acid substitutions at S92 and T94.

In some embodiments, the α chain constant region comprises amino acid substitutions at Q6, S21, K83 and S84.6; Q6, S21, K83 and I114; Q6, S21, S84.6 and I114; Q6, K83, S84.6 and I114; or S21, K83, S84.6 and I114; and the β chain constant region comprises amino acid substitutions at S92 and Q107.

In some embodiments, the α chain constant region comprises amino acid substitutions at Q6, S21, K83 and S84.6; Q6, S21, K83 and I114; Q6, S21, S84.6 and I114; Q6, K83, S84.6 and I114; or S21, K83, S84.6 and I114; and the β chain constant region comprises amino acid substitutions at T94 and Q107.

In some embodiments, the α chain constant region comprises amino acid substitutions at Q6, S21, K83 and S84.6; Q6, S21, K83 and I114; Q6, S21, S84.6 and I114; Q6, K83, S84.6 and I114; or S21, K83, S84.6 and I114; and the β chain constant region comprises amino acid substitutions at S92, T94 and Q107.

In some embodiments, the α chain constant region comprises amino acid substitutions at Q6, S21, K83, S84.6 and I114; and the β chain constant region comprises an amino acid substitution at S92.

In some embodiments, the α chain constant region comprises amino acid substitutions at Q6, S21, K83, S84.6 and I114; and the β chain constant region comprises an amino acid substitution at T94.

In some embodiments, the α chain constant region comprises amino acid substitutions at Q6, S21, K83, S84.6 and I114; and the β chain constant region comprises an amino acid substitution at Q107.

In some embodiments, the α chain constant region comprises amino acid substitutions at Q6, S21, K83, S84.6 and I114; and the β chain constant region comprises amino acid substitutions at S92 and T94.

In some embodiments, the α chain constant region comprises amino acid substitutions at Q6, S21, K83, S84.6 and I114; and the β chain constant region comprises amino acid substitutions at S92 and Q107.

In some embodiments, the α chain constant region comprises amino acid substitutions at Q6, S21, K83, S84.6 and I114; and the β chain constant region comprises amino acid substitutions at T94 and Q107.

In some embodiments, the α chain constant region comprises amino acid substitutions at Q6, S21, K83, S84.6 and I114; and the β chain constant region comprises amino acid substitutions at S92, T94 and Q107.

In some embodiments, the α chain constant region comprises amino acid substitutions at Q6, S21, K83, S84.6 and I114, and/or the β chain constant region comprises amino acid substitutions at S92, T94 and Q107. In some embodiments, the α chain constant region comprises amino acid substitutions at Q6, S21, K83 and S84.6, and/or the β chain constant region comprises amino acid substitutions at T94 and Q107. In some embodiments, the α chain constant region comprises amino acid substitutions at S21, K83, S84.6 and I114, and/or the β chain constant region comprises amino acid substitutions at T94 and Q107. In some embodiments, the α chain constant region comprises amino acid substitutions at S21, K83 and S84.6, and/or the β chain constant region comprises amino acid substitutions at T94 and Q107. In some embodiments, the α chain constant region comprises amino acid substitutions at S21 and S84.6, and/or the β chain constant region comprises amino acid substitutions at T94 and Q107.

In some embodiments, the α chain constant region comprises one or more of the amino acid substitutions S21I, K83P and S84.6D; preferably comprises at least two of the amino acid substitutions S211, K83P and S84.6D; and more preferably comprises the amino acid substitutions S21I, K83P and S84.6D. In some embodiments, the α chain constant region comprises S21I, K83P and S84.6D. In some embodiments, the α chain constant region comprises S21I and S84.6D.

In some embodiments, the β chain constant region comprises one or more of the amino acid substitutions T94D and Q107L; preferably comprises the amino acid substitutions T94D and Q107L. In some embodiments, the β chain constant region comprises T94D and Q107L. In some embodiments, the β chain constant region comprises T94D. In some embodiments, the β chain constant region comprises Q107L.

In some embodiments, the multi-specific antigen binding molecule comprises amino acid substitutions selected from any one of the following groups (1)-(21):
(1) S211, K83P and S84.6D of the α chain constant region, and T94D and Q107L of the β chain constant region;
(2) K83P and S84.6D of the α chain constant region, and T94D and Q107L of the β chain constant region;
(3) S21I and S84.6D of the α chain constant region, and T94D and Q107L of the β chain constant region;
(4) S21I and K83P of the α chain constant region, and T94D and Q107L of the β chain constant region;
(5) S21I of the α chain constant region, and T94D, and Q107L of the β chain constant region;
(6) K83P of the α chain constant region, and T94D, and Q107L of the β chain constant region;
(7) S84.6D of the α chain constant region, and T94D, and Q107L of the β chain constant region;
(8) S21I, K83P and S84.6D of the α chain constant region, and Q107L of the β chain constant region;
(9) S21I, K83P and S84.6D of the α chain constant region, and T94D of the β chain constant region;
(10) K83P and S84.6D of the α chain constant region, and T94D of the β chain constant region;
(11) K83P and S84.6D of the α chain constant region, and Q107L of the β chain constant region;
(12) S21I and S84.6D of the α chain constant region, and T94D of the β chain constant region;
(13) S21I and S84.6D of the α chain constant region, and Q107L of the β chain constant region;
(14) S21I and K83P of the α chain constant region, and T94D of the β chain constant region;
(15) S21I and K83P of the α chain constant region, and Q107L of the β chain constant region;
(16) S21I of the α chain constant region, and Q107L of the β chain constant region;
(17) S21I of the α chain constant region, and T94D of the β chain constant region;
(18) K83P of the α chain constant region, and T94D of the β chain constant region;
(19) K83P of the α chain constant region, and Q107L of the β chain constant region;
(20) S84.6D of the α chain constant region, and T94D of the β chain constant region;
(21) S84.6D of the α chain constant region, and Q107L of the β chain constant region.

In some embodiments, the TCR comprises amino acid substitutions selected from any one of (1)-(21) above.

In some embodiments, the α chain constant region further comprises one or more of the amino acid mutations N1.2K, T84C and an FFPSP deletion at positions 120-124; and/or the β chain constant region further comprises one or more of the amino acid mutations S79C, C85.1A and N97D. In some preferred embodiments, the α chain constant region further comprises T84C and optionally N1.2K and/or an FFPSP deletion at positions 120-124; and/or the β chain constant region further comprises S79C and optionally C85.1A and/or N97D. In some more preferred embodiments, the α chain constant region further comprises N1.2K, T84C and an FFPSP deletion at positions 120-124, and the β chain constant region further comprises S79C, C85.1A and N97D.

In some embodiments, the α chain constant region has at least 80%, at least 85%, at least 90%, at least 91%, at least 92%, at least 93%, at least 94%, at least 95%, at least 96%, at least 97%, at least 98% or at least 99% sequence identity to SEQ ID NO.1, and/or the β chain constant region has at least 80%, at least 85%, at least 90%, at least 91%, at least 92%, at least 93%, at least 94%, at least 95%, at least 96%, at least 97%, at least 98% or at least 99% sequence identity to SEQ ID NO.14.

In some embodiments, the α chain constant region has at least 80%, at least 85%, at least 90%, at least 91%, at least 92%, at least 93%, at least 94%, at least 95%, at least 96%, at least 97%, at least 98% or at least 99% sequence identity to SEQ ID NO.2, and/or the β chain constant region has at least 80%, at least 85%, at least 90%, at least 91%, at least 92%, at least 93%, at least 94%, at least 95%, at least 96%, at least 97%, at least 98% or at least 99% sequence identity to SEQ ID NO.15.

In some embodiments, the α chain constant region comprises an amino acid sequence as shown in any one of SEQ ID NOs.3-13, or comprises an amino acid sequence having at least 80%, at least 85%, at least 90%, at least 95% or at least 99% sequence identity to any one of SEQ ID NOs.3-13; and/or the β chain constant region comprises an amino acid sequence as shown in any one of SEQ ID NOs.16-23, or comprises an amino acid sequence having at least 80%, at least 85%, at least 90%, at least 95% or at least 99% sequence identity to any one of SEQ ID NOs.16-23.

In some embodiments, the α chain constant region comprises the amino acid substitutions S21I, K83P and S84.6D, and the β chain constant region comprises the amino acid substitutions T94D and Q107L. In preferred embodiments, the α chain constant region comprises an amino acid sequence as shown in SEQ ID NO.13, and the β chain constant region comprises an amino acid sequence as shown in SEQ ID NO.23.

In some embodiments, the α chain constant region comprises K83P and S84.6D, and the β chain constant region comprises T94D and Q107L. In preferred embodiments, the α chain constant region comprises an amino acid sequence as shown in SEQ ID NO.10, and the β chain constant region comprises an amino acid sequence as shown in SEQ ID NO.23.

In some embodiments, the α chain constant region comprises S21I and S84.6D, and the β chain constant region comprises T94D and Q107L. In preferred embodiments, the α chain constant region comprises an amino acid sequence as shown in SEQ ID NO.11, and the β chain constant region comprises an amino acid sequence as shown in SEQ ID NO.23.

In some embodiments, the α chain constant region comprises S21I and K83P, and the β chain constant region comprises T94D and Q107L. In preferred embodiments, the α chain constant region comprises an amino acid sequence as shown in SEQ ID NO.12, and the β chain constant region comprises an amino acid sequence as shown in SEQ ID NO.23.

In some embodiments, the α chain constant region comprises S21I, K83P and S84.6D, and the β chain constant region comprises Q107L. In preferred embodiments, the α chain constant region comprises an amino acid sequence as shown in SEQ ID NO.13, and the β chain constant region comprises an amino acid sequence as shown in SEQ ID NO.22.

In some embodiments, the α chain constant region comprises S21I, K83P and S84.6D, and the β chain constant region comprises T94D. In preferred embodiments, the α chain constant region comprises an amino acid sequence as shown in SEQ ID NO.13, and the β chain constant region comprises an amino acid sequence as shown in SEQ ID NO.21.

In some embodiments, the multi-specific antigen binding molecule comprises two or more antigen binding regions that bind two or more antigens, and the two or more antigens are each independently selected from tumor-associated antigens (TAAs), tumor-specific antigens (TSAs), viral antigens, self-antigens, and immune cell surface molecules.

In some embodiments, the TAA is selected from melanoma-associated antigens (e.g., gp100, MAGEA1, MAGEA3, MAGEA6, MAGEA4, MAGEA2, MAGEA12, MAGEA2B, MAGEA9B, MAGEA10, MAGEA11, MAGEB2, MAGEC1, MAGEC2), IGF2BP1, GNGT1, PI4K2B, CCR8, NPSR1, COX7B2, ONECUT3, SMC1B, FOXI3, GAGE2A, FBXO43, BRDT, PAGE2, GAGE13, POU5F1B, CTAG1A and endogenous retroviral antigens.

In some embodiments, the TSA is selected from KRAS (e.g., KRAS G12 mutant antigens such as G12D, G12V, G12C, G12R, G12A; G13D; Q61H; G125), TP53 (e.g., R175H, R173H, R273C, R248W, R248Q, R282W, Y220C, V157F, G245S, Y163C, R249S), PIK3CA (e.g., E542K, E545K, H1047R), CTNNB1 (e.g., S45P, T41A), EGFR (e.g., L858R, T790M), BRAF (e.g., V600E) and GNAS (e.g., R201C, R201H).

In some embodiments, the viral antigen is selected from HPV antigens (e.g., HPV E6 or E7 antigens), CMV antigens, HBV antigens, EBV antigens, herpesvirus antigens, human immunodeficiency virus (HIV) antigens, influenza virus antigens and coronavirus antigens.

In some embodiments, the self-antigen is selected from AFP, CEA, CD19, CD20, BCMA, CD22, CD30, SLAM, CLDN18.2, GD2, mesothelin, CD38, Her2, GPC3, MUC1, Ro52, Ro60, La, Jo-1, SRP, IFIH1, CENPA, CENPB, SNRPA1, SNRNP70, SNR-PD3, RNAP3, TOPO1, Insulin, GAD65, IA2, Znt8, PL7, TARS, ARS, MI2, topoisomerase 1, EXOSC9, EXOSC107, POLR3A, POLR3K, PTRN, GAD2, SLC30A8, AchR, MUSK, LRP4, PLA2R, THSD7A, TSHR, IFN-γ, CHRNA1, MUSK, LRP4, AQP4, MOG, GRIN1, COL4A3, PLA2R, GM-SCF, PR3 and MPO.

In some embodiments, the immune cell surface molecule is selected from CD3 (e.g., CD3γ, CD3δ and CD3ε chains), CD4, CD8, CD10, CD11b, CD11c, CD14, CD16, CD18, CD25, CD32a, CD32b, CD41, CD41b, CD42a, CD42b, CD44, CD45RA, CD49, CD61, CD64, CD68, CD94, CD90, CD117, Nkp46, NKG2D, FcεRI, TCRα/β, TCR γ/δ, HLA-DR, CD28, 4-1BB(CD137), OX40(CD134), ICOS(CD278), 2B4 (CD244), HVEM, LAG3, DAP10, DAP12, CD27, CD40, GITR, LFA-1, MyD88, CD2, CD7, LIGHT, B7-H3, CTLA-4, PD-1, PD-L1, CD80, BTLA, TIM3, TIGIT and LAG-3.

In some embodiments, one of the two or more antigens is selected from tumor-associated antigens (TAAs), tumor-specific antigens (TSAs), viral antigens and self-antigens; and another is immune cell surface molecules. In some embodiments, the antigen bound by the TCR-derived antigen binding region is selected from tumor-associated antigens (TAAs), tumor-specific antigens (TSAs), viral antigens and self-antigens.

In some embodiments, the TAA is a melanoma-associated antigen (e.g., gp100, MAGEA1). In some embodiments, the TSA is a KRAS antigen (e.g., KRAS G12V, KRAS G12D). In some embodiments, the viral antigen is an HPV antigen (e.g., HPV E6 or E7). In some embodiments, the immune cell surface molecule is selected from CD3, CD28, 4-1BB (CD137), PD-1 and PD-L1. In some embodiments, the immune cell surface molecule is CD3. In some embodiments, the immune cell surface molecule is CD28.

In some embodiments, the multi-specific antigen binding molecule binds KRAS G12V, and comprises a TCR α chain variable region (TRAV) and a TCR β chain variable region (TRBV) binding KRAS G12V. In some embodiments, the TRAV comprises α chain CDR1, CDR2 and CDR3 having the amino acid sequences as shown in SEQ ID NOs.38, 39 and 40, respectively, and the TRBV comprises β chain CDR1, CDR2 and CDR3 having the amino acid sequences as shown in SEQ ID NOs.41, 42 and 43, respectively. In some embodiments, the TRAV comprises an amino acid sequence having at least 80%, at least 85%, at least 90%, at least 95% or 100% sequence identity to SEQ ID NO.24, and the TRBV comprises an amino acid sequence having at least 80%, at least 85%, at least 90%, at least 95% or 100% sequence identity to SEQ ID NO.25. In some embodiments, the TRAV comprises α chain CDR1, CDR2 and CDR3 having the amino acid sequences as shown in SEQ ID NOs.44, 45 and 46, respectively, and the TRBV comprises β chain CDR1, CDR2 and CDR3 having the amino acid sequences as shown in SEQ ID NOs.47, 48 and 49, respectively. In some embodiments, the TRAV comprises an amino acid sequence having at least 80%, at least 85%, at least 90%, at least 95% or 100% sequence identity to SEQ ID NO.26, and the TRBV comprises an amino acid sequence having at least 80%, at least 85%, at least 90%, at least 95% or 100% sequence identity to SEQ ID NO.27. In some embodiments, the TRAV comprises α chain CDR1, CDR2 and CDR3 having the amino acid sequences as shown in SEQ ID NOs.50, 51 and 52, respectively, and the TRBV comprises β chain CDR1, CDR2 and CDR3 having the amino acid sequences as shown in SEQ ID NOs.53, 54 and 55, respectively. In some embodiments, the TRAV comprises an amino acid sequence having at least 80%, at least 85%, at least 90%, at least 95% or 100% sequence identity to SEQ ID NO.28, and the TRBV comprises an amino acid sequence having at least 80%, at least 85%, at least 90%, at least 95% or 100% sequence identity to SEQ ID NO.29.

In some embodiments, the multi-specific antigen binding molecule binds KRAS G12D, and comprises a TCR α chain variable region (TRAV) and a TCR β chain variable region (TRBV) binding KRAS G12D. In some embodiments, the TRAV comprises α chain CDR1, CDR2, and CDR3 having the amino acid sequences as shown in SEQ ID NOs.78, 79, and 80, respectively, and the TRBV comprises β chain CDR1, CDR2, and CDR3 having the amino acid sequences as shown in SEQ ID NOs.81, 82, and 83, respectively. In some embodiments, the TRAV comprises an amino acid sequence having at least 80%, at least 85%, at least 90%, at least 95%, or 100% sequence identity to SEQ ID NO.34, and the TRBV comprises an amino acid sequence having at least 80%, at least 85%, at least 90%, at least 95%, or 100% sequence identity to SEQ ID NO.35. In some embodiments, the TRAV comprises α chain CDR1, CDR2, and CDR3 having the amino acid sequences as shown in SEQ ID NOs.84, 85, and 86, respectively, and the TRBV comprises β chain CDR1, CDR2, and CDR3 having the amino acid sequences as shown in SEQ ID NOs.87, 88, and 89, respectively. In some embodiments, the TRAV comprises an amino acid sequence having at least 80%, at least 85%, at least 90%, at least 95%, or 100% sequence identity to SEQ ID NO.90, and the TRBV comprises an amino acid sequence having at least 80%, at least 85%, at least 90%, at least 95%, or 100% sequence identity to SEQ ID NO.91.

In some embodiments, the multi-specific antigen binding molecule binds MAGEA1, and comprises a TCR α chain variable region (TRAV) and a TCR β chain variable region (TRBV) binding MAGEA1. In some embodiments, the TRAV comprises α chain CDR1, CDR2, and CDR3 having the amino acid sequences as shown in SEQ ID NOs.56, 57, and 58, respectively, and the TRBV comprises β chain CDR1, CDR2, and CDR3 having the amino acid sequences as shown in SEQ ID NOs.59, 60, and 61, respectively. In some embodiments, the TRAV comprises an amino acid sequence having at least 80%, at least 85%, at least 90%, at least 95%, or 100% sequence identity to SEQ ID NO.30, and the TRBV comprises an amino acid sequence having at least 80%, at least 85%, at least 90%, at least 95%, or 100% sequence identity to SEQ ID NO.31. In some embodiments, the TRAV comprises α chain CDR1, CDR2, and CDR3 having the amino acid sequences as shown in SEQ ID NOs.62, 63, and 64, respectively, and the TRBV comprises β chain CDR1, CDR2, and CDR3 having the amino acid sequences as shown in SEQ ID NOs.65, 66, and 67, respectively. In some embodiments, the TRAV comprises an amino acid sequence having at least 80%, at least 85%, at least 90%, at least 95%, or 100% sequence identity to SEQ ID NO.32, and the TRBV comprises an amino acid sequence having at least 80%, at least 85%, at least 90%, at least 95%, or 100% sequence identity to SEQ ID NO.33. In some embodiments, the TRAV comprises an amino acid sequence having at least 80%, at least 85%, at least 90%, at least 95%, or 100% sequence identity to SEQ ID NO.68, and the TRBV comprises an amino acid sequence having at least 80%, at least 85%, at least 90%, at least 95%, or 100% sequence identity to SEQ ID NO.69.

In some embodiments, the multi-specific antigen binding molecule binds HPV E7 and comprises a TCR α chain variable region (TRAV) and a TCR β chain variable region (TRBV) binding HPV E7. In some embodiments, the TRAV comprises α chain CDR1, CDR2, and CDR3 having the amino acid sequences as shown in SEQ ID NOs.70, 71, and 72, respectively, and the TRBV comprises β chain CDR1, CDR2, and CDR3 having the amino acid sequences as shown in SEQ ID NOs.73, 74, and 75, respectively. In some embodiments, the TRAV comprises an amino acid sequence having at least 80%, at least 85%, at least 90%, at least 95%, or 100% sequence identity to SEQ ID NO.76, and the TRBV comprises an amino acid sequence having at least 80%, at least 85%, at least 90%, at least 95%, or 100% sequence identity to SEQ ID NO.77.

In some embodiments, the multi-specific antigen binding molecule binds gp100, and comprises a TCR α chain variable region (TRAV) and a TCR β chain variable region (TRBV) binding gp100. In some embodiments, the TRAV comprises an amino acid sequence having at least 80%, at least 85%, at least 90%, at least 95%, or 100% sequence identity to SEQ ID NO.94, and the TRBV comprises an amino acid sequence having at least 80%, at least 85%, at least 90%, at least 95%, or 100% sequence identity to SEQ ID NO.95.

In some embodiments, the multi-specific antigen binding molecule does not comprise a TRAV having an amino acid sequence as shown in SEQ ID NO.24, and does not comprise a TRBV having an amino acid sequence as shown in SEQ ID NO.25. In some embodiments, the multi-specific antigen binding molecule does not comprise a TRAV having CDR1, CDR2, and CDR3 with the amino acid sequences as shown in SEQ ID NOs.38-40, and does not comprise a TRBV having CDR1, CDR2, and CDR3 with the amino acid sequences as shown in SEQ ID NOs.41-43. In some embodiments, the multi-specific antigen binding molecule does not bind the KRAS epitope VVGAVGVGK or a complex thereof with HLA-A*11. In some embodiments, the multi-specific antigen binding molecule does not bind KRAS antigens.

In some embodiments, the TCR does not comprise a TRAV having an amino acid sequence as shown in SEQ ID NO.94, and does not comprise a TRBV having an amino acid sequence as shown in SEQ ID NO.95.

In some embodiments, the TCR does not bind gp100.

In some embodiments, the multi-specific antigen binding molecule comprises, on at least two polypeptide chains, a first binding region that binds a first antigen and a second binding region that binds a second antigen,
wherein the first binding region comprises α chain variable region (TRAV) and β chain variable region (TRBV) derived from a TCR that binds the first antigen-MHC complex;
wherein the second binding region comprises a heavy-chain variable region (VH) and a light-chain variable region (VL) derived from an antibody that binds the second antigen;
wherein the first antigen is selected from tumor-associated antigens (TAAs), tumor-specific antigens (TSAs), viral antigens, and self-antigens, and the second antigen is an immune cell surface molecule;
and wherein the multi-specific antigen binding molecule further comprises at least one of the following functional domains:
   1) a hinge region derived from an antibody;
   2) an Fc domain derived from an antibody or a dimerization portion thereof;
   3) a CH3 domain derived from an antibody;
   4) an albumin (Alb) or a binding portion thereof;
   5) a TCR constant region or a fragment thereof.

In some embodiments, the functional domain comprises a TCR constant region or a fragment thereof.

In some embodiments, the bispecific polypeptide molecule comprises two polypeptide chains, and the two polypeptide chains respectively comprise:
TRAV-VH and VL-TRBV;
TRAV-VL and VH-TRBV;
TRBV-VH and VL-TRAV; or
TRBV-VL and VH-TRAV;
and, adjacent variable regions of the two polypeptide chains are connected via linkers; and the TCR constant region or a fragment thereof is connected via an optional linker to the C-terminus of each polypeptide chain.

In some embodiments, the TCR constant region or a fragment thereof is the TCR constant region or a fragment thereof as described herein. In some embodiments, the TCR constant region comprises the TRAC as described herein and/or the TRBC as described herein. In some embodiments, one polypeptide chain of the bispecific polypeptide molecule comprises TRAC and the other polypeptide chain comprises TRBC.

In some embodiments, the bispecific polypeptide molecule comprises a first polypeptide chain and a second polypeptide chain, wherein the first polypeptide chain comprises, from the N-terminus to the C-terminus: TRAV-linker-VH-optional linker-TRAC, and the second polypeptide chain comprises, from the N-terminus to the C-terminus: VL-linker-TRBV-optional linker-TRBC.

In some embodiments, the bispecific polypeptide molecule comprises a first polypeptide chain and a second polypeptide chain, wherein the first polypeptide chain comprises, from the N-terminus to the C-terminus: TRAV-linker-VH-optional linker-TRBC, and the second polypeptide chain comprises, from the N-terminus to the C-terminus: VL-linker-TRBV-optional linker-TRAC.

In some embodiments, the bispecific polypeptide molecule comprises a first polypeptide chain and a second polypeptide chain, wherein the first polypeptide chain comprises, from the N-terminus to the C-terminus: TRAV-linker-VL-optional linker-TRAC, and the second polypeptide chain comprises, from the N-terminus to the C-terminus: VH-linker-TRBV-optional linker-TRBC.

In some embodiments, the bispecific polypeptide molecule comprises a first polypeptide chain and a second polypeptide chain, wherein the first polypeptide chain comprises, from the N-terminus to the C-terminus: TRAV-linker-VL-optional linker-TRBC, and the second polypeptide chain comprises, from the N-terminus to the C-terminus: VH-linker-TRBV-optional linker-TRAC.

In some embodiments, the bispecific polypeptide molecule comprises a first polypeptide chain and a second polypeptide chain, wherein the first polypeptide chain comprises, from the N-terminus to the C-terminus: TRBV-linker-VH-optional linker-TRAC, and the second polypeptide chain comprises, from the N-terminus to the C-terminus: VL-linker-TRAV-optional linker-TRBC.

In some embodiments, the bispecific polypeptide molecule comprises a first polypeptide chain and a second polypeptide chain, wherein the first polypeptide chain comprises, from the N-terminus to the C-terminus: TRBV-linker-VH-optional linker-TRBC, and the second polypeptide chain comprises, from the N-terminus to the C-terminus: VL-linker-TRAV-optional linker-TRAC.

In some embodiments, the bispecific polypeptide molecule comprises a first polypeptide chain and a second polypeptide chain, wherein the first polypeptide chain comprises, from the N-terminus to the C-terminus: TRBV-linker-VL-optional linker-TRAC, and the second polypeptide chain comprises, from the N-terminus to the C-terminus: VH-linker-TRAV-optional linker-TRBC.

In some embodiments, the bispecific polypeptide molecule comprises a first polypeptide chain and a second polypeptide chain, wherein the first polypeptide chain comprises, from the N-terminus to the C-terminus: TRBV-linker-VL-optional linker-TRBC, and the second polypeptide chain comprises, from the N-terminus to the C-terminus: VH-linker-TRAV-optional linker-TRAC.

In some embodiments, the multi-specific antigen binding molecule does not comprise the following first polypeptide chain and second polypeptide chain: the first polypeptide chain comprises, from the N-terminus to the C-terminus: TRAV-ANIGGGSGGGG (SEQ ID NO.36)-VH-S-TRAC, and the second polypeptide chain comprises, from the N-terminus to the C-terminus: VL-GGGSGGGGD (SEQ ID NO.37)-TRBV-TRBC.

In some embodiments, the multi-specific antigen binding molecule does not comprise the following first polypeptide chain and second polypeptide chain: the first polypeptide chain comprises, from the N-terminus to the C-terminus: TRAV-linker-VH-linker-TRAC, and the second polypeptide chain comprises, from the N-terminus to the C-terminus: VL-linker-TRBV-optional linker-TRBC.

In some embodiments, the multi-specific antigen binding molecule does not comprise the following first polypeptide chain and second polypeptide chain: the first polypeptide chain comprises, from the N-terminus to the C-terminus: TRAV-linker-VH-optional linker-TRAC, and the second polypeptide chain comprises, from the N-terminus to the C-terminus: VL-linker-TRBV-optional linker-TRBC.

In some embodiments, the two polypeptide chains of the bispecific polypeptide molecule do not comprise TRAV-VH and VL-TRBV.

In some embodiments, the multi-specific antigen binding molecule does not comprise the amino acid sequences as shown in SEQ ID NOs.92 and 93.
SEQ ID NO. 92 
RKEVEQDPGPFNVPEGATVAFNCTYSNSASQSFFWYRQDCRKEPKLLMSVYSSGNEDGRFTAQLNRA
SQYISLLIRDSKLSDSATYLCVVPGGTGGGNKLTFGTGTQLKVELANIGGGSGGGGEVQLVESGGGLV
QPGGSLRLSCAASGYSFTGYTMNWVRQAPGKCLEWVALINPYKGVSTYNQKFKDRFTISVDKSKNTA
YLQMNSLRAEDTAVYYCARSGYYGDSDWYFDVWGQGTLVTVSSSQKPDPAVYQLRDSKSSDKIVCL
FTDFDSQTNVSQSKDSDVYITDPCVLDMRDMDFKSNSAVAWSNKSDFACANAFNNSIIPEDT
SEQ ID NO. 93

DIQMTQSPSSLSASVGDRVTITCRASQDIRNYLNWYQQKPGKAPKLLIYYTSRLESGVPSRFSGSGSGT
DYTLTISSLQPEDFATYYCQQGNTLPWTFGCGTKVEIKGGGSGGGGDTAVSQTPKYLVTQMGNDKSI
KCEQNLGHDTMDWYKQDSKKFLKIMFSYNNKELIINETVPNRFSPKSPDKAHLNLHINSLELGDSAVY

FCASSHWGAQETQYFGPGTRLLVLEDLKNVFPPEVAVFEPSEAEISHTQKATLVCLATGFYPDHVELS
WWVNGKEVHSGVCTDPQPLKEQPALNDSRYALSSRLRVSADFWQDPRNHFRCQVLFYGLSENDEWT
QDRAKPVTQIVSAEAWGRAD

In some embodiments, the antigen binding region derived from a TCR comprises TRAV and TRBV, and further comprises a TRAC connected to the C-terminus of the TRAV via an optional linker, and a TRBC connected to the C-terminus of the TRBV via an optional linker; and wherein the TRAC is the TRAC as described herein; and/or the TRBC is the TRBC as described herein.

In some embodiments, the TRAC as described herein and the TRBC as described herein are respectively connected to the C-terminus of the TRAV and the C-terminus of the TRBV via optional linkers; or the TRAC and the TRBC are respectively connected to the C-terminus of the TRBV and the C-terminus of the TRAV via optional linkers.

In some embodiments, the bispecific polypeptide molecule comprises two polypeptide chains.

In some embodiments, the multi-specific antigen binding molecule comprises a first binding region that binds a first antigen and a second binding region that binds a second antigen,
wherein the first binding region comprises α chain variable region (TRAV1) and β chain variable region (TRBV1) derived from a TCR that binds the first antigen-MHC complex;
wherein the second binding region comprises a heavy-chain variable region VH1 and a light-chain variable region VL1, derived from an antibody that binds the second antigen, or comprises a single heavy-chain variable domain (VHH1) of a single-domain antibody or nanobody that binds the second antigen;
wherein the first antigen is selected from tumor-associated antigens (TAAs), tumor-specific antigens (TSAs), viral antigens, and self-antigens, and the second antigen is an immune cell surface molecule;
wherein the N-terminus of the antibody is connected to the C-terminus of the TCR;
and wherein the multi-specific antigen binding molecule further comprises at least one of the following functional domains:
   1) a hinge region derived from an antibody;
   2) an Fc domain derived from an antibody or a dimerization portion thereof;
   3) a CH3 domain derived from an antibody;
   4) an albumin (Alb) or a binding portion thereof;
   5) a TCR constant region or a fragment thereof.

In some embodiments, the N-terminus of the antibody is connected to the C-terminus of the TCR.

In some embodiments, TRAV1 and TRBV1 are distributed on different polypeptide chains. In some embodiments, VH1 and VL1 are distributed on one polypeptide chain (e.g., in a form of scFv1 or Fab1); and TRAV1 and TRBV1 are distributed on different polypeptide chains. In some embodiments, in some embodiments, VH1 and VL1 are connected by a linker.

In some embodiments, the multi-specific antigen binding molecule comprises, on two polypeptide chains, a first binding region that binds a first antigen and a second binding region that binds a second antigen;
wherein TRAV1 and TRBV1 are distributed on different polypeptide chains, VHH1, or VH1 and VL1, are distributed on one polypeptide chain (e.g., in a form of scFv1 or Fab1) and are connected to the C-terminus of the TCR; or TRAV1 and TRBV1 are distributed on different polypeptide chains, VH1 and VL1 are distributed on different polypeptide chains, and one of polypeptide chains where VH1 or VL1 is located is connected to C-terminus of the polypeptide chain where TRAV1 is located, and the other polypeptide chain is connected to C-terminus of the polypeptide chain where TRBV1 is located.

In some embodiments, the multi-specific antigen binding molecule comprises, on three polypeptide chains, a first binding region that binds a first antigen and a second binding region that binds a second antigen.

In some embodiments, TRAV1 and TRBV1 are distributed on different polypeptide chains, VH1 and VL1 are distributed on different polypeptide chains, and the polypeptide chain where VH1 or VL1 is located is respectively connected to the C-terminus of the polypeptide chain where TRBV1 or TRAV1 is located. In some embodiments, the three polypeptide chains of the multi-specific polypeptide molecule respectively comprise: TRBV1-VL1, VH1, and TRAV1; or TRBV1-VH1, VL1, and TRAV1; or TRAV1-VL1, VH1, and TRBV1; or TRAV1-VH1, VL1, and TRBV1.

In some embodiments, the polypeptide chains of the multi-specific antigen binding molecule respectively comprise:
TRBV1-scFv1 and TRAV1; or
TRAV1-scFv1 and TRBV1; or
TRBV1-VHH1 and TRAV1; or
TRAV1-VHH1 and TRBV1; or
TRBV1-VL1 and TRAV1-VH1; or
TRAV1-VL1 and TRBV1-VH1; or
TRBV1-VL1-VH1 and TRAV1; or
TREV1-VH1-VL1 and TRAV1; or
TRAV1-VL1-VH1 and TRBV1; or
TRAV1-VH1-VL1 and TRBV1; or
TRBV1-VL1, VH1, and TRAV1; or
TRBV1-VH1, VL1, and TRAV1; or
TRAV1-VL1, VH1, and TRBV1; or
TRAV1-VH1, VL1, and TRBV1.

In some embodiments, the multi-specific antigen binding molecule further comprises a third binding region that binds a third antigen, the third binding region comprising a α chain variable region (TRAV2) and a β chain variable region (TRBV2) derived from a TCR that binds the third antigen-MHC complex; wherein the third antigen is selected from tumor-associated antigens (TAAs), tumor-specific antigens (TSAs), viral antigens, and self-antigens, and the third antigen is the same as or different from the first antigen.

In some embodiments, TRAV2 is the same as TRAV1, and/or TRBV2 is the same as TRBV1.

In some embodiments, TRAV2 and TRBV2 are connected on the same polypeptide chain via a linker and are preferably connected between VH1 and VL1.

In some embodiments, the multi-specific antigen binding molecule comprises two polypeptide chains, the two polypeptide chains respectively comprise:
TRBV1-VL1-TRAV2-TRBV2-VH1 and TRAV1; or
TRBV1-VL1-TRBV2-TRAV2-VH1 and TRAV1; or
TRBV1-VH1-TRBV2-TRAV2-VL1 and TRAV1; or
TRBV1-VH1-TRAV2-TRBV2-VL1 and TRAV1; or
TRAV1-VL1-TRBV2-TRAV2-VH1 and TRBV1; or
TRAV1-VL1-TRAV2-TRBV2-VH1 and TRBV1; or
TRAV1-VH1-TRAV2-TRBV2-VL1 and TRBV1; or
TRAV1-VH1-TRBV2-TRAV2-VL1 and TRBV1.

In some embodiments, the multi-specific antigen binding molecule further comprises a fourth binding region that binds a fourth antigen, the fourth binding region comprising a heavy-chain variable region VH2 and a light-chain variable region VL2 derived from an antibody that binds the fourth antigen, or comprising a single heavy-chain variable domain (VHH2) of a single-domain antibody or nanobody that binds the fourth antigen; wherein the fourth antigen is an immune cell surface molecule, and the fourth antigen is the same as or different from the second antigen.

In some embodiments, VH2 is the same as VH1, and/or VL2 is the same as VL1, and/or VHH2 is the same as VHH1.

In some embodiments, VHH2, or VH2 and VL2 in tandem on one polypeptide chain (e.g., in a form of scFv2 or Fab2), is connected to the N-terminus of TRAV1 or TRBV1, or one of VH2 and VL2 is connected to the N-terminus of TRAV1 and the other is connected to the N-terminus of TRBV1.

In some embodiments, the multi-specific antigen binding molecule comprises two polypeptide chains, the two polypeptide chains respectively comprise:
scFv2-TRBV1-scFv1 and TRAV1; or
scFv2-TRAV1-scFv1 and TRBV1; or
scFv2-TRBV1-VHH1 and TRAV1; or
scFv2-TRAV1-VHH1 and TRBV1; or
scFv2-TRBV1-VL1 and TRAV1-VH1; or
scFv2-TRAV1-VL1 and TRBV1-VH1; or
TRBV1-scFv1 and scFv2-TRAV1; or
TRAV1-scFv1 and scFv2-TRBV1; or
TRBV1-VHH1 and scFv2-TRAV1; or
TRAV1-VHH1 and scFv2-TRBV1; or
TRBV1-VL1 and scFv2-TRAV1-VH1; or
TRAV1-VL1 and scFv2-TRBV1-VH1; or
VHH2-TRBV1-scFvl and TRAV1; or
VHH2-TRAV1-scFv1 and TRBV1; or
VHH2-TRBV1-VHH1 and TRAV1; or
VHH2-TRAV1-VHH1 and TRBV1; or
VHH2-TRBV1-VL1 and TRAV1-VH1; or
VHH2-TRAV1-VL1 and TRBV1-VH1; or
TRBV1-scFv1 and VHH2-TRAV1; or
TRAV1-scFv1 and VHH2-TRBV1; or
TRBV1-VHH1 and VHH2-TRAV1; or
TRAV1-VHH1 and VHH2-TRBV1; or
TRBV1-VL1 and VHH2-TRAV1-VH1; or
TRAV1-VL1 and VHH2-TRBV1-VH1; or
VL2-TRBV1-scFv1 and VH2-TRAV1; or
VH2-TRBV1-scFv1 and VL2-TRAV1; or
VL2-TRAV1-scFv1 and VH2-TRBV1; or
VH2-TRAV1-scFv1 and VL2-TRBV1; or
VL2-TRBV1-VHH1 and VH2-TRAV1; or
VH2-TRBV1-VHH1 and VL2-TRAV1; or
VL2-TRAV1-VHH1 and VH2-TRBV1; or
VH2-TRAV1-VHH1 and VL2-TRBV1; or
VL2-TRBV1-VL1 and VH2-TRAV1-VH1; or
VH2-TRBV1-VL1 and VL2-TRAV1-VH1; or
VL2-TRAV1-VL1 and VH2-TRBV1-VH1; or
VH2-TRAV1-VL1 and VL2-TRBV1-VH1.

In some embodiments, the multi-specific antigen binding molecule comprises a first binding region, a second binding region, a third binding region, and a fourth binding region, and the multi-specific antigen binding molecule comprises two polypeptide chains, the two polypeptide chains respectively comprise:
scFv2-TRBV1-VL1-TRAV2-TRBV2-VH1 and TRAV1; or
scFv2-TRBV1-VL1-TRBV2-TRAV2-VH1 and TRAV1; or
scFv2-TRBV1-VH1-TRBV2- TRAV2-VL1 and TRAV1; or
scFv2-TRBV1-VH1-TRAV2- TRBV2-VL1 and TRAV1; or
scFv2-TRAV1-VL1-TRBV2- TRAV2-VH1 and TRBV1; or
scFv2-TRAV1-VL1-TRAV2- TRBV2-VH1 and TRBV1; or
scFv2-TRAV1-VH1-TRAV2-TRBV2-VL1 and TRBV1; or
scFv2-TRAV1-VH1-TRBV2-TRAV2-VL1 and TRBV1; or
TRBV1-VL1-TRAV2-TRBV2-VH1 and scFv2-TRAV1; or
TRBV1-VL1-TRBV2-TRAV2-VH1 and scFv2-TRAV1; or
TRBV1-VH1-TRBV2- TRAV2-VL1 and scFv2-TRAV1; or
TRBV1-VH1-TRA V2- TRBV2-VL1 and scFv2-TRAV1; or
TRAV1-VL1-TRBV2- TRAV2-VH1 and scFv2-TRBV1; or
TRAV1-VL1-TRAV2-TRBV2-VH1 and scFv2-TRBV1; or
TRAV1-VH1-TRAV2-TRBV2-VL1 and scFv2-TRBV1; or
TRAV1-VH1-TRBV2-TRAV2-VL1 and scFv2-TRBV1; or
VHH2-TRBV1-VL1-TRAV2-TRBV2-VH1 and TRAV1; or
VHH2-TRBV1-VL1-TRBV2-TRAV2-VH1 and TRAV1; or
VHH2-TRBV1-VH1-TRBV2- TRAV2-VL1 and TRAV1; or
VHH2-TRBV1-VH1-TRAV2- TRBV2-VL1 and TRAV1; or
VHH2-TRAV1-VL1-TRBV2- TRAV2-VH1 and TRBV1; or
VHH2-TRAV1-VL1-TRAV2- TRBV2-VH1 and TRBV1; or
VHH2-TRAV1-VH1-TRAV2-TRBV2-VL1 and TRBV1; or
VHH2-TRAV1-VH1-TRBV2-TRAV2-VL1 and TRBV1; or
TRBV1-VL1-TRAV2-TRBV2-VH1 and VHH2-TRAV1; or
TRBV1-VL1-TRBV2-TRAV2-VH1 and VHH2-TRAV1; or
TRBV1-VH1-TRBV2-TRAV2-VL1 and VHH2-TRAV1; or
TRBV1-VH1-TRAV2- TRBV2-VL1 and VHH2-TRAV1; or
TRAV1-VL1-TRBV2- TRAV2-VH1 and VHH2-TRBV1; or
TRAV1-VL1-TRAV2- TRBV2-VH1 and VHH2-TRBV1; or
TRAV1-VH1-TRAV2-TRBV2-VL1 and VHH2-TRBV1; or
TRAVI1-VH1-TRBV2-TRAV2-VL1 and VHH2-TRBV1; or
VL2-TRBV1-VL1-TRAV2-TRBV2-VH1 and VH2-TRAV1; or
VL2-TRBV1-VL1-TRBV2-TRAV2-VH1 and VH2-TRAV1; or
VH2-TRBV1-VL1-TRAV2-TRBV2-VH1 and VL2-TRAV1; or
VH2-TRBV1-VL1-TRBV2-TRAV2-VH1 and VL2-TRAV1; or
VL2-TRBV1-VH1-TRBV2- TRAV2-VL1 and VH2-TRAV1; or
VL2-TRBV1-VH1-TRAV2- TRBV2-VL1 and VH2-TRAV1; or
VH2-TRBV1-VH1-TRBV2- TRAV2-VL1 and VL2-TRAV1; or
VH2-TRBV1-VH1-TRAV2- TRBV2-VL1 and VL2-TRAV1; or
VL2-TRAV1-VL1-TRBV2- TRAV2-VH1 and VH2-TRBV1; or
VL2-TRAV1-VL1-TRAV2- TRBV2-VH1 and VH2-TRBV1; or
VH2-TRAV1-VL1-TRBV2-TRAV2-VH1 and VL2-TRBV1; or
VH2-TRAV1-VL1-TRAV2-TRBV2-VH1 and VL2-TRBV1; or
VL2-TRAV1-VH1-TRAV2-TRBV2-VL1 and VH2-TRBV1; or
VL2-TRAV1-VH1-TRBV2-TRAV2-VL1 and VH2-TRBV1; or
VH2-TRAV1-VH1-TRAV2-TRBV2-VL1 and VL2-TRBV1; or
VH2-TRAV1-VH1-TRBV2-TRAV2-VL1 and VL2-TRBV1.

In some embodiments, the functional domain is derived from albumin or a binding portion thereof and optionally from an antibody hinge region; and the functional domain is connected to the C-terminus or N-terminus of any polypeptide chain of the multi-specific antigen binding molecule, preferably the C-terminus, via an optional linker.

In some embodiments, the albumin is serum albumin, preferably human serum albumin; and/or the antibody hinge region is a human-derived hinge domain or a portion thereof, e.g. derived from human IgG1, IgG2, or IgG4.

In some embodiments, the functional domain is derived from an antibody hinge region and/or an antibody Fc domain or a dimerization portion thereof and/or an antibody CH3 domain; and any two polypeptide chains of the multi-specific antigen binding molecule are connected via covalent or noncovalent bonds between the two chains of the functional domain;
preferably, the two chains of the functional domain are connected to the C-terminus or N-terminus, preferably the C-terminus, of any two polypeptide chains of the multi-specific antigen binding molecule via optional linkers; or
the first binding region and the second binding region are connected via covalent or noncovalent bonds between the two chains of the functional domain.

In some embodiments, the antibody hinge region is a human-derived hinge domain or a portion thereof, e.g., derived from human IgG1, IgG2, or IgG4; and/or
the antibody Fc domain or dimerization portion thereof is a human-derived Fc domain or a portion thereof, e.g., derived from human IgG1, IgG2, or IgG4.

In some embodiments, the antibody hinge region comprises at least one amino acid mutation capable of reducing or eliminating binding of the multi-specific molecule to an FcR receptor and/or C1q, e.g., the mutation site is selected from positions 233, 234, 235, and 236; and/or
the antibody hinge region comprises at least one mutation capable of reducing or eliminating formation of disulfide bonds between the hinge region and a non-hinge region, e.g., the mutation site is position 220; and/or
the CH2 domain in the Fc domain comprises at least one amino acid mutation capable of reducing or eliminating binding of the multi-specific molecule to an FcR receptor and/or C1q; e.g., the mutation site is selected from positions 237, 250, 252, 254, 256, 270, 297, 322, 327, 330, and 331; and/or
the CH3 domain in the Fc domain comprises at least one amino acid mutation capable of reducing or eliminating binding of the multi-specific molecule to an FcR receptor and/or C1q; e.g., the mutation site is selected from positions 428 and 434;
the CH3 domain in the Fc domain comprises at least one of the following mutations capable of promoting formation of a heterodimer of the polypeptide molecule:
   a) comprising mutations of at least two cysteine residues to form an interchain disulfide bond, e.g. 354C and 349C, or 242C and 334C;
   b) comprising mutations allowing the two CH3 domains to form a knobs-into-holes structure; e.g., each of the two CH3 domains independently comprise one or more mutations at positions selected from 366, 368, 405, and 407, and, preferably, one of the CH3 domains comprises a 366W mutation and the other comprises 366S, 368A, and 407V mutations;
   c) comprising mutations capable of altering the charge properties of the interaction interface of the CH3 regions to promote formation of a heterodimer, e.g., mutation sites on the two CH3 chains are independently selected from at least one of Y391, K392, T393, T394, P395, P396, V397, L398, D399, S400, Q347, V348, Y349, T350, S354, R355, E356, E357, F405, Y407 and K409.

In some embodiments, the antibody CH3 domain is a human-derived CH3 domain, e.g., derived from human IgG1, IgG2, or IgG4; and/or
the CH3 domain comprises at least one of the following mutations capable of promoting formation of a heterodimer of the polypeptide molecule:
a) comprising mutations of at least two cysteine residues to form an interchain disulfide bond, e.g. 354C and 349C, or 242C and 334C;
b) comprising mutations allowing the two CH3 domains to form a knobs-into-holes structure; e.g., each of the two CH3 domains independently comprise one or more mutations at positions selected from 366, 368, 405, and 407, and, preferably, one of the CH3 domains comprises a 366W mutation and the other comprises 366S, 368A, and 407V mutations;
c) comprising mutations capable of altering the charge properties of the interaction interface of the CH3 regions to promote formation of a heterodimer, e.g., mutation sites on the two CH3 chains are independently selected from at least one of Y391, K392, T393, T394, P395, P396, V397, L398, D399, S400, Q347, V348, Y349, T350, S354, R355, E356, E357, F405, Y407 and K409.

In some embodiments, the functional domain is derived from a TCR constant region or a fragment thereof and optionally an antibody hinge region; and any two polypeptide chains of the multi-specific antigen binding molecule are connected via covalent or noncovalent bonds between the two chains of the functional domain;
preferably, the two chains of the functional domain are connected to the C-terminus or N-terminus, preferably the C-terminus, of any two polypeptide chains of the multi-specific antigen binding molecule via optional linkers; or
the first binding region and the second binding region are connected via covalent or noncovalent bonds between the two chains of the functional domain.

In some embodiments, the antibody hinge region is a human-derived hinge domain or a portion thereof, e.g., derived from human IgG1, IgG2, or IgG4; and/or
the TCR constant region or a fragment thereof is derived from human or murine, and is selected from a TCR α chain constant region (TRAC) and a TCR β chain constant region (TRBC); preferably, the TRAC and/or the TRBC comprise at least one cysteine mutation relative to the wild-type sequence so as to form a disulfide bond between TRAC and TRBC, more preferably, the cysteine mutations are at the following positions: position 48 of the wild-type TCR α chain constant region and position 57 of the wild-type TCR β chain constant region.

In some embodiments, the multi-specific antigen binding molecule further comprises the following constant domain connecting to the C-terminus of TRAV1 and/or TRBV1 via an optional linker:
(1) a TCR constant region or a fragment thereof, e.g. a TCR constant region including a TCR α chain constant region (TRAC) and/or a β chain constant region (TRBC);
   preferably, the TRAC and/or TRBC comprise at least one cysteine mutation relative to the wild-type sequence so as to form a disulfide bond between TRAC and TRBC, more preferably, the cysteine mutations are at the following positions: position 48 of the wild-type TCR α chain constant region and position 57 of the wild-type TCR β chain constant region;
(2) CH3, wherein the CH3 is derived from an antibody Fc domain, and preferably the CH3 in any one or both of the polypeptide chains comprises at least one mutation capable of promoting formation of a heterodimer of the polypeptide molecule;
   preferably, each of the CH3 domain independently comprises one or more mutations at positions selected from 366, 368, 405, and 407, and the two CH3 domains do not comprise identical mutations, more preferably, one of the CH3 domains comprises a T366W mutation and the other comprises T366S, L368A, and Y407V mutations;
(3) CL and CH1, wherein CL is derived from an antibody light-chain CL domain, and CH1 is derived from an antibody heavy-chain CH1 domain.

In some embodiments, the multi-specific antigen binding molecule comprises the TRAC of the invention and the TRBC of the invention connected to the C-terminus of TRAV1 and TRBV1 respectively via optional linkers. In some embodiments, the multi-specific antigen binding molecule further comprises CH1 and CL (derived from an antibody) connected to VH1 and VL1 respectively, wherein VH1, VL1, CH1, and CL form a single-chain Fab which is connected to the C-terminus of TRAV1 or TRBV1 via a linker.

In some embodiments, from the N-terminus to the C-terminus, the polypeptide chains of the multi-specific antigen binding molecule respectively comprise:
a first polypeptide chain: TRBV1-optional linker-TRBC-linker-VL1-linker-VH1-optional linker-hinge region-CH2-CH3, and a second polypeptide chain: TRAV1-optional linker-TRAC-optional linker-hinge region-CH2-CH3; or
a first polypeptide chain: TRAV1-optional linker-TRAC-linker-VL1-linker-VH1-optional linker-hinge region-CH2-CH3, and a second polypeptide chain: TRBV1-optional linker-TRBC-optional linker-hinge region-CH2-CH3; or
a first polypeptide chain: TRBV1-optional linker-TRBC-linker-VH1-linker-VL1-optional linker-hinge region-CH2-CH3, and a second polypeptide chain: TRAV1-optional linker-TRAC-optional linker-hinge region-CH2-CH3; or
a first polypeptide chain: TRAV1-optional linker-TRBC-linker-VH1-linker-VL1-optional linker-hinge region-CH2-CH3, and a second polypeptide chain: TRBV1-optional linker-TRBC-optional linker-hinge region-CH2-CH3; or
a first polypeptide chain: TRBV 1-optional linker-TRBC-linker-VL1-linker-VH1-linker-CH2-CH3, and a second polypeptide chain: TRAV 1-optional linker-TRAC-linker-CH2-CH3; or
a first polypeptide chain: TRBV1-optional linker-TRBC-linker-VL1-linker-VH1-optional linker-hinge region-CH3, and a second polypeptide chain: TRAV1-optional linker-TRAC-optional linker-hinge region-CH3; or
a first polypeptide chain: TRBV1-optional linker-TRBC-linker-VL1-linker-VH1-optional linker-hinge region-TRBC, and a second polypeptide chain: TRAV1-optional linker-TRAC-optional linker-hinge region-TRAC; or
a first polypeptide chain: TRBV1-optional linker-TRBC-linker-VL1-linker-VH1-optional linker-hinge region, and a second polypeptide chain: TRAV 1-optional linker-TRAC-optional linker-hinge region-Alb; or
a first polypeptide chain: TRBV1-optional linker-TRBC-linker-VL1-linker-VH1-linker-Alb, and a second polypeptide chain: TRAV 1-optional linker-TRAC; or
a first polypeptide chain: TRAV1-optional linker-CH3-linker-VL1-linker-VH1-optional linker- optional hinge region-CH2-CH3, and a second polypeptide chain: TRBV1-optional linker-CH3-optional linker- optional hinge region-CH2-CH3; or
a first polypeptide chain: TRAV1-optional linker-CH1-linker-VL1-linker-VH1-optional linker-optional hinge region-CH2-CH3, and a second polypeptide chain: TRBV1-optional linker-CL-optional linker- optional hinge region-CH2-CH3; or
a first polypeptide chain: TRBV1-optional linker-TRBC-linker-VHH1-optional linker-hinge region-CH2-CH3, and a second polypeptide chain: TRAV 1-optional linker-TRAC-optional linker-hinge region-CH2-CH3; or
the antibody is an antibody Fab, and wherein a first polypeptide chain: TRBV1-optional linker-TRBC-linker-VL1-CL-linker-VH1-CH1-optional linker-hinge region-CH2-CH3, and a second polypeptide chain: TRAV1-optional linker-TRAC-optional linker-hinge region-CH2-CH3; or
a first polypeptide chain: TRBV1-optional linker-TRBC-linker-VL1, second polypeptide chain: VH1-optional linker-hinge region-CH2-CH3, and a third polypeptide chain: TRAV1-optional linker-TRAC-optional linker-hinge region-CH2-CH3; or
a first polypeptide chain: TRBV1-optional linker-TRBC-linker-VH1, second polypeptide chain: VL1-optional linker-hinge region-CH2-CH3, and a third polypeptide chain: TRAV1-optional linker-TRAC-optional linker-hinge region-CH2-CH3; or
a first polypeptide chain: TRAV1-optional linker-TRAC-linker-VL1, second polypeptide chain: VH1-optional linker-hinge region-CH2-CH3, and a third polypeptide chain: TRBV1-optional linker-TRBC-optional linker-hinge region-CH2-CH3; or
a first polypeptide chain: TRAV1-optional linker-TRAC-linker-VH1, second polypeptide chain: VL1-optional linker-hinge region-CH2-CH3, and a third polypeptide chain: TRBV1-optional linker-TRBC-optional linker-hinge region-CH2-CH3; or
a first polypeptide chain: TRBV1-optional linker-TRBC-linker-VL1-optional linker-hinge region-CH2-CH3, second polypeptide chain: VH1-optional linker-hinge region-CH2-CH3, and a third polypeptide chain: TRAV1-optional linker-TRAC; or
a first polypeptide chain: TRBV1-optional linker-TRBC-linker-VH1-optional linker-hinge region-CH2-CH3, second polypeptide chain: VL1-optional linker-hinge region-CH2-CH3, and 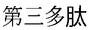 : TRAV1-optional linker-TRAC; or
a first polypeptide chain: TRAV1-optional linker-TRAC-linker-VL1-optional linker-hinge region-CH2-CH3, second polypeptide chain: VH1-optional linker-hinge region-CH2-CH3, and a third polypeptide chain: TRBV1-optional linker-TRBC; or
a first polypeptide chain: TRAV1-optional linker-TRAC-linker-VH1-optional linker-hinge region-CH2-CH3, second polypeptide chain: VL1-optional linker-hinge region-CH2-CH3, and a third polypeptide chain: TRBV1-optional linker-TRBC.

In some embodiments, the multi-specific antigen binding molecule comprises a first polypeptide chain and a second polypeptide chain; wherein:
the first polypeptide chain comprises, from the N-terminus to the C-terminus: TRBV 1-optional linker-TRBC-linker-VL1-linker-TRAV2-linker-TRBV2-linker-VH1-optional linker-hinge region-CH2-CH3, and the second polypeptide chain comprises, from the N-terminus to the C-terminus: TRAV1-optional linker-TRAC-optional linker-hinge region-CH2-CH3.

In some embodiments, the multi-specific antigen binding molecule comprises a first polypeptide chain and a second polypeptide chain; wherein:
the first polypeptide chain comprises, from the N-terminus to the C-terminus: VL2-linker-VH2-linker-TRBV1-optional linker-TRBC-linker-VL1-linker-VH1-optional linker-hinge region-CH2-CH3, and the second polypeptide chain comprises, from the N-terminus to the C-terminus: TRAV1-optional linker-TRAC-optional linker-hinge region-CH2-CH; or
the first polypeptide chain comprises, from the N-terminus to the C-terminus: VL2-linker-TRBV1-linker-VL1-linker-VH1-optional linker-hinge region-CH2-CH3, and the second polypeptide chain comprises, from the N-terminus to the C-terminus: VH2-linker-TRAV1-optional linker-hinge region-CH2-CH3; or
the first polypeptide chain comprises, from the N-terminus to the C-terminus: VL2-linker-TRBV1-optional linker-TRBC-linker-VL1-linker-VH1-optional linker-hinge region-CH2-CH3, and the second polypeptide chain comprises, from the N-terminus to the C-terminus: VH2-linker-TRAV1-optional linker-TRAC-optional linker-hinge region-CH2-CH3.

In some embodiments, the multi-specific antigen binding molecule comprises a first polypeptide chain and a second polypeptide chain; wherein:
the first polypeptide chain comprises, from the N-terminus to the C-terminus: VL2-linker-VH2-linker-TRBV1-optional linker-TRBC-linker-VL1-linker-TRAV2-linker-TRBV2-linker-VH1-optional linker-hinge region-CH2-CH3, and the second polypeptide chain comprises, from the N-terminus to the C-terminus: TRAV1-optional linker-TRAC-optional linker-hinge region-CH2-CH3.

In preferred embodiments, the multi-specific antigen binding molecule comprises a first polypeptide chain and a second polypeptide chain, wherein
the first polypeptide chain, from the N-terminus to the C-terminus, comprises TRBV1-optional linker-TRBC-linker-VL1-linker-VH1-optional linker-hinge region-CH2-CH3, and the second polypeptide chain, from the N-terminus to the C-terminus, comprises TRAV1-optional linker-TRAC-optional linker-hinge region-CH2-CH3; or
the first polypeptide chain, from the N-terminus to the C-terminus, comprises TRAV1-optional linker-TRAC-linker-VL1-linker-VH1-optional linker-hinge region-CH2-CH3, and the second polypeptide chain, from the N-terminus to the C-terminus, comprises TRBV 1-optional linker-TRBC-optional linker-hinge region-CH2-CH3; or
the first polypeptide chain, from the N-terminus to the C-terminus, comprises TRBV1-optional linker-TRBC-linker-VH1-linker-VL1-optional linker-hinge region-CH2-CH3, and the second polypeptide chain, from the N-terminus to the C-terminus, comprises TRAV1-optional linker-TRAC-optional linker-hinge region-CH2-CH3; or
the first polypeptide chain, from the N-terminus to the C-terminus, comprises TRAV1-optional linker-TRBC-linker-VH1-linker-VL1-optional linker-hinge region-CH2-CH3, and the second polypeptide chain, from the N-terminus to the C-terminus, comprises TRBV 1-optional linker-TRBC-optional linker-hinge region-CH2-CH3.

In a twelfth aspect, the present disclosure provides a nucleic acid encoding the TCR α chain constant region, the TCR β chain constant region, the TCR constant region, the polypeptide molecule, the TCR, or the multi-specific antigen binding molecule as described herein.

In a thirteenth aspect, the present disclosure provides a vector comprising the nucleic acid as described herein.

As used herein, the term "vector" is a nucleic acid molecule used as a vehicle for transferring (exogenous) genetic materials into a host cell in which the nucleic acid molecule as a vector can, for example, be replicated and/or expressed. The term "vector" encompasses, but is not limited to, plasmids, viral vectors (including retroviral vectors, lentiviral vectors, adenoviral vectors, cowpox virus vectors, polyomavirus vectors and adenovirusassociated vectors (AAVs)), phages, phasmids, cosmids and artificial chromosomes (including BACs and YACs). The vector itself is usually a nucleotide sequence, usually comprises a DNA sequence comprising an insert (transgene) and a larger sequence that serves as the "backbone" of the vector. The engineered vector usually comprises an origin for self-replication in the host cell (if stable expression of polynucleotides is desired), a selection marker and a restriction enzyme cleavage site (e.g., a polyclonal site, MCS). The vector may additionally comprise a promoter, a genetic marker, a reporter gene, a targeting sequence, and/or a protein purification tag. As known to those skilled in the art, a large number of suitable vectors are known to those skilled in the art, and many are commercially available. Examples of suitable vectors are provided in J. Sambrook et al, Molecular Cloning: A Laboratory Manual (4th ed.), Cold Spring Harbor Laboratory, Cold Spring Harbor Laboratory Press, New York (2012), which is incorporated herein by reference in their entirety.

In some embodiments, the vector is preferably selected from the group consisting of lentiviral vectors, retroviral vectors, plasmids, and DNA vectors, mRNA vectors, transposon-based vectors, and artificial chromosomes.

In a fourteenth aspect, the present disclosure provides a host cell comprising the TCR α chain constant region, the TCR β chain constant region, the TCR constant region, the polypeptide molecule, the TCR, the multi-specific antigen binding molecule, the nucleic acid, or the vector as described herein.

As used herein, the term "host cell" refers to any type of cell capable of expressing the antigen binding protein of the present disclosure. The cell may be a eukaryotic cell, e.g., a vegetation (which does not have the potential to develop into a plant), animal, fungus, or algae cell, or may be a prokaryotic cell, e.g., a bacterium or protozoan cell. The cell may be a cultured cell or a primary cell, i.e. directly isolated from an organism, e.g. a human. The cell may be an adherent cell or suspension cell, i.e. cell grown in suspension. Suitable host cell is known in the art and includes, for example, a DH5α E. coli cell, Chinese hamster ovary cell, monkey VERO cell, COS cell, HEK293 cell, and the like. For the purpose of producing the antigen binding protein of the present disclosure, the cell is preferably a mammalian cell. Most preferably, the host cell is a human cell.

In a fifteenth aspect, the present disclosure provides a conjugate comprising the polypeptide molecule, the TCR, or the multi-specific antigen binding molecule as described herein, and a chemical moiety conjugated to the polypeptide molecule, the TCR, or the multi-specific antigen binding molecule.

In some embodiments, the chemical moiety is selected from the group consisting of detectable markers, immunostimulatory molecules, and therapeutic agents. Preferably, the detectable marker is selected from the group consisting of biotins, streptavidin, enzymes or catalytically active fragments thereof, radionuclides, nanoparticles, paramagnetic metal ions, nucleic acid probes, contrast agents, and fluorogenic, phosphorescent or chemiluminescent molecules. Preferably, the immunostimulatory molecule is selected from the group consisting of cytokines (e.g. IL-2 and IFN-γ), chemokines (e.g. IL-8), platelet factors (e.g. platelet factor 4), and complement initiators. Preferably, the therapeutic agent is selected from the group consisting of immunomodulators, radioactive compounds, enzymes, chemotherapeutic agents and toxins. Other suitable therapeutic agents include small molecule cytotoxic agents, i.e. compounds having the ability to kill mammalian cells and having a molecular weight of less than 700 Daltons. Such compounds may also contain toxic metals having cytotoxic effects. In addition, it should be understood that these small molecule cytotoxic agents also include precursor drugs, i.e. compounds that decay or are transformed under physiological conditions to release cytotoxic agents. Examples of such agents include cisplatin, maytenin derivatives, rachelmycin, calicheamicin, docetaxel, etoposide, gemcitabine, isocyclic phosphorylamine, irinotecan, melphalan, mitoxantrone, sorfimer sodiumphotofrin II, temozolomide, topotecan, trimetreate glucuronate, auristatin E, vincristine, and doxorubicin; peptide cytotoxin, i.e., proteins or the fragments thereof having the ability to kill mammalian cells; e.g., ricin, diphtheritoxins, Pseudomonas bacterial exotoxin A, Dnase, and RNase; radionuclides, i.e., unstable isotopes of elements that emit one or more alpha or beta particles or gamma rays while decaying, e.g., iodine-131, rhenium-186, indium-111, yttrium-90, bismuth-210 and bismuth-213, actinium-225, and astatine-213; chelating agents which can be used to facilitate the binding of these radionuclides to molecules or multimers thereof; or heterologous protein domains, homologous protein domains, viral/bacterial protein domains, viral/bacterial peptides.

In a sixteenth aspect, the present disclosure provides a composition comprising the polypeptide molecule, the TCR, the multi-specific antigen binding molecule, the nucleic acid, the vector, the cell, or the conjugate as described herein, and a pharmaceutically acceptable carrier or excipient.

The term "excipient" includes fillers, adhesives, disintegrants, coating agents, adsorbents, anti-adhesives, flow aids, preservatives, antioxidants, flavoring agents, coloring agents, sweeteners, solvents, co-solvents, buffers, chelating agents, viscosity imparting agents, surfactants, diluents, wetting agents, carriers, diluents, preservatives, emulsifiers, stabilizers and tension regulators. It is known to those skilled in the art to select suitable excipients to prepare the composition of the present invention. Exemplary carriers for use in the composition of the present invention include saline, buffered saline, glucose, and water. Typically, the selection of a suitable excipient depends in particular on the active agent used, the disease to be treated and the desired dosage form of the composition.

Depending on the active agent employed (e.g. soluble TCR), the composition of the present disclosure may be formulated to various forms, such as solid, liquid, gaseous or lyophilized forms, and in particular may be in the form of ointments, creams, transdermal patches, gels, powders, tablets, solutions, aerosols, granules, pills, suspensions, emulsions, capsules, syrups, liquids, elixirs, infusions, tinctures or fluid extracts, or a form particularly suitable for the desired administration method. The processes of pharmaceutical production known to the present invention are shown in the 22nd edition of Remington's Pharmaceutical Sciences (Ed. MaackPublishing Co, Easton, Pa., 2012) and may include, for example, the conventional processes of mixing, lysing, granulating, sugar-coating, grinding, emulsifying, encapsulating, embedding, or lyophilizing. Compositions comprising, for example, the host cell or the soluble TCR as described herein are typically provided in liquid form and preferably comprise pharmaceutically acceptable buffering agents.

In some embodiments, the composition further comprises a second therapeutic agent, preferably second therapeutic agent is selected from antibodies, chemotherapeutic agents, and small-molecule drugs.

Preferred examples of the second therapeutic agent include known anti-cancer drugs such as cisplatin, maytenin derivatives, rachelmycin, calicheamicin, docetaxel, etoposide, gemcitabine, isocyclic phosphorylamine, irinotecan, melphalan, mitoxantrone, sorfimer sodiumphotofrin II, temozolomide, topotecan, trimetreate glucuronate, auristatin E, vincristine, and doxorubicin; and peptide cytotoxin, such as ricin, diphtheritoxins, Pseudomonas bacterial exotoxin A, Dnase, and RNase; radionuclides, e.g. iodine 131, rhenium 186, indium 111, iridium 90, bismuth 210 and 213, actinium 225 and astatine 213; prodrugs, e.g. antibody-directed enzyme prodrugs; immunostimulants, e.g. IL-2, chemokines e.g. IL-8, platelet factor 4; antibodies or fragments thereof, e.g. anti-CD3 antibodies or fragments thereof; complement initiators; heterologous protein domains, homologous protein domains, viral/bacterial protein domains, and viral/bacterial peptides.

In a seventeenth aspect, the present disclosure provides a method of treating a disease in a subject, comprising administering to a subject an effective amount of the polypeptide molecule, the TCR, the multi-specific antigen binding molecule, the nucleic acid, the vector, the cell, the conjugate, or the composition as described herein.

In some embodiments, the disease is selected from cancers, infectious diseases, autoimmune diseases, and inflammatory diseases.

Cancer can be any cancer, such as cancers of the hematologic system, cancers of the central and peripheral nervous systems, cancers of the lymphoid lineage, cancers of the myeloid lineage, cancers of mesenchymal origin, solid tumors, and the like. Examples of cancers include but not limited to acute lymphocytic cancer, acute myeloid leukemia, alveolar rhabdomyosarcoma, bone cancer, brain cancer, breast cancer, anal cancer, anal canal cancer or rectal anal cancer, eye cancer, intrahepatic cholangiocarcinoma, joint cancer, neck cancer, gallbladder cancer or pleural cancer, nasal cancer, nasal cavity cancer or middle ear cancer, oral cancer, vaginal cancer, vulvar cancer, chronic lymphocytic leukemia, chronic myeloid cancer, colon cancer, esophageal cancer, cervical cancer, gastrointestinal carcinoid tumors, glioma, Hodgkin's lymphoma, hypopharyngeal cancer, kidney cancer, laryngeal cancer, liver cancer, lung cancer, malignant mesothelioma, melanoma, multiple myeloma, nasopharyngeal carcinoma, non-Hodgkin's lymphoma, oropharyngeal cancer, ovarian cancer, penile cancer, pancreatic cancer, peritoneal cancer, omental cancer and mesenteric cancer, pharyngeal cancer, prostate cancer, rectal cancer, kidney cancer, skin cancer, small bowel cancer, soft tissue cancer, stomach cancer, testicular cancer, thyroid cancer, uterine cancer, ureteral cancer, and bladder cancer.

Infectious diseases are diseases caused by infection with pathogens and include infectious diseases and noninfectious diseases. Pathogens that cause infectious diseases include viruses, bacteria, mycoplasma, chlamydia, rickettsia, prions, fungi, spirochetes, and parasites, among others. Examples of viruses include but not limited to HPV, CMV, HBV, EBV, herpesviruses, human immunodeficiency virus (HIV), influenza viruses, and coronaviruses.

Common autoimmune and inflammatory diseases include, without limitation, rheumatoid arthritis, systemic lupus erythematosus, ankylosing spondylitis, psoriatic arthritis, type 1 diabetes mellitus (autoimmune), Sjögren's syndrome, Hashimoto's thyroiditis, Graves' disease (diffuse toxic goiter), autoimmune hemolytic anemia, immune thrombocytopenia (ITP), inflammatory bowel disease (e.g., ulcerative colitis and Crohn's disease), psoriasis, glomerulonephritis, nephrotic syndrome, anti-glomerular basement membrane (anti-GBM) disease (Goodpasture syndrome), membranous nephropathy, systemic sclerosis, polymyositis, myasthenia gravis, pemphigus, vitiligo, autoimmune diseases of the central nervous system, celiac disease, autoimmune gastritis, primary biliary cholangitis, autoimmune hepatitis, autoimmune oophoritis, autoimmune orchitis, idiopathic leukopenia, primary adrenal cortical atrophy (autoimmune adrenalitis), antiphospholipid antibody syndrome, post-infection fibrosis, post-myocardial infarction fibrosis, hepatic fibrosis, and keloid.

In some embodiments, the dose administered to a subject may vary with the regimen, the drug employed, the route of administration, the site to be treated and the subject to be treated. However, the dose should be sufficient to provide a therapeutic response. A clinician can determine an effective amount for treating a medical condition in a human or other subject. The precise amount required to be therapeutically effective may depend on numerous factors, such as the activity of the active agent and the route of administration.

Doses of the polypeptide molecule, the TCR, the multi-specific antigen binding molecule, the cell, the conjugate, or the composition of the present invention can be administered to a mammal in a single administration or as a series of subdoses over an appropriate period of time, e.g., as needed, once daily, semiweekly, weekly, biweekly, semimonthly, bimonthly, semiannually, or annually. A unit dose containing an effective amount of the polypeptide molecule, the TCR, the multi-specific antigen binding molecule, the cell, the conjugate, or the composition can be administered as a single daily dose, or the total daily dose can be administered, as needed, in two, three, four, or more divided doses per day.

An appropriate mode of administration may be selected by a physician. The route of administration can be parenteral, for example by injection, intranasal administration, pulmonary administration, or transdermal administration. Systemic or local administration may be carried out by intravenous, intramuscular, intraperitoneal, or subcutaneous injection. In some embodiments, a polypeptide molecule, a conjugate, or a pharmaceutical composition is selected for parenteral delivery, inhalation, or delivery through the gastrointestinal tract, e.g., oral administration. The dosage and regimen can vary with the subject's weight, age, and condition, and may be selected appropriately.

In some embodiments, the method further comprises administering a second therapeutic agent. In certain embodiments, the polypeptide molecule, the TCR, the multi-specific antigen binding molecule, the cell, the conjugate, or the composition of the present invention is administered before, substantially simultaneously with, or after the second therapeutic agent. Preferably, the second therapeutic agent is selected from antibodies, chemotherapeutic agents, and small-molecule drugs. Preferred examples of the second therapeutic agent are as described above.

In an eighteenth aspect, the present disclosure provides a TCR comprising a TCR α chain variable region (TRAV) and a TCR β chain variable region (TRBV), wherein the TRAV comprises α chain CDR1, CDR2, and CDR3 having amino acid sequences as shown in SEQ ID NO.56 (DSSMTY), SEQ ID NO.57 (IFAFESM) and SEQ ID NO.58 (AGSGGGTDK), respectively, or functional variants formed by insertion, deletion, or substitution of one or several amino acids; and/or the TRBV comprises β chain CDR1, CDR2, and CDR3 having amino acid sequences as shown in SEQ ID NO.59 (DFEATT), SEQ ID NO.60 (TDYGSKA) and SEQ ID NO.61 (SAREPGQGPWE), respectively, or functional variants formed by insertion, deletion, or substitution of one or several amino acids.

In some embodiments, the TRAV comprises α chain CDR1, CDR2, and CDR3 having amino acid sequences as shown in SEQ ID NO.56 (DSSMTY), SEQ ID NO.57 (IFAFESM) and SEQ ID NO.58 (AGSGGGTDK), respectively; and/or the TRBV comprises β chain CDR1, CDR2, and CDR3 having amino acid sequences as shown in SEQ ID NO.59 (DFEATT), SEQ ID NO.60 (TDYGSKA) and SEQ ID NO.61 (SAREPGQGPWE), respectively.

In some embodiments, the TRAV comprises an amino acid sequence having at least 80%, at least 85%, at least 90%, at least 95%, or 100% sequence identity to SEQ ID NO.30, and/or the TRBV comprises an amino acid sequence having at least 80%, at least 85%, at least 90%, at least 95%, or 100% sequence identity to SEQ ID NO.31.

In a nineteenth aspect, the present disclosure provides a TCR comprising a TCR α chain variable region (TRAV) and a TCR β chain variable region (TRBV), wherein the TRAV comprises α chain CDR1, CDR2, and CDR3 having amino acid sequences as shown in SEQ ID NO.62 (DSSMTY), SEQ ID NO.63 (IFFYQDM) and SEQ ID NO.64 (AGSGGGTDK), respectively, or functional variants formed by insertion, deletion, or substitution of one or several amino acids; and/or the TRBV comprises a β chain CDR1, CDR2, and CDR3 having amino acid sequences as shown in SEQ ID NO.65 (DFEATT), SEQ ID NO.66 (TDYGSKA) and SEQ ID NO.67 (SAREPGQAWSD), respectively, or functional variants formed by insertion, deletion, or substitution of one or several amino acids.

In some embodiments, the TRAV comprises α chain CDR1, CDR2, and CDR3 having amino acid sequences as shown in SEQ ID NO.62 (DSSMTY), SEQ ID NO.63 (IFFYQDM) and SEQ ID NO.64 (AGSGGGTDK), respectively, and/or the TRBV comprises β chain CDR1, CDR2, and CDR3 having the amino acid sequences as shown in SEQ ID NO.65 (DFEATT), SEQ ID NO.66 (TDYGSKA) and SEQ ID NO.67 (SAREPGQAWSD), respectively.

In some embodiments, the TRAV comprises an amino acid sequence having at least 80%, at least 85%, at least 90%, at least 95%, or 100% sequence identity to SEQ ID NO.32, and the TRBV comprises an amino acid sequence having at least 80%, at least 85%, at least 90%, at least 95%, or 100% sequence identity to SEQ ID NO.33.

In a twentieth aspect, the present disclosure provides the TCR α chain constant region, the TCR β chain constant region, the TCR constant region, the polypeptide molecule, the TCR, the multi-specific antigen binding molecule, the nucleic acid, the vector, the cell, the conjugate, or the composition as described herein for use in the manufacture of a medicament for treating or preventing a disease in a subject.

In a twenty-first aspect, the present disclosure provides the TCR α chain constant region, the TCR β chain constant region, the TCR constant region, the polypeptide molecule, the TCR, the multi-specific antigen binding molecule, the nucleic acid, the vector, the cell, the conjugate, or the composition as described herein, for treating or preventing a disease in a subject.

In a twenty-second aspect, the present disclosure provides a method for detecting a disease and/or condition in a subject caused by a positive antigen, wherein the method comprises: (i) contacting a sample obtained from the subject with the polypeptide molecule, the TCR, the multi-specific antigen binding molecule, the nucleic acid, the vector, the cell, the conjugate, or the composition as described herein; and (ii) detecting the presence of the positive antigen in the sample, wherein the presence of the positive antigen indicates a disease and/or condition caused by the positive antigen.

In a twenty-third aspect, the present disclosure provides a method for detecting a disease and/or condition in a subject caused by a positive antigen, wherein the method comprises administering to the subject an effective amount of the polypeptide molecule, the TCR, the multi-specific antigen binding molecule, the nucleic acid, the vector, the cell, the conjugate, or the composition as described herein that is conjugated to a detectable label (e.g., radionuclides).

In a twenty-fourth aspect, the present disclosure provides a use of the polypeptide molecule, the TCR, the multi-specific antigen binding molecule, the nucleic acid, the vector, the cell, the conjugate, or the composition of the present disclosure in the preparation of a product (e.g., a kit) for detecting (e.g., diagnosing) the presence of a positive antigen in a test sample.

In a twenty-fifth aspect, the present disclosure provides the polypeptide molecule, the TCR, the multi-specific antigen binding molecule, the nucleic acid, the vector, the cell, the conjugate, or the composition as described herein, for detecting (e.g., diagnosing) the presence of a positive antigen in a test sample. The presence of the positive antigen indicates a disease and/or condition caused by the positive antigen.

In a twenty-sixth aspect, the present disclosure provides a kit comprising the polypeptide molecule, the TCR, the multi-specific antigen binding molecule, the nucleic acid, the vector, the cell, the conjugate, or the composition as described herein.

In a specific embodiment, the kit is used to detect (e.g., diagnose) a disease and/or condition in a subject caused by a positive antigen, and comprises the polypeptide molecule, the TCR, the multi-specific antigen binding molecule, the nucleic acid, the vector, the cell, the conjugate, or the composition as described herein.

In some embodiments of the methods and uses of the present invention, the disease or condition is selected from cancers, infectious diseases, autoimmune diseases, and inflammatory diseases. The cancers, infectious diseases, autoimmune diseases, and inflammatory diseases are as defined above.

In some embodiments of the methods and uses of the present invention, the positive antigen is an HPV positive antigen, more preferably an HPV E7 positive antigen. In some embodiments, the HPV-positive disease or condition is selected from HPV infections, HPV-associated precancerous lesions, and HPV-associated cancers. Preferably, the cancer is preferably selected from cervical cancer, head and neck cancer, oropharyngeal cancer, esophageal adenocarcinoma, anal cancer, anal canal cancer, rectal cancer, vaginal cancer, vulvar cancer, and penile cancer.

In some embodiments of the methods and uses of the present invention, the positive antigen is a KRAS mutation positive antigen, e.g., a G12V mutation or a G12D mutation. In some embodiments, the disease and/or condition caused by the KRAS mutation positive antigen is selected from KRAS mutation-induced tumors, precancerous lesions, and cancers. In some embodiments, the cancer is selected from solid tumors and hematologic tumors. More preferably, the tumors include at least one of pancreatic cancer, colon cancer, rectal cancer, colorectal cancer, endometrial cancer, lung cancer, melanoma, prostate cancer, cholangiocarcinoma, cervical cancer, bladder cancer, liver cancer, and breast cancer.

In some embodiments of the methods and uses of the present invention, the positive antigen is a MAGE positive antigen, preferably a MAGE-A1 positive antigen. In some embodiments, the MAGE-A1 positive cancers are selected from lung cancer, liver cancer (e.g., hepatocellular carcinoma), gastric cancer, melanoma, esophageal cancer, colorectal cancer, cervical cancer, breast cancer, bladder cancer (e.g., bladder urothelial carcinoma), ovarian cancer (e.g., ovarian serous cystadenocarcinoma), head and neck cancer (e.g., head and neck squamous cell carcinoma), uterine cancer, endometrial cancer, cholangiocarcinoma, prostate cancer, adrenocortical carcinoma, mesothelioma, sarcoma, glioblastoma multiforme, pheochromocytoma, neuroblastoma, retinoblastoma, paraganglioma, and thymic carcinoma.

### Examples

The present invention is further elaborated by the following specific examples. It should be understood that these examples are intended to illustrate the invention only and are not intended to limit the scope of the invention. Experimental methods for which specific conditions are not indicated in the following examples are performed through conventional conditions in the art, e.g., those described in Sambrook and Russeii et al, Molecular Cloning: a Laboratory Manual (Third Edition) (2001), CSHL Press, or through those as recommended by the manufacturer. Unless otherwise stated, the experimental materials and reagents used in the following examples are commercially available.

### Example 1. Information on mutant sequences of the TCR constant region

By introducing a novel disulfide bond into the truncated human TCR constant regions as shown in SEQ ID NO.2 and SEQ ID NO.15, the success rate and yield of soluble expression of TCR were improved; however, for TCRs with a relatively poor baseline expression level, such a strategy still cannot successfully achieve soluble expression.

To increase the soluble expression of TCRs, the inventors performed computer prediction using SEQ ID NO.2 and SEQ ID NO.15 as parental sequences, introduced a single amino acid site mutation into the TCR constant region, and combined wet-lab validation, ultimately screened out beneficial mutations that significantly improved the soluble expression properties of TCRs. The amino acid sequences of the wild-type TCR constant region, the parental TCR constant region, and the TCR constant regions screened by the present invention are as follows (mutation sites are numbered according to the IMGT nomenclature):
SEQ ID NO.1: Homo sapiens TRAC*01 
NIQNPDPAVYQLRDSKSSDKSVCLFTDFDSQTNVSQSKDSDVYITDKTVLDMRSMDFKSNSAVAWSN
KSDFACANAFNNSIIPEDTFFPSP
SEQ ID NO.2: Parental TRAC, comprising N1.2>K, T84>C, and an FFPSP deletion at positions 120-124, relative to SEQ ID NO.1 
NIQKPDPAVYQLRDSKSSDKSVCLFTDFDSQTNVSQSKDSDVYITDKCVLDMRSMDFKSNSAVAWSN
KSDFACANAFNNSIIPEDT
SEQ ID NO.3: Introducing a single site mutation Q6>R relative to Seq ID No.2 
NIQKPDPAVYRLRDSKSSDKSVCLFTDFDSQTNVSQSKDSDVYITDKCVLDMRSMDFKSNSAVAWSN
KSDFACANAFNNSIIPEDT
SEQ ID NO.4: Introducing a single site mutation S21>I relative to Seq ID No.2 
NIQKPDPAVYQLRDSKSSDKIVCLFTDFDSQTNVSQSKDSDVYITDKCVLDMRSMDFKSNSAVAWSN
KSDFACANAFNNSIIPEDT
SEQ ID NO.5: Introducing a single site mutation K83>P relative to Seq ID No.2 
NIQKPDPAVYQLRDSKSSDKSVCLFTDFDSQTNVSQSKDSDVYITDPCVLDMRSMDFKSNSAVAWSN
KSDFACANAFNNSIIPEDT
SEQ ID NO.6: Introducing a single site mutation S84.6>D relative to Seq ID No.2 
NIQKPDPAVYQLRDSKSSDKSVCLFTDFDSQTNVSQSKDSDVYITDKCVLDMRDMDFKSNSAVAWSN
KSDFACANAFNNSIIPEDT
SEQ ID NO.7: Introducing a single site mutation S84.6>E relative to Seq ID No.2 
NIQKPDPAVYQLRDSKSSDKSVCLFTDFDSQTNVSQSKDSDVYITDKCVLDMREMDFKSNSAVAWSN
KSDFACANAFNNSIIPEDT
SEQ ID NO.8: Introducing a single site mutation S84.6>N relative to Seq ID No.2 
NIQKPDPAVYQLRDSKSSDKSVCLFTDFDSQTNVSQSKDSDVYITDKCVLDMRNMDFKSNSAVAWSN
KSDFACANAFNNSIIPEDT
SEQ ID NO.9: Introducing a single site mutation I114>E relative to Seq ID No.2 
NIQKPDPAVYQLRDSKSSDKSVCLFTDFDSQTNVSQSKDSDVYITDKCVLDMRSMDFKSNSAVAWSN
KSDFACANAFNNSEIPEDT
SEQ ID NO.10: Introducing multiple-site mutations K83>P and S84.6>D relative to Seq ID No.2 
NIQKPDPAVYQLRDSKSSDKSVCLFTDFDSQTNVSQSKDSDVYITDPCVLDMRDMDFKSNSAVAWSN
KSDFACANAFNNSIIPEDT
SEQ ID NO.11: Introducing multiple-site mutations S21>I and S84.6>D relative to Seq ID No.2 
NIQKPDPAVYQLRDSKSSDKIVCLFTDFDSQTNVSQSKDSDVYITDKCVLDMRDMDFKSNSAVAWSN
KSDFACANAFNNSIIPEDT
SEQ ID NO.12: Introducing multiple-site mutations S21>I and K83>P relative to Seq ID No.2 
NIQKPDPAVYQLRDSKSSDKIVCLFTDFDSQTNVSQSKDSDVYITDPCVLDMRSMDFKSNSAVAWSN
KSDFACANAFNNSIIPEDT
SEQ ID NO.13: Introducing multiple-site mutations S21>I, K83>P, and S84.6>D relative to Seq ID No.2 
NIQKPDPAVYQLRDSKSSDKIVCLFTDFDSQTNVSQSKDSDVYITDPCVLDMRDMDFKSNSAVAWSN
KSDFACANAFNNSIIPEDT
SEQ ID NO.14: Homo sapiens TRBC2*01 
EDLKNVFPPEVAVFEPSEAEISHTQKATLVCLATGFYPDHVELSWWVNGKEVHSGVSTDPQPLKEQPA
LNDSRYCLSSRLRVSATFWQNPRNHFRCQVQFYGLSENDEWTQDRAKPVTQIVSAEAWGRAD
SEQ ID NO.15: Parental TRBC, comprising S79>C, C85.1>A, and N97>D relative to SEQ ID NO.14 
EDLKNVFPPEVAVFEPSEAEISHTQKATLVCLATGFYPDHVELSWWVNGKEVHSGVCTDPQPLKEQP
ALNDSRYALSSRLRVSATFWQDPRNHFRCQVQFYGLSENDEWTQDRAKPVTQIVSAEAWGRAD
SEQ ID NO.16: Introducing a single site mutation S92>P relative to Seq ID No.15 
EDLKNVFPPEVAVFEPSEAEISHTQKATLVCLATGFYPDHVELSWWVNGKEVHSGVCTDPQPLKEQP
ALNDSRYALSSRLRVPATFWQDPRNHFRCQVQFYGLSENDEWTQDRAKPVTQIV SAEAWGRAD
SEQ ID NO.17: Introducing a single site mutation S92>E relative to Seq ID No.15 
EDLKNVFPPEVAVFEPSEAEISHTQKATLVCLATGFYPDHVELSWWVNGKEVHSGVCTDPQPLKEQP
ALNDSRYALSSRLRVEATFWQDPRNHFRCQVQFYGLSENDEWTQDRAKPVTQIV SAEAWGRAD
SEQ ID NO.18: Introducing a single site mutation S92>D relative to Seq ID No.15 
EDLKNVFPPEVAVFEPSEAEISHTQKATLVCLATGFYPDHVELSWWVNGKEVHSGVCTDPQPLKEQP
ALNDSRYALSSRLRVDATFWQDPRNHFRCQVQFYGLSENDEWTQDRAKPVTQIV SAEAWGRAD
SEQ ID NO.19: Introducing a single site mutation S92>L relative to Seq ID No.15 
EDLKNVFPPEVAVFEPSEAEISHTQKATLVCLATGFYPDHVELSWWVNGKEVHSGVCTDPQPLKEQP
ALNDSRYALSSRLRVLATFWQDPRNHFRCQVQFYGLSENDEWTQDRAKPVTQIV SAEAWGRAD
SEQ ID NO.20: Introducing a single site mutation S92>N relative to Seq ID No.15 
EDLKNVFPPEVAVFEPSEAEISHTQKATLVCLATGFYPDHVELSWWVNGKEVHSGVCTDPQPLKEQP
ALNDSRYALSSRLRVNATFWQDPRNHFRCQVQFYGLSENDEWTQDRAKPVTQIV SAEAWGRAD
SEQ ID NO.21: Introducing a single site mutation T94>D relative to Seq ID No.15 
EDLKNVFPPEVAVFEPSEAEISHTQKATLVCLATGFYPDHVELSWWVNGKEVHSGVCTDPQPLKEQP
ALNDSRYALSSRLRVSADFWQDPRNHFRCQVQFYGLSENDEWTQDRAKPVTQIV SAEAWGRAD
SEQ ID NO.22: Introducing a single site mutation Q107>L relative to Seq ID No.15 
EDLKNVFPPEVAVFEPSEAEISHTQKATLVCLATGFYPDHVELSWWVNGKEVHSGVCTDPQPLKEQP
ALNDSRYALSSRLRVSATFWQDPRNHFRCQVLFYGLSENDEWTQDRAKPVTQIV SAEAWGRAD
SEQ ID NO.23: Introducing multiple-site mutations T94>D and Q107>L relative to Seq ID No.15

EDLKNVFPPEVAVFEPSEAEISHTQKATLVCLATGFYPDHVELSWWVNGKEVHSGVCTDPQPLKEQP
ALNDSRYALSSRLRVSADFWQDPRNHFRCQVLFYGLSENDEWTQDRAKPVTQIV SAEAWGRAD

### Example 2. Selection of mutation strategies and methods

In principle, the native-state stability and expressibility of a protein are related, although the former is a thermodynamic property whereas the latter is governed mainly by the folding trajectory. This relationship arises because protein stability is determined by the free-energy difference between the folded state and the misfolded or unfolded states. Misfolded states can be regarded as traps that prevent nascent chains from folding into the native state, thereby hindering the folding trajectory and reducing the yield of natively folded protein; misfolded states may also result in terminal aggregation. Accordingly, the free-energy gap between the folded and misfolded states is also one of the determinants of expressibility. As illustrated schematically in FIG. 2, the goal of stability design can be considered as follows: the energy landscape of a marginally stable target protein contains many misfolded states whose energies are close to that of the native state and thus a large number of misfolded states may exist. In the native expression host, the proteostasis network suppresses the formation and accumulation of such misfolded states; however, misfolded states may limit expression in heterologous hosts and restrict the lifetime of the protein in vitro. The goal of stability design is to increase the energy gap between the native folded state and the misfolded or unfolded states. This can be achieved by lowering the energy of the correctly folded state while also eliminating or destabilizing as many misfolded states as possible, so that the remaining misfolded states are maintained at higher energy relative to the new correctly folded state, thereby improving protein expressibility.

As macromolecular organic compounds, proteins exist in water in a dispersed (colloidal) state; strictly speaking, proteins do not have a true thermodynamic solubility in water, and the amount or level of dispersion in water is accordingly referred to as protein solubility. Phenomenologically, protein solubility is influenced by pH, ionic strength, temperature, and solvent type; ultimately, however, it is determined by the amino-acid sequence. By optimizing the amino-acid sequence, reducing intermolecular aggregation, and increasing molecular dispersibility in the solvent system constitute another important approach to increasing protein yield.

In light of the foregoing, the inventors established strategies and methods for optimizing the soluble-expression performance of TCR fusion proteins:
a. Optimize soluble expression along two dimensions: increasing protein stability and improving solubility;
b. Target the TCR constant region and predict stability and solubility after single-amino-acid mutations in this region by computational (bioinformatic) methods; select mutations with significant gain effects from the computational results for wet-lab validation;
c. Use the open-source algorithms I-Mutant 2.0 and SODA to calculate protein stability and solubility, respectively;
d. Combine beneficial mutations verified by wet experiments in limited combinations to determine whether synergistic gain effects exist among beneficial amino-acid mutations at different spatial positions.

### Example 3. Algorithmic prediction and wet-lab validation

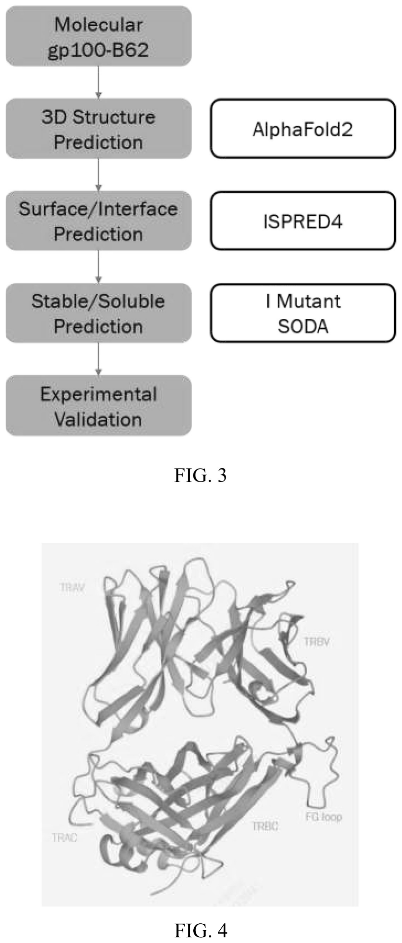

To facilitate functional validation of the TCR fusion protein after expression, the inventors constructed the gp100-TCR sequence into a self-developed TCR fusion protein format (format 62, B62#) for mutation prediction, expression of mutated the protein, and validation of protein activity. The overall experimental flow is as shown in FIG. 3.

Format 62 comprises the following two polypeptide chains:
The first polypeptide chain: TRBV-TRBC-first linker-VL-second linker-VH-hinge region-CH2-CH3 Knob
The second polypeptide chain: TRAV-TRAC-hinge region-CH2-CH3 Hole
The amino acid sequences of the components are as follows:
   TRAV 
SQQGEEDPQALSIQEGENATMNCSYKTSINNLQWYRQNSGRGLVHLILIRSNEREKHSGRLRVTLDTS
KKSSSLLITASRAADTASYFCATDGSTPMQFGKGTRLSVI (SEQ ID NO.94)
   TRBV 
DGGITQSPKYLFRKEGQNVTLSCEQNLNHDAMYWYRQDPGQGLRLIYYSWAQGDFQKGDIAEGYSV
SREKKESFPLTVTSAQKNPTAFYLCASSWGAPYEQYFGPGTRLTVT (SEQ ID NO.95)
   TRAC: SEQ ID NO.2
   TRBC: SEQ ID NO.15
   VH 
EVQLVESGGGLVQPGGSLRLSCAASGYSFTGYTMNWVRQAPGKCLEWVALINPYKGVSTYNQKFKD
RFTISVDKSKNTAYLQMNSLRAEDTAVYYCARSGYYGDSDWYFDVWGQGTLVTVSS (SEQ ID NO.
96)
   VL 
DIQMTQSPSSLSASVGDRVTITCRASQDIRNYLNWYQQKPGKAPKLLIYYTSRLESGVPSRFSGSGSGT
DYTLTISSLQPEDFATYYCQQGNTLPWTFGCGTKVEIK (SEQ ID NO. 97)
   First linker
      GGGGSGGGGS (SEQ ID NO.98)
   Second linker
      GGGGSGGGGSGGGGS (SEQ ID NO.99)
   Hinge region-CH2-CH3 Hole 
EPKSSDKTHTCPPCPAPPVAGPSVFLFPPKPKDTLMISRTPEVTCVVVDVSHEDPEVKFNWYVDGVEV
HNAKTKPREEQYQSTYRVVSVLTVLHQDWLNGKEYKCKVSNKALPASIEKTISKAKGQPREPQVCTL
PPSRDELTKNQVSLSCAVKGFYPSDIAVEWESNGQPENNYKTTPPVLDSDGSFFLVSKLTVDKSRWQQ
GNVFSCSVMHEALHNHYTQKSLSLSPGK (SEQ ID NO. 100)
   Hinge region-CH2-CH3 Knob

EPKSSDKTHTCPPCPAPPVAGPSVFLFPPKPKDTLMISRTPEVTCVVVDVSHEDPEVKFNWYVDGVEV
HNAKTKPREEQYQSTYRVVSVLTVLHQDWLNGKEYKCKVSNKALPASIEKTISKAKGQPREPQVYTL
PPCRDELTKNQVSLWCLVKGFYPSDIAVEWESNGQPENNYKTTPPVLDSDGSFFLYSKLTVDKSRWQ
QGNVFSCSVMHEALHNHYTQKSLSLSPGK (SEQ ID NO. 101)

### 3.1 Computer prediction

### 3.1.1 Three-dimensional structure prediction

AlphaFold2 exhibits exceptionally strong applicability in the three-dimensional structure prediction of various proteins, with particularly outstanding performance in antibody structure prediction. The computationally predicted structures are highly consistent with the wet-lab validated structures. Using this algorithm, the inventors successfully achieved three-dimensional structure prediction for TCRs and TCR fusion proteins; the predicted three-dimensional structures are as shown in FIG.s 4 and 5.

### 3.1.2 Prediction and screening of mutated amino acid sites

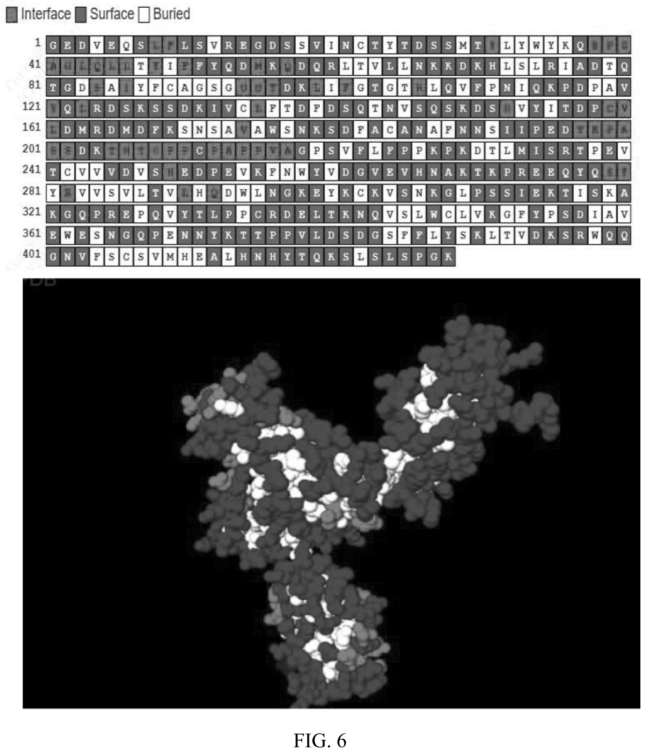

ISPRED4 is a web server that predicts protein-protein interaction (PPI) sites starting from protein structure. Trained by deep machine learning, ISPRED4 can extract several features from protein sequences and structures to predict the interaction state of residues on the protein surface and to predict whether each amino acid is located on the protein surface, interaction interface, or buried in the protein core. An exemplary PPI prediction result for A chain of the TCR fusion protein gp100-B62 is as shown in FIG. 6.

Based on the prediction results for both chains of the TCR fusion protein gp100-B62, a total of 130 amino acid sites were screened, which either located in PPI regions or located on the protein surface but are weakly hydrophilic. We considered that mutating these sites would be more likely to improve the expression properties of the TCR fusion protein.

### 3.1.3 Prediction and screening of mutation stability and solubility

The 130 amino acid sites selected above were then subjected to random saturation mutagenesis (each amino acid site mutated to the other 19 amino acids) using the I-Mutant and SODA algorithms, and the stability ("Stable") and solubility ("Soluble") of the mutated protein were scored and ranked by the algorithms. Finally, out of a total of 2,470 mutant sequences, 150 mutant sequences were selected for protein expression to validate the effects of these mutations on the activity of the TCR fusion proteins through wet-lab experiments. The selection principles were as follows:
a. Multisite mutations at the same amino acid site confer gain-of-function, only the top two with the highest scores were selected for validation;
b. Consider the I-Mutant and SODA scores separately and select the top 100 for each;
c. Take the union of the I-Mutant and SODA top-100 mutant sequences, remove duplicates, and finally screen mutants with increases in both scores (50 clones) and mutants with an increase in only one score (100 clones).

### 3.2 Wet-lab validation

### 3.2.1 Validation of the activity of TCR fusion proteins with single site mutation

A total of 150 clones entered wet-lab validation. To facilitate rapid expression of the protein molecules and activity validation, CHO-K1 cells were transiently transfected in 12-well plates to express the proteins. Supernatants harvested on day 7 of expression were serially diluted, and the activity of proteins in the supernatants was detected by ELISPOT. Here, protein activity was used as the indicator to evaluate the comprehensive gain effects on the activity and expression level of the TCR fusion protein brought by amino acid mutations. The specific operations were as follows.

CHO-K1 cells were seeded into 12-well plates and placed in a CO₂ incubator for culture at 37°C with 6% CO₂. When cell confluence reached 80%-95%, the plasmid (µg) to transfection reagent (µL) ratio was set at 1:2.5 to transfect the plasmid encoding the protein to be screened into the cells using Mirus transfection reagent. The cells were then placed in a CO₂ incubator and cultured at 37 °C with 6% CO₂. On culture day 7, 1 mL of culture supernatant was pirated from each well into an EP tube, centrifuged at 500 g for 5 min, and the supernatant was transferred to a new EP tube. ELISPOT detection was performed using 5-fold and 20-fold dilutions of the supernatant. The parental TCR constant regions (TRAC and TRBC sequences as shown in SEQ ID NO.2 and SEQ ID NO.15, respectively) served as the wild-type control.

For the ELISPOT assay, T2 target cells with a viable cell density of 1×10⁶ cells/mL were suspended in centrifuge tube, then the corresponding concentrations of epitope peptides were added, and the mixture was incubated at 37°C for 2 hours, with gentle flicking to mix thoroughly every 20 minutes during the incubation period. Effector cells (PBMCs) were resuspended in 1640 medium supplemented with 10% FBS, and the cell density was adjusted to 1×10⁶ cells/mL. In accordance with the experimental protocol, the corresponding quantities and types of effector cells, target cells, and protein drugs were added to the ELISPOT wells. The plates were then placed in an incubator at 37°C with 5% CO₂ for incubation lasting 16 - 20 hours. The medium and cells in the wells were discarded, and the plates were washed with PBS. The plates were incubated with biotin - labeled antibodies at 37°C for 2 hours, followed by another washing step. TMB chromogenic substrate solution was added to the wells for color development. When distinct spots were observed, the color development was terminated, and the ELISPOT plates were subjected to spot counting.

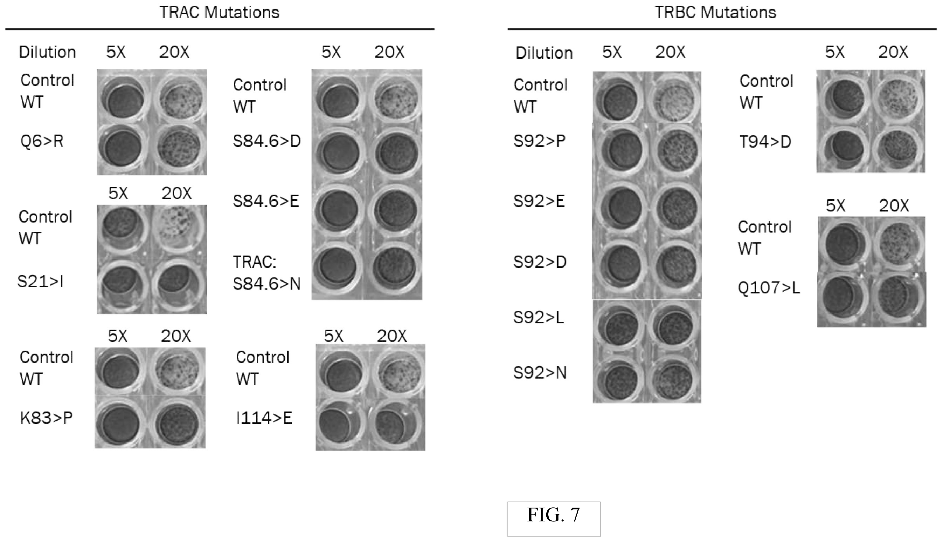

Through multiple rounds of protein expression and ELISPOT experiments, 14 TCR constant region variants with single site mutant that significantly improved in vitro activity of the TCR fusion protein were screened out. These 14 significantly beneficial mutants were approximately evenly distributed between TRAC and TRBC and included seven amino acid mutations located in TRAC (Q6R, S21I, K83P, S84.6D, S84.6E, S84.6N, I114E) and seven amino acid mutations located in TRBC (S92P, S92E, S92D, S92L, S92N, T94D, Q107L). ELISPOT results thereof are as shown in FIG. 7.

The ELISPOT results in FIG. 7 show that, under 20-fold dilution of the experimental group supernatants of the mutants, PBMC activation activity was significantly stronger than that of the wild-type control group; this increase in activity may derive from gains in protein yield, activity, and other aspects.

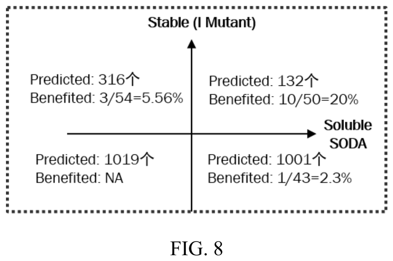

In addition, the results of the ELISPOT validation were compared with dual-parameter Stable/Soluble scoring prediction results in Example 3.1.3, and the results are shown in FIG. 8. The results indicated that among the experimental groups with increased scores in dual-parameter Stable/Soluble scoring, 20% of the mutant clones showed significant activity benefits. In contrast, the positive rates in the experimental groups with increased scores in single-parameter Soluble scoring were significantly lower (Stable: 5.56%; Soluble: 2.3%). These findings suggest that clones with increased scores in dual-parameter Stable/Soluble scoring are more likely to significantly improve protein activity.

### 3.2.2 Expression validation of TCR fusion proteins bearing multisite mutations

The inventors previously independently developed a TCR Engager format (B62#). In early studies this format exhibited excellent expression stability and supported soluble expression of TCRs derived from different species, but a few special TCRs (such as the KRAS G12V-specific TCR, CR-KVA11-N03) fail to achieve successful expression after being incorporated into this format.

The amino acid sequences of the CR-KVA11-N03 TCR are as follows:
TRAV 
RKEVEQDPGPFNVPEGATVAFNCTYSNSASQSFFWYRQDCRKEPKLLMSVYSSGNEDGRFTAQLNRA
SQYISLLIRDSKLSDSATYLCVVPGGTGGGNKLTFGTGTQLKVEL (SEQ ID NO. 24)
TRBV

DTAVSQTPKYLVTQMGNDKSIKCEQNLGHDTMDWYKQDSKKFLKIMFSYNNKELIINETVPNRFSPK
SPDKAHLNLHINSLELGDSAVYFCASSHWGAQETQYFGPGTRLLVL (SEQ ID NO. 25)

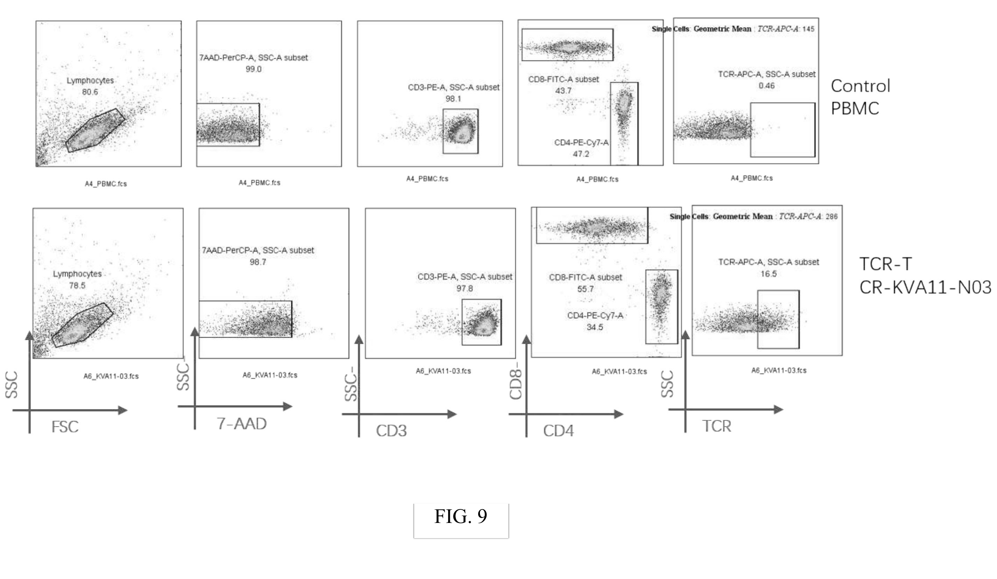

It has been revealed by in-depth studies that this TCR struggles to achieve high-level expression even on the surface of T cell membranes (as shown in FIG. 9). Such results may be attributed to its special V-region sequences. Does this mean that TCRs with such characteristics are destined to be inapplicable in TCR fusion proteins? Considering the scarcity of TCRs, the inventors have attempted to improve the soluble expression properties of this TCR through the aforementioned screened TCR constant region mutations.

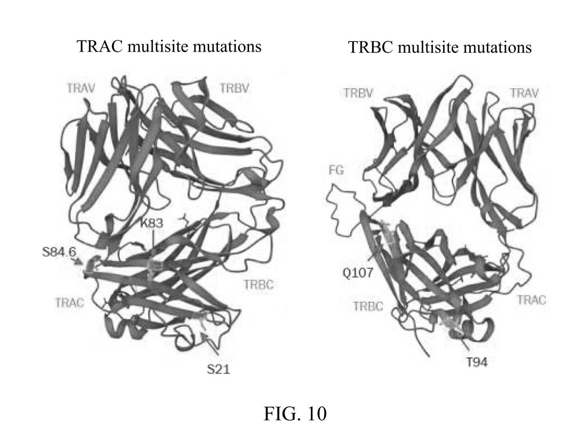

Guided by the computer-predicted three-dimensional structural model of the CR-KVA11-N03 TCR (FIG. 10), multisite mutations with non-interfering spatial positions were selected from the above-screened effective singlesite mutations for combination (the mutation combination was named CMM). An attempt was made to incorporate the CR-KVA11-N03 TCR into the B62# format (designated as CR-KVA11-N03-B62) and introduce the CMM mutations, aiming to observe whether the expression of the TCR fusion protein could be effectively improved.

To compare the relative contributions of constant-region mutations and variable-region mutations to the soluble-expression properties of the TCR, high-scoring mutations in the framework (FR) regions of the CR-KVA11-N03 variable region were predicted and screened using the same method as in Example 3.1.3 (increases in both the I-Mutant and SODA scores), and were combined (named "VMM1", "VMM2", "VMM3") and introduced into the CR-KVA11-N03-B62 format. Within the same batch expression experiment, we validated the impact on soluble expression of the following: the mutation-free wild type (WT, CR-KVA11-N03 TCR variable region wild type + parental constant region), the constant-region combination mutation (CMM1 (the combination of S21I, K83P and S84.6D in TRAC and T94D and Q107L in TRBC)), the variable-region combination mutations (VMM1, VMM2 and VMM3), and the combined variable-region (VMM1) + constant-region (CMM1) mutations (VCMM1).

The nucleotide sequence encoding the above CR-KVA11-N03-B62 molecule was cloned into an expression vector. After sequence confirmation of the resulting vector clone, amplification culture and plasmid extraction were carried out. Plasmids were then transfected into CHO cells, which were then cultured for 6-10 days, and the culture supernatant was collected. Protein A affinity purification was performed on the collected culture supernatant to obtain purified protein. The purified protein was subjected to denaturing (R) or non-denaturing (N) treatment, followed by SDS-PAGE gel electrophoresis to evaluate the protein expression purity and aggregation degree.

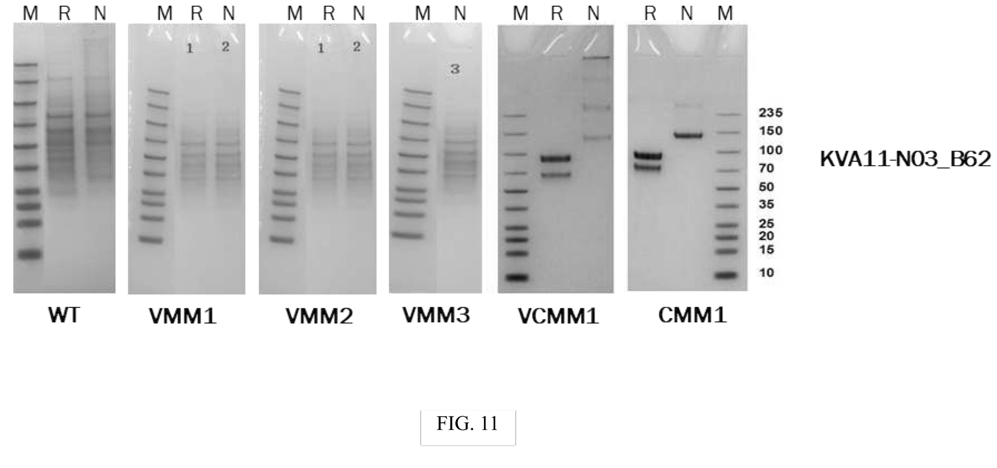

The SDS-PAGE results are as shown in FIG. 11. The results show that CR-KVA11-N03-B62 (WT) without mutations failed to express and assemble into a functional molecule; although the variable-region mutant molecules received high scores in algorithmic predictions, the variable-region combinations VMM1, VMM2 and VMM3 did not significantly improve expression of the CR-KVA11-N03-B62 molecule; introducing the constant-region combination CMM1 successfully achieved soluble expression of the CR-KVA11-N03-B62 molecule, and the SDS-PAGE results showed a molecular size consistent with expectation. At the same time, the expression product was subjected to only one step of Protein A affinity purification. SDS-PAGE results showed that the purified protein had high purity, with only a small amount of aggregates formed. Relative to VMM1, which did not improve soluble expression, introduction of the CMM1 combination (VCMM1) markedly increased soluble expression of the CR-KVA11-N03-B62 molecule.

### 3.2.3 Effect of the CMM1 mutations on the yield of TCR fusion proteins

The foregoing experiments have demonstrated that the CMM1 mutations can effectively improve soluble expression of TCRs, and can even enable soluble expression of certain TCRs that are very difficult to express on the membrane. We next further verified whether this property of CMM1 is compatible with different TCRs. Five TCRs derived from different species and recognizing different epitopes (CR-KVA11-N03, CR-KVA11-N04, CR-KVA11-N02, A1A2-M01-625, A1A2-M01-648) were each incorporated into the B62 format and were expressed using the B62-WT (TCR constant region is the parental TCR constant region) format and B62-CMM1 (TCR constant region contains the CMM1 mutations) format, respectively. In addition, the A1A2-M01-648 TCR was incorporated into the B62-LJH2 format (referred to as A1A2-M01-648-LJH2), and was expressed using the above WT and CMM1 formats, respectively.

The amino acid sequences of the above five TCRs are as follows:
CR-KVA11-N03: comprises the TRAV as shown in SEQ ID NO. 24 and the TRBV as shown in SEQ ID NO. 25
CR-KVA11-N04
TRAV 
GESVGLHLPTLGVQEGDNSIINCAYSNSASDYFIWYKQESGKGPQFIIDIRSNMDKRQGQRVTVLLNKT
VKHLSLQIAATQPGDSAVYFCAENSDGTSYGKLTFGQGTILTVHP (SEQ ID NO. 26)
TRBV 
EAGVAQSPRYKIIEKRQSVAFWCNPISGHATLYWYQQILGQGPKLLIQFQNNGVVDDSQLPKDRFSAE
RLKGVDSTLKIQPAKLEDSAVYLCASSLVGSPDYEQYFGPGTRLTVT (SEQ ID NO. 27)
CR-KVA 11-N02
TRAV 
AQSVAQPEDQVNVAEGNPLTVKCTYSVSGNPYLFWYVQYPNRGLQFLLKYITGDNLVKGSYGFEAEF
NKSQTSFHLKKPSALVSDSALYFCAVRDIEGAGNNRKLIWGLGTSLAVNP (SEQ ID NO. 28)
TRBV 
DAGVIQSPRHEVTEMGQEVTLRCKPISGHNSLFWYRQTMMRGLELLIYFNNNVPIDDSGMPEDRFSAK
MPNASFSTLKIQPSEPRDSAVYFCASSEGFYTEAFFGQGTRLTVV (SEQ ID NO. 29)
A1A2-M01-625
TRAV 
GEDVEQSLFLSVREGDSSVINCTYTDSSMTYLYWYKQEPGAGLQLLTYIFAFESMKQDQRLTVLLNKK
DKHLSLRIADTQTGDSAIYFCAGSGGGTDKLIFGTGTRLQVFP (SEQ ID NO. 30)
TRBV 
GAVVSQHPSWVICKSGTSVKIECRSLDFEATTMFWYRQFPKQSLMLMATTDYGSKATYEQGVEKDKF
LINHASLTLSTLTVTSAHPEDSSFYICSAREPGQGPWEQYFGPGTRLTVT (SEQ ID NO. 31)
A1A2-M01-648
TRAV 
GEDVEQSLFLSVREGDSSVINCTYTDSSMTYLYWYKQEPGAGLQLLTYIFFYQDMKQDQRLTVLLNK
KDKHLSLRIADTQTGDSAIYFCAGSGGGTDKLIFGTGTRLQVFP (SEQ ID NO. 32)
TRBV

GAVVSQHPSWVICKSGTSVKIECRSLDFEATTMFWYRQFPKQSLMLMATTDYGSKATYEQGVEKDKF
LINHASLTLSTLTVTSAHPEDSSFYICSAREPGQAWSDQYFGPGTRLTVT (SEQ ID NO. 33)

The B62-LJH2 format comprises the following two polypeptide chains:
The first polypeptide chain: TRBV-TRBC-first linker-VH-second linker-VL-hinge region-CH2-CH3;
The second polypeptide chain: TRAV-TRAC-hinge region-CH2-CH3.

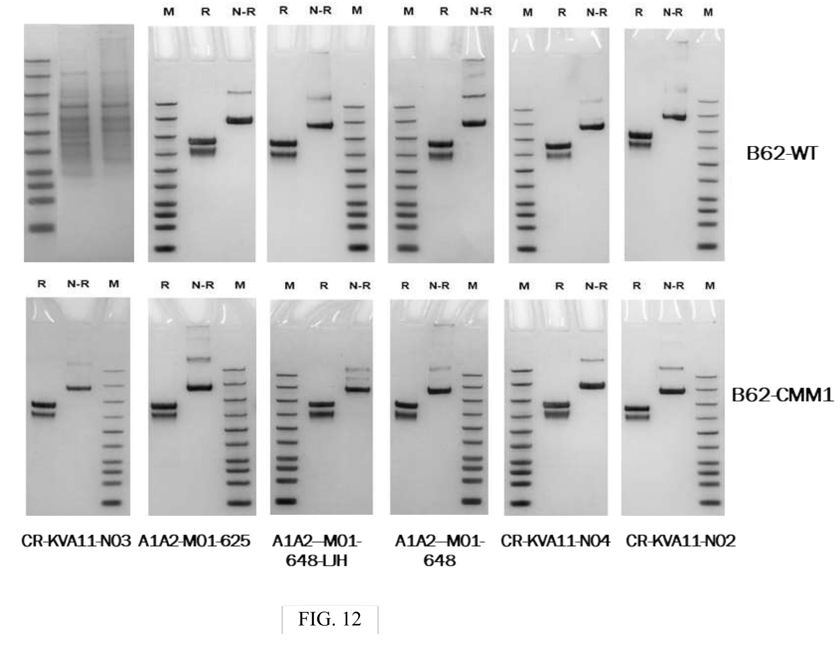

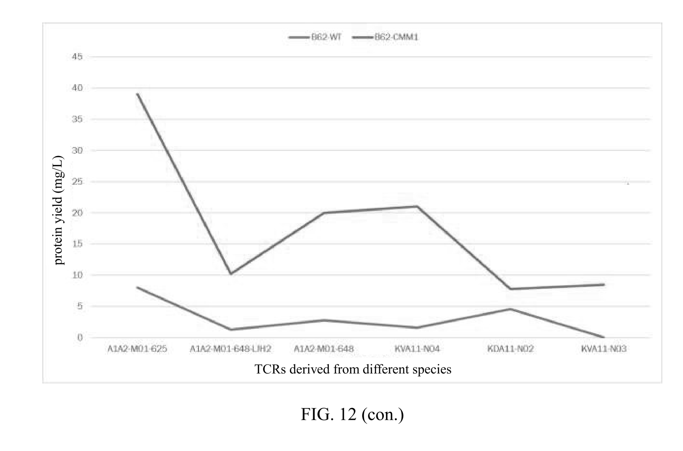

Protein expression and SDS-PAGE were performed as described in Example 3.2.2, and protein yield was calculated based on the SDS-PAGE results. The results are as shown in FIG. 12.

The results demonstrate that the CMM1 mutations can broadly increase the soluble-expression yield of multiple different TCRs, and can be applied to multiple different TCR fusion proteins.

### 3.2.4 Effect of the CMM1 mutations on the activity of TCR fusion proteins

It is well known that structural changes in protein molecules caused by amino acid mutations may directly affect protein activity. Although, structurally, the TCR constant region is remote from the CDRs and the TCR-pMHC interaction interface, it cannot be ruled out whether local conformational changes in the constant region might indirectly exert distal effects on the TCR-pMHC interaction interface.

Therefore, B62 formats incorporated with TCRs targeting different antigen epitopes (A1A2-M01-625 (amino acid sequence thereof is as described in Example 3.2.3), KDA11-N02, KVA11-N04 (amino acid sequences thereof are as described in Example 3.2.3)) and a B62-LJH2 format incorporated with the MAGE-A1 specific TCR A1A2-M01-648 (amino acid sequence thereof is as described in Example 3.2.3) (referred to as A1A2-M01-648-LJH2_B62) were used as carriers to validate whether the CMM1 mutations would adversely affect protein activity. Early studies had clarified that these TCR-B62 protein molecules can, in vitro, stimulate PBMCs to produce specific activation in the presence of unique pMHC epitopes thereof; whereas in the absence of pMHC epitopes they cannot activate PBMCs under appropriate protein concentration conditions, but at high protein concentrations (e.g., -7 M) they readily induce non-specific activation of PBMCs. Therefore, the CMM1 mutations were introduced into these TCR-B62 protein molecules. The parental TCR constant region served as the wild-type control.

The amino acid sequence of KDA11-N02 is as follows:
TRAV 
RKEVEQDPGPFNVPEGATVAFNCTYSNSASQSFFWYRQDCRKEPKLLMSVYSSGNEDGRFTAQLNRA
SQYISLLIRDSKLSDSATYLCVVTNARLMFGDGTQLVVKP (SEQ ID NO. 34)
TRBV

EAQVTQNPRYLITVTGKKLTVTCSQNMNHEYMSWYRQDPGLGLRQIYYSMNVEVTDKGDVPEGYK
VSRKEKRNFPLILESPSPNQTSLYFCASTSIVSSGRGEQFFGPGTRLTVL (SEQ ID NO. 35)

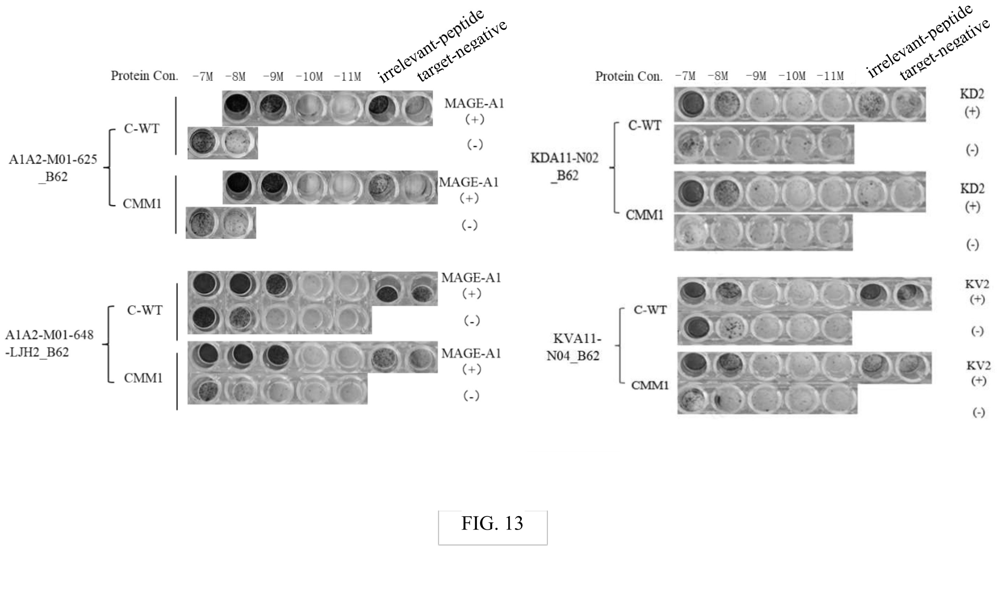

Protein expression and ELISPOT experiments were performed using experimental methods similar to those in Example 3.2.1. The ELISPOT results are as shown in FIG. 13.

The experimental results show:
a) The CMM1 mutations do not affect activity against the target: introducing the CMM1 mutations did not negatively affect the activities of the A1A2-M01-625_B62, A1A2-M01-648-LJH2_B62, KDA11-N02_B62 and KVA11-N04_B62 proteins; at low protein concentrations, the activation effects of the control group (C-WT) and the CMM1 experimental group were similar, and in some cases the CMM1 experimental group exhibited stronger activation;
b) The CMM 1 mutations reduce non-specific activation caused by high protein concentrations: under high protein concentration (-7 M) conditions, PBMCs in the target-negative cohort of the control group all exhibited clear non-specific activation, and the magnitude of non-specific activation showed a certain correlation with the magnitude of specific activation; whereas in the target-negative cohort of the **CMM1** experimental group, non-specific activation of PBMCs was significantly reduced without exception;
c) The CMM1 mutations reduce non-specific activation caused by irrelevant epitopes: under high protein concentration (-7 M) conditions, PBMCs in the irrelevant-peptide cohort of the control group all exhibited clear non-specific activation, and the magnitude of non-specific activation showed a certain correlation with the magnitude of specific activation; whereas in the target-negative cohort of the CMM1 experimental group, non-specific activation of PBMCs was significantly reduced without exception.

These results indicate that introducing the CMM1 mutations into the B62 and B62-LJH2 formats does not reduce activity against the target, and at the same time effectively reduces non-specific activation of PBMCs under high protein-concentration conditions.

### 3.2.5 Compatibility of CMM1 with different TCR fusion proteins

To verify whether the above-screened CMM1 can be applied to different TCR fusion-protein formats, a format 22-2 fusion protein was used for protein expression and ELISPOT experiments, in which the TCR was CR-KVA11-N03, and the parental TCR constant region served as the wild-type control.

Format 22-2 comprises the following two polypeptide chains:
The first polypeptide chain: TRAV-linker-VH-linker-TRAC, with an amino acid sequence as shown in SEQ ID NO. 92;
The second polypeptide chain: VL-linker-TRBV-TRBC, with an amino acid sequence as shown in SEQ ID NO. 93;
Specific experimental operations were as follows:

### 1. Vector construction and plasmid extraction

The nucleotide sequences encoding SEQ ID NO. 92 and SEQ ID NO. 93 were directly cloned into the expression vector pTT5. After sequence confirmation of the resulting vector clones, amplification culture and plasmid extraction were carried out.

### 2. Transfection

CHO cells were adjusted to a density of 1 × 10⁶ cells/mL, with a liquid volume of 40 mL per bottle, then the caps were tightened and the flasks were placed on a shaker for continued culture; after incubation at 36.5°C, 175 rpm, 5% CO₂ for 2-4 hours, the cells were transfected with plasmids. Preparation of the transfection mixture (1 mL): approximately 800 µL of sterile 150 mM NaCl solution was used to dilute 10 µg DNA, mixed and left on the bench for 5 min; approximately 50 µL of transfection reagent was added to the DNA dilution and mixed, giving a final transfection-mixture volume of 1 mL. After standing on the bench for 10 min, the transfection mixture was added dropwise to the cell culture, shaken to mix, then the caps were tightened and the flasks were returned to the shaker (36.5°C, with 5% CO₂ off, 175 rpm). At 20-24 h post-transfection, SMS 293-I feed (0.7 mL/flask) was added, and thereafter feeding was performed every other day for 6-10 days of culture, and the culture supernatants were finally collected.

The collected culture supernatants were serially diluted (typically 2-fold and 5-fold dilutions). The corresponding target peptide and irrelevant peptide were dissolved in DMSO and diluted with water to a working concentration of 10⁻⁴ M. T2 cells were loaded with 10⁻⁶ M target peptide and irrelevant peptide, respectively. Complete RPMI-1640 medium containing 10% FBS was added to the ELISPOT plate and blocked at room temperature for 30 min. The medium was discarded and PBMCs at 5 × 10⁵ cells/mL (100 µL/well), peptide-loaded T2 cells at 5 × 10⁵ cells/mL (100 µL/well), and different concentrations of the formats (2-fold and 5-fold dilutions) were added. For the negative control, unloaded T2 cells and PBMCs were added, with the concentration of the candidate peptide molecules consistent with that in the experimental groups; the positive control was prepared by adding 10µL of the anti-CD3-2 mAb provided with the kit to PBMCs; the irrelevant-peptide control was prepared by adding an irrelevant peptide. After all samples were added, the plate was covered and incubated in a 37°C, 5% CO₂ incubator for 20-24 hours; IFN-γ secretion was then determined by ELISPOT to evaluate immune cell activation induced by the formats via the target antigen peptide-MHC complex.

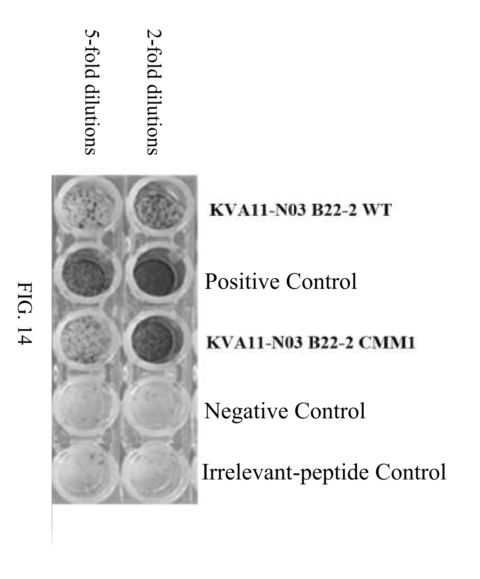

The results are as shown in FIG. 14. The results indicate that the supernatants of the CMM1 experiment group were able to activate immune cells at both 2-fold and 5-fold dilutions, and exhibited significantly stronger PBMC activation activity than that of the wild-type control group; such an increase in activity may arise from multiple benefits including protein yield and activity, indicating that the CMM1 mutations can be applied to multiple different TCR fusion proteins to increase soluble expression of the fusion proteins.

### 3.2.6 Evaluation of different CMM combinations

Further, on the basis of the **CMM1** mutation combination, each individual mutated amino acid site in the combination was subjected to reverse mutation (mutated back to the wild type [WT]) in order to achieve the best gain effect with as few mutations as possible, while retaining more natural amino acid sequences in the protein molecule.

The combined mutations derived from the reverse mutations of CMM1 include CMM2 (comprising mutations S21I, K83P, S84.6D, Q107L), CMM3 (comprising mutations S21I, K83P, S84.6D, T94D), CMM4 (comprising mutations K83P, S84.6D, T94D, Q107L), and CMM5 (comprising mutations S21I, K83P, T94D, Q107L).

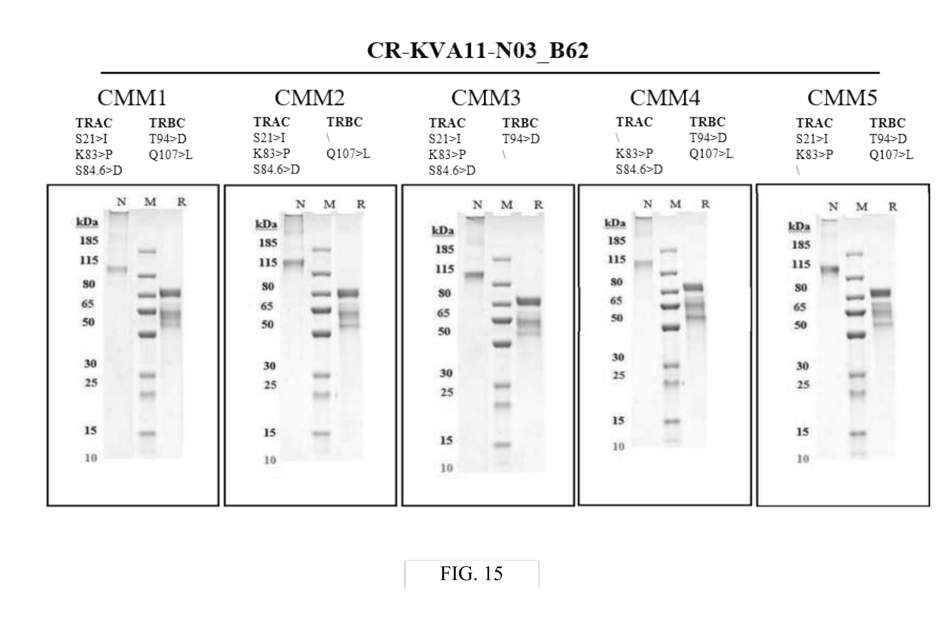

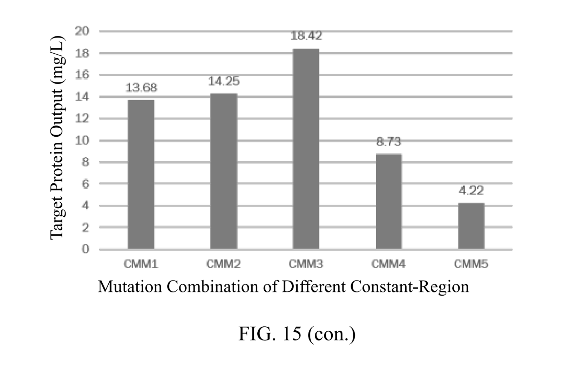

Using the B62 format loaded with the KRAS-specific TCR CR-KVA11-N03 (amino acid sequence is shown in Example 3.2.4) (referred to as CR-KVA1 1-N03_B62) as the carrier, the above-mentioned CMM mutations were incorporated into the CR-KVA11-N03_B62 molecule. Protein expression and SDS-PAGE were performed as described in Example 3.2.2, and the protein yield was calculated based on the SDS-PAGE results. The results are shown in FIG. 15.

Protein expression results showed that reverse mutation of the TRAC mutation sites S21>I and S84.6>D led to a significant reduction in the yield of soluble protein molecules; reverse mutation of the TRBC mutation site T94>D did not result in a significant change in the yield of soluble protein molecules; and reverse mutation of Q107>L resulted in an even higher protein yield.

The above results indicate that although the spatial distances between each individual mutation in the three-dimensional conformation are relatively large, some mutations in the combination exhibit positive synergistic effects, such as S21>I and S84.6>D in TRAC. In contrast, some mutations do not actively contribute, for instance, T94>D in TRBC; furthermore, the inclusion of Q107>L in TRBC in the combination even leads to a reduction in expression yield.

The present application makes reference to various published patents, published patent applications, journal articles and other publications, all of which are incorporated herein by reference. If there is a conflict between any of the incorporated references and the present specification, the specification prevails. In addition, any specific embodiment of the present invention falling within the scope of the prior art may be expressly excluded from any one or more of the claims. Because the embodiments are considered to be known to those skilled in the art, they may be excluded even if the exclusion is not expressly set forth in the present application. Any specific embodiment of the present invention may be excluded from any claim for any reason, whether or not it relates to the existence of prior art.

While the present invention has been described with reference to particular embodiments thereof, it should be understood by those skilled in the art that various changes can be made and equivalents can be substituted without departing from the true spirit and scope of the present invention. In addition, many modifications may be made to make particular instances, materials, compositions, methods, steps of methods suitable for the purpose, spirit and scope of the present invention. All such modifications are intended to be within the scope of the claims.

## Claims

1. A T cell receptor (TCR) α chain constant region (TRAC), wherein the α chain constant region (TRAC) comprises amino acid substitution(s) at one or more of the positions Q6, S21, K83, S84.6, and I114;
wherein the amino acid positions are numbered according to the International Immunogenetics Information System (IMGT) numbering rules based on the TCR α chain constant region as shown in SEQ ID NO. 1.

2. The TCR α chain constant region according to claim 1, wherein
(1) the amino acid substitution at position Q6 of the α chain constant region is selected from Q6R, Q6K, Q6D and Q6E, preferably selected from Q6R and Q6K; and/or
(2) the amino acid substitution at position S21 of the α chain constant region is selected from S21I, S21L, S21V, S21G, S21A and S21P, preferably selected from S21I, S21L and S21V; and/or
(3) the amino acid substitution at position K83 of the α chain constant region is selected from K83P, K83G, K83A, K83I, K83L and K83V, preferably selected from K83P, K83G and K83A; and/or
(4) the amino acid substitution at position S84.6 of the α chain constant region is selected from S84.6D, S84.6E, S84.6N, S84.6Q, S84.6K and S84.6R, preferably selected from S84.6D, S84.6E, S84.6N and S84.6Q; and/or
(5) the amino acid substitution at position I114 of the α chain constant region is selected from I114E, I114D, I114K and I114R, preferably selected from I114E and I114D.

3. The TCR α chain constant region according to claim 1 or 2, wherein
(1) the amino acid substitution at position Q6 of the α chain constant region is Q6R; and/or
(2) the amino acid substitution at position S21 of the α chain constant region is S21I; and/or
(3) the amino acid substitution at position K83 of the α chain constant region is K83P; and/or
(4) the amino acid substitution at position S84.6 of the α chain constant region is selected from S84.6D, S84.6E and S84.6N; and/or
(5) the amino acid substitution at position I114 of the α chain constant region is I114E.

4. The TCR α chain constant region according to any one of claims 1 to 3, wherein the α chain constant region comprises amino acid substitutions at positions S21, K83 and S84.6.

5. The TCR α chain constant region according to any one of claims 1 to 4, wherein the α chain constant region comprises one or more of the amino acid substitutions S21I, K83P and S84.6D; preferably comprises at least two amino acid substitutions of S21I, K83P and S84.6D; more preferably comprises amino acid substitutions of S21I, K83P and S84.6D.

6. The TCR α chain constant region according to any one of claims 1 to 5, wherein the α chain constant region comprises amino acid substitutions selected from any one of the following groups (1) to (4):
(1) S21I, K83P and S84.6D;
(2) K83P and S84.6D;
(3) S21I and S84.6D;
(4) S21I and K83P.

7. The TCR α chain constant region according to any one of claims 1 to 6, wherein the α chain constant region further comprises one or more of the amino acid mutations N1.2K, T84C and an FFPSP deletion at positions 120-124;
preferably comprises T84C, and optionally N1.2K and/or an FFPSP deletion at positions 120-124;
more preferably comprises N1.2K, T84C and an FFPSP deletion at positions 120-124.

8. The TCR α chain constant region according to any one of claims 1 to 7, wherein the α chain constant region comprises an amino acid sequence as shown in any one of SEQ ID NOs.3-13 or an amino acid sequence having at least 80%, at least 85%, at least 90%, at least 95% or at least 99% sequence identity to any one of SEQ ID NOs.3-13.

9. A TCR α chain constant region (TRAC), wherein the TCR α chain constant region (TRAC) comprises amino acid substitutions S21I, K83P and S84.6D, wherein the amino acid positions are numbered according to the International Immunogenetics Information System (IMGT) numbering rules based on the TCR α chain constant region as shown in SEQ ID NO. 1;
preferably, the TCR α chain constant region comprises an amino acid sequence as shown in SEQ ID NO.13 or an amino acid sequence having at least 80%, at least 85%, at least 90%, at least 95% or at least 99% sequence identity to SEQ ID NO.13.

10. A TCR β chain constant region (TRBC), wherein the β chain constant region (TRBC) comprises amino acid substitution(s) at one or more of the positions S92, T94 and Q107;
wherein the amino acid positions are numbered according to the International Immunogenetics Information System (IMGT) numbering rules based on the TCR β constant region as shown in SEQ ID NO. 14.

11. The TCR β chain constant region according to claim 10, wherein
(1) the amino acid substitution at position S92 of the β chain constant region is selected from S92P, S92E, S92D, S92L, S92N, S92G, S92A, S92I, S92V, S92Q, S92K and S92R, preferably selected from S92P, S92E, S92D, S92L, S92N, S92G, S92A, S92I, S92V and S92Q; and/or
(2) the amino acid substitution at position T94 of the β chain constant region is selected from T94D, T94E, T94K and T94R, preferably selected from T94D and T94E; and/or
(3) the amino acid substitution at position Q107 of the β chain constant region is selected from Q107L, Q107I, Q107V, Q107G, Q107A and Q107P, preferably selected from Q107L, Q107I and Q107V.

12. The TCR β chain constant region according to claim 10 or 11, wherein
(1) the amino acid substitution at position S92 of the β chain constant region is selected from S92P, S92E, S92D, S92L and S92N; and/or
(2) the amino acid substitution at position T94 of the β chain constant region is T94D; and/or
(3) the amino acid substitution at position Q107 of the β chain constant region is Q107L.

13. The TCR β chain constant region according to any one of claims 10 to 12, wherein the β chain constant region comprises amino acid substitutions at T94 and Q107.

14. The TCR β chain constant region according to any one of claims 10 to 13, wherein the β chain constant region comprises one or more of the amino acid substitutions T94D and Q107L; preferably comprises amino acid substitutions T94D and Q107L.

15. The TCR β chain constant region according to any one of claims 10 to 14, wherein the TCR β chain constant region comprises amino acid substitution(s) selected from any one of the following groups (1) to (3):
(1) T94D and Q107L;
(2) Q107L;
(3) T94D.

16. The TCR β chain constant region according to any one of claims 10 to 15, wherein the TCR β chain constant region further comprises one or more of the amino acid mutations S79C, C85.1A and N97D; preferably comprises S79C and optionally C85.1A and/or N97D; more preferably comprises S79C, C85.1A and N97D.

17. The TCR β chain constant region according to any one of claims 10 to 16, wherein the TCR β chain constant region comprises an amino acid sequence as shown in any one of SEQ ID NOs. 16-23 or an amino acid sequence having at least 80%, at least 85%, at least 90%, at least 95% or at least 99% sequence identity to any one of SEQ ID NOs. 16-23.

18. A TCR β chain constant region (TRBC), wherein the TCR β chain constant region (TRBC) comprises amino acid substitutions T94D and Q107L, wherein the amino acid positions are numbered according to the International Immunogenetics Information System (IMGT) numbering rules based on the TCR β chain constant region as shown in SEQ ID NO. 14;
preferably, the TCR β chain constant region comprises an amino acid sequence as shown in SEQ ID NO.23 or an amino acid sequence having at least 80%, at least 85%, at least 90%, at least 95% or at least 99% sequence identity to SEQ ID NO.23.

19. A TCR constant region comprising the TCR α chain constant regions (TRAC) according to any one of claims 1 to 9 and the TCR β chain constant regions (TRBC) according to any one of claims 10 to 18;
preferably, the TCR constant region comprises amino acid substitutions selected from any one of the following groups (1) to (6):
(1) S21I, K83P and S84.6D of the α chain constant region, and T94D and Q107L of the β chain constant region;
(2) K83P and S84.6D of the α chain constant region, and T94D and Q107L of the β chain constant region;
(3) S21I and S84.6D of the α chain constant region, and T94D and Q107L of the β chain constant region;
(4) S21I and K83P of the α chain constant region, and T94D and Q107L of the β chain constant region;
(5) S21I, K83P and S84.6D of the α chain constant region, and Q107L of the β chain constant region;
(6) S21I, K83P and S84.6D of the α chain constant region, and T94D of the β chain constant region;
more preferably, the α chain constant region comprises the amino acid substitutions S21I, K83P and S84.6D, and the β chain constant region comprises amino acid substitutions T94D and Q107L;
further more preferably, the TCR α chain constant region comprises an amino acid sequence as shown in SEQ ID NO.13 or an amino acid sequence having at least 80%, at least 85%, at least 90%, at least 95% or at least 99% sequence identity to SEQ ID NO.13; and the TCR β chain constant region comprises an amino acid sequence as shown in SEQ ID NO.23 or an amino acid sequence having at least 80%, at least 85%, at least 90%, at least 95% or at least 99% sequence identity to SEQ ID NO.23.

20. A polypeptide molecule comprising the TCR α chain constant region according to any one of claims 1 to 9, the TCR β chain constant region according to any one of claims 10 to 18, or the TCR constant region according to claim 19.

21. The polypeptide molecule according to claim 20, further comprising an antigen binding region that binds at least one antigen.

22. The polypeptide molecule according to claim 21, wherein the antigen binding region is selected from an antigen binding region derived from an antibody and an antigen binding region derived from a TCR.

23. A T cell receptor (TCR) comprising an α chain and a β chain, wherein the α chain comprises the TCR α chain constant region according to any one of claims 1 to 9, and/or the β chain comprises the TCR β chain constant region according to any one of claims 10 to 18; or the TCR comprises the TCR constant region according to claim 19.

24. The TCR according to claim 23, wherein the TCR binds an antigen selected from the group consisting of melanoma-associated antigens (e.g., gp100, MAGEA1), HPV antigens (e.g., HPV E6 or E7) and KRAS antigens (e.g., KRAS G12V, KRAS G12D).

25. The TCR according to claim 23 or 24, wherein the TCR comprises a variable region derived from a murine TCR or a human TCR.

26. The TCR according to any one of claims 23 to 25, wherein the TCR is isolated or purified.

27. A multi-specific antigen binding molecule comprising the TCR α chain constant region according to any one of claims 1 to 9, the TCR β chain constant region according to any one of claims 10 to 18, the TCR constant region according to claim 19, the polypeptide molecule according to any one of claims 20 to 22, or the TCR according to any one of claims 23 to 26.

28. A multi-specific antigen binding molecule comprising at least one antigen binding region derived from a TCR and any one selected from the following groups (1)-(2):
(1) the TCR α chain constant regions (TRAC) according to any one of claims 1 to 9, and/or the TCR β chain constant regions (TRBC) according to any one of claims 10 to 18;
(2) the TCR constant region according to claim 19.

29. The multi-specific antigen binding molecule according to claim 28, wherein the multi-specific antigen binding molecule comprises two or more antigen binding regions that bind two or more antigens, and the two or more antigens are each independently selected from the group consisting of tumor-associated antigens (TAAs), tumor-specific antigens (TSAs), viral antigens, self-antigens and immune-cell surface molecules;
preferably, one of the two or more antigens is selected from the group consisting of tumor-associated antigens (TAAs), tumor-specific antigens (TSAs), viral antigens and self-antigens, and the other is immune-cell surface molecules;
more preferably, the TAA is melanoma-associated antigens (e.g., gp100, MAGEA1); and/or
the TSA is KRAS antigens (e.g., KRAS G12V, KRAS G12D); and/or
the viral antigen is HPV antigens (e.g., HPV E6 or E7); and/or
the immune-cell surface molecule is selected from the group consisting of CD3, CD28, 4-1BB (CD137), PD-1 and PD-L1.

30. The multi-specific antigen binding molecule according to claim 28 or 29, wherein the multi-specific antigen binding molecule comprises, on at least two polypeptide chains, a first binding region that binds a first antigen and a second binding region that binds a second antigen,
wherein the first binding region comprises an α chain variable region (TRAV) and a β chain variable region (TRBV) derived from a TCR that binds the first antigen-MHC complex;
wherein the second binding region comprises a heavy-chain variable region (VH) and a light-chain variable region (VL) derived from an antibody that binds the second antigen;
wherein the first antigen is selected from the group consisting of tumor-associated antigens (TAAs), tumor-specific antigens (TSAs), viral antigen and self-antigens, and the second antigen is immune-cell surface molecules;
and wherein the multi-specific antigen binding molecule further comprises at least one functional domain selected from the group consisting of:
1) a hinge region derived from an antibody;
2) an Fc domain derived from an antibody or a dimerization portion thereof;
3) an CH3 domain derived from an antibody;
4) an albumin (Alb) or a binding portion thereof;
5) a TCR constant region or a fragment thereof.

31. The multi-specific antigen binding molecule according to claim 30, wherein the TRAC and the TRBC are respectively connected to the C-terminus of the TRAV and the TRBV via optional linkers; or the TRAC and the TRBC are respectively connected to the C-terminus of the TRBV and the TRAV via optional linkers; and/or the N-terminus of the antibody is connected to the C-terminus of the TCR.

32. The multi-specific antigen binding molecule according to claim 30 or 31, wherein the functional domain is derived from an antibody hinge region, and/or an antibody Fc domain or a dimerization portion thereof, and/or an antibody CH3 domain, and/or a TCR constant region or a fragment thereof; and any two polypeptide chains of the multi-specific antigen binding molecule are connected via covalent or non-covalent bonds between the two chains of the functional domain;
preferably, the two chains of the functional domain are respectively linker to the C-terminus or N-terminus, preferably to the C-terminus, of any two polypeptide chains of the multi-specific antigen binding molecule via optional linkers; or
the first binding region and the second binding region are connected via covalent or non-covalent bonds between the two chains of the functional domain;
more preferably, the multi-specific antigen binding molecule comprises a first polypeptide chain and a second polypeptide chain, wherein
the first polypeptide chain comprises, from the N-terminus to the C-terminus: TRBV-optional linker-TRBC-linker-VL-linker-VH-optional linker-hinge region-CH2-CH3, and the second polypeptide chain comprises, from the N-terminus to the C-terminus: TRAV-optional linker-TRAC-optional linker-hinge region-CH2-CH3; or
the first polypeptide chain comprises, from the N-terminus to the C-terminus: TRAV-optional linker-TRAC-linker-VL-linker-VH-optional linker-hinge region-CH2-CH3, and the second polypeptide chain comprises, from the N-terminus to the C-terminus: TRBV-optional linker-TRBC-optional linker-hinge region-CH2-CH3; or
the first polypeptide chain comprises, from the N-terminus to the C-terminus: TRBV-optional linker-TRBC-linker-VH-linker-VL-optional linker-hinge region-CH2-CH3, and the second polypeptide chain comprises, from the N-terminus to the C-terminus: TRAV-optional linker-TRAC-optional linker-hinge region-CH2-CH3; or
the first polypeptide chain comprises, from the N-terminus to the C-terminus: TRAV-optional linker-TRBC-linker-VH-linker-VL-optional linker-hinge region-CH2-CH3, and the second polypeptide chain comprises, from the N-terminus to the C-terminus: TRBV-optional linker-TRBC-optional linker-hinge region-CH2-CH3; or
the multi-specific antigen binding molecule comprises a first polypeptide chain, a second polypeptide chain and a third polypeptide chain, wherein the first polypeptide chain comprises, from the N-terminus to the C-terminus: TRBV-optional linker-TRBC-linker-VL, the second polypeptide chain comprises, from the N-terminus to the C-terminus: VH-optional linker-hinge region-CH2-CH3, and the third polypeptide chain comprises, from the N-terminus to the C-terminus: TRAV-optional linker-TRAC-optional linker-hinge region-CH2-CH3.

33. A nucleic acid encoding the TCR α chain constant region according to any one of claims 1 to 9, the TCR β chain constant region according to any one of claims 10 to 18, the TCR constant region according to claim 19, the polypeptide molecule according to any one of claims 20 to 22, the TCR according to any one of claims 23 to 26, or the multi-specific antigen binding molecule according to any one of claims 27 to 32.

34. A vector comprising the nucleic acid according to claim 33.

35. The vector according to claim 34, wherein the vector is selected from the group consisting of lentiviral vectors, retroviral vectors, plasmids, DNA vectors, mRNA vectors, transposon-based vectors, and artificial chromosomes.

36. A host-cell comprising the TCR α chain constant region according to any one of claims 1 to 9, the TCR β chain constant region according to any one of claims 10 to 18, the TCR constant region according to claim 19, the polypeptide molecule according to any one of claims 20 to 22, the TCR according to any one of claims 23 to 26, the multi-specific antigen binding molecule according to any one of claims 27 to 32, the nucleic acid according to claim 33, or the vector according to claim 34 or 35.

37. A conjugate comprising the polypeptide molecule according to any one of claims 20 to 22, the TCR according to any one of claims 23 to 26, or the multi-specific antigen binding molecule according to any one of claims 27 to 32, and a chemical moiety conjugated to the polypeptide molecule, the TCR or the multi-specific antigen binding molecule.

38. The conjugate according to claim 37, wherein the chemical moiety is selected from the group consisting of detectable labels, immunostimulatory molecules and therapeutic agents;
preferably, the detectable label is selected from the group consisting of biotins, streptavidin, enzymes or a catalytically active fragment thereof, radionuclides, nanoparticles, paramagnetic metal ions, nucleic-acid probes, contrast agents, fluorescents, phosphorescent and chemiluminescent molecules;
preferably, the immunostimulatory molecule is selected from the group consisting of cytokines, chemokines, platelet factors and complement activators;
preferably, the therapeutic agent is selected from the group consisting of immunomodulators, radioactive compounds, enzymes, chemotherapeutic agents and toxins.

39. A composition comprising the polypeptide molecule according to any one of claims 20 to 22, the TCR according to any one of claims 23 to 26, the multi-specific antigen binding molecule according to any one of claims 27 to 32, the nucleic acid according to claim 33, the vector according to claim 34 or 35, the host cell according to claim 36, or the conjugate according to claim 37 or 38; and a pharmaceutically acceptable carrier or excipient.

40. The composition according to claim 39, wherein the composition further comprises a second therapeutic agent, preferably selected from the group consisting of antibodies, chemotherapeutic agents and small-molecule drugs.

41. A method for treating a disease in a subject, comprising administering to the subject an effective amount of the polypeptide molecule according to any one of claims 20 to 22, the TCR according to any one of claims 23 to 26, the multi-specific antigen binding molecule according to any one of claims 27 to 32, the nucleic acid according to claim 33, the vector according to claim 34 or 35, the host cell according to claim 36, the conjugate according to claim 37 or 38, or the composition according to claim 39 or 40.

42. The method according to claim 41, wherein the disease is selected from the group consisting of cancers, infectious diseases, autoimmune diseases and inflammatory diseases.

43. The method according to claim 41 or 42, wherein the method further comprises administering a second therapeutic agent, preferably selected from the group consisting of antibodies, chemotherapeutic agents and small-molecule drugs.
